# EUROPEAN PATENT SPECIFICATION

(11) **EP 3 214 183 B1**
(45) Date of publication and mention of the grant of the patent: **21.11.2018**
(21) Application number: 16000496.6
(22) Date of filing: 01.03.2016
(51) Int. Cl.: C12Q 1/6809

(54) **TRANSCRIPTION ACTIVATOR-LIKE EFFECTOR (TALE)-BASED DECODING OF CYTOSINE NUCLEOBASES BY SELECTIVE MODIFICATION RESPONSE**
DECODIERUNG AUF BASIS VON TRANSKRIPTIONSAKTIVATORARTIGEM EFFEKTOR (TALE) VON CYTOSINNUKLEOBASEN DURCH SELEKTIVE MODIFIKATIONSREAKTION
DÉCODAGE À BASE D'EFFECTEUR DE TYPE ACTIVATEUR DE LA TRANSCRIPTION (TALE) DE CYTOSINE NUCLÉOBASES PAR RÉPONSE DE MODIFICATION SÉLECTIVE

(43) Date of publication of application: 06.09.2017
(73) Proprietor: Universität Konstanz, 78464 Konstanz (DE)
(72) Inventor: Summerer, Daniel, 44137 Dortmund (DE); Giess, Mario, 44227 Dortmund (DE); Kubik, M. Grzegorz, 78467 Konstanz (DE); Maurer, Sara, 44137 Dortmund (DE)
(74) Representative: Müller-Boré & Partner Patentanwälte PartG mbB

(56) References cited:
- WO-A1-2013/140250
- WO-A1-2014/206568
- DONG DENG ET AL: "Recognition of methylated DNA by TAL effectors", CELL RESEARCH, vol. 22, no. 10, 1 October 2012 (2012-10-01), pages 1502-1504, XP055084118, ISSN: 1001-0602, DOI: 10.1038/cr.2012.127
- MIAO YU ET AL: "Base-Resolution Analysis of 5-Hydroxymethylcytosine in the Mammalian Genome", CELL, vol. 149, no. 6, 1 June 2012 (2012-06-01), pages 1368-1380, XP055064960, ISSN: 0092-8674, DOI: 10.1016/j.cell.2012.04.027
- GRZEGORZ KUBIK ET AL: "TALEored Epigenetics: A DNA-Binding Scaffold for Programmable Epigenome Editing and Analysis", CHEMBIOCHEM - A EUROPEAN JOURNAL OF CHEMICAL BIOLOGY., vol. 17, no. 11, 20 April 2016 (2016-04-20), pages 975-980, XP055294731, DE ISSN: 1439-4227, DOI: 10.1002/cbic.201600072

### SEQUENCE LISTING

<110> Universität Konstanz
<120> Transcription Activator-Like Effector (TALE)-based Decoding of Cytosine Nucleobases by Selective Modification Response
<130> K 5037EU - kl
<160> 52
<170> PatentIn version 3.5
<210> 1
   <211> 23
   <212> DNA
   <213> Artificial sequence
<220>
   <223> sequencing primer
<400> 1
   gggttatgct agttattgct cag 23
<210> 2
   <211> 17
   <212> DNA
   <213> Artificial sequence
<220>
   <223> sequencing primer
<400> 2
   cgtagaggat cgagatc 17
<210> 3
   <211> 17
   <212> DNA
   <213> Artificial sequence
<220>
   <223> primer
<400> 3
   gagtaacagc ggtagag 17
<210> 4
   <211> 79
   <212> DNA
   <213> Artificial sequence
<220>
   <223> primer
<220>
   <221> modified_base
   <222> (67)..(67)
   <223> mC
<400> 4
   tggattccca ctcttcagcc ccagcgttac agcatcttca gtggcttctt ccaccgtgag    60
   ctcttccgtt tccacatcc                                                 79
<210> 5
   <211> 18
   <212> DNA
   <213> Artificial sequence
<220>
   <223> primer
<400> 5
   ggatgtggaa acggaaga 18
<210> 6
   <211> 79
   <212> DNA
   <213> Artificial sequence
<220>
   <223> primer
<400> 6
   tggattccca ctcttcagcc ccagcgttac agcatcttca gtggcttctt ccaccgtgag    60
   ctcttccgtt tccacatcc                                                 79
<210> 7
   <211> 79
   <212> DNA
   <213> Artificial sequence
<220>
   <223> primer
<220>
   <221> modified_base
   <222> (67)..(67)
   <223> hmC
<400> 7
   tggattccca ctcttcagcc ccagcgttac agcatcttca gtggcttctt ccaccgtgag    60
   ctcttccgtt tccacatcc                                                 79
<210> 8
   <211> 20
   <212> DNA
   <213> Artificial sequence
<220>
   <223> sequencing primer
<400> 8
   ttgatgcctg gcagttccct 20
<210> 9
   <211> 20
   <212> DNA
   <213> Artificial sequence
<220>
   <223> sequencing primer
<400> 9
   cgaaccgaac aggcttatgt 20
<210> 10
   <211> 17
   <212> DNA
   <213> Artificial sequence
<220>
   <223> sequencing primer
<400> 10
   aatgcgcaaa ccaaccc 17
<210> 11
   <211> 19
   <212> DNA
   <213> Artificial sequence
<220>
   <223> primer
<400> 11
   agatatgatt gcggccctg 19
<210> 12
   <211> 26
   <212> DNA
   <213> Artificial sequence
<220>
   <223> primer for radiolabeling
<400> 12
   gggagcagca tggagccttc ggctga 26
<210> 13
   <211> 36
   <212> DNA
   <213> Artificial sequence
<220>
   <223> primer
<400> 13
   ggcgatagcc accacttggt ccggagtcag gccatg 36
<210> 14
   <211> 49
   <212> DNA
   <213> Artificial sequence
<220>
   <223> primer
<220>
   <221> misc_feature
   <222> (25)..(26)
   <223> n is a, c, g, or t
<400> 14
   gaccaagtgg tggctatcgc cagcnnkggc ggcaagcaag cgctcgaaa 49
<210> 15
   <211> 46
   <212> DNA
   <213> Artificial sequence
<220>
   <223> primer
<220>
   <221> misc_feature
   <222> (25)..(26)
   <223> n is a, c, g, or t
<400> 15
   gaccaagtgg tggctatcgc cagcnnkggc aagcaagcgc tcgaaa 46
<210> 16
   <211> 17
   <212> DNA
   <213> Artificial sequence
<220>
   <223> sequencing primer
<400> 16
   agaagcgcga tcacatg 17
<210> 17
   <211> 79
   <212> DNA
   <213> Artificial sequence
<220>
   <223> primer
<220>
   <221> modified_base
   <222> (67)..(67)
   <223> caC
<400> 17
   tggattccca ctcttcagcc ccagcgttac agcatcttca gtggcttctt ccaccgtgag 60
   ctcttccgtt tccacatcc 79
<210> 18
   <211> 79
   <212> DNA
   <213> Artificial sequence
<220>
   <223> primer
<220>
   <221> modified_base
   <222> (67)..(67)
   <223> fC
<400> 18
   tggattccca ctcttcagcc ccagcgttac agcatcttca gtggcttctt ccaccgtgag 60
   ctcttccgtt tccacatcc 79
<210> 19
   <211> 80
   <212> DNA
   <213> Artificial sequence
<220>
   <223> mutagenesis primer
<400> 19
   ttttccatgg cctgactccg gaccaagtgg tggctatcgc cagcggcggc ggcaagcaag 60
   cgctcgaaac ggtgcagcgg 80
<210> 20
   <211> 80
   <212> DNA
   <213> Artificial sequence
<220>
   <223> mutagenesis primer
<400> 20
   ttttctcgag ggtctcggtc ctggcacagc accggcaaca gccgctgcac cgtttcgagc 60
   gcttgcttgc cgccgccgct 80
<210> 21
   <211> 80
   <212> DNA
   <213> Artificial sequence
<220>
   <223> mutagenesis primer
<400> 21
   ttttccatgg cctgactccg gaccaagtgg tggctatcgc cagcggcggc aagcaagcgc 60
   tcgaaacggt gcagcggctg 80
<210> 22
   <211> 80
   <212> DNA
   <213> Artificial sequence
<220>
   <223> mutagenesis primer
<400> 22
   ttttctcgag ggtctcggtc ctggcacagc accggcaaca gccgctgcac cgtttcgagc 60
   gcttgcttgc cgccgctggc 80
<210> 23
   <211> 80
   <212> DNA
   <213> Artificial sequence
<220>
   <223> mutagenesis primer
<400> 23
   ttttccatgg cctgactccg gaccaagtgg tggctatcgc cagcggcaag caagcgctcg 60
   aaacggtgca gcggctgttg 80
<210> 24
   <211> 80
   <212> DNA
   <213> Artificial sequence
<220>
   <223> mutagenesis primer
<400> 24
   ttttctcgag ggtctcggtc ctggcacagc accggcaaca gccgctgcac cgtttcgagc 60
   gcttgcttgc cgctggcgat 80
<210> 25
   <211> 80
   <212> DNA
   <213> Artificial sequence
<220>
   <223> mutagenesis primer
<400> 25
   ttttccatgg cctgactccg gaccaagtgg tggctatcgc cagcaagcaa gcgctcgaaa 60
   cggtgcagcg gctgttgccg 80
<210> 26
   <211> 80
   <212> DNA
   <213> Artificial sequence
<220>
   <223> mutagenesis primer
<400> 26
   ttttctcgag ggtctcggtc ctggcacagc accggcaaca gccgctgcac cgtttcgagc 60
   gcttgcttgc tggcgatagc 80
<210> 27
   <211> 80
   <212> DNA
   <213> Artificial sequenec
<220>
   <223> mutagenesis primer
<400> 27
   ttttccatgg cctgactccg gaccaagtgg tggctatcgc caataagcaa gcgctcgaaa 60
   cggtgcagcg gctgttgccg 80
<210> 28
   <211> 80
   <212> DNA
   <213> Artificial sequence
<220>
   <223> mutagenesis primer
<400> 28
   ttttctcgag ggtctcggtc ctggcacagc accggcaaca gccgctgcac cgtttcgagc 60
   gcttgcttat tggcgatagc 80
<210> 29
   <211> 60
   <212> DNA
   <213> Artificial sequence
<220>
   <223> mutagenesis primer
<400> 29
   ttttccatgg cctgactccg gaccaagtgg tggctatcgc cagcggcggc ggcaagcaag 60
<210> 30
   <211> 60
   <212> DNA
   <213> Artificial sequence
<220>
   <223> mutagenesis primer
<400> 30
   ttttctcgag ggtctcctgc accgtttcga gcgcttgctt gccgccgccg ctggcgatag 60
<210> 31
   <211> 60
   <212> DNA
   <213> Artificial sequence
<220>
   <223> mutagenesis primer
<400> 31
   ttttccatgg cctgactccg gaccaagtgg tggctatcgc cagcggcggc aagcaagcgc 60
<210> 32
   <211> 60
   <212> DNA
   <213> Artificial sequence
<220>
   <223> mutagenesis primer
<400> 32
   ttttctcgag ggtctcctgc accgtttcga gcgcttgctt gccgccgctg gcgatagcca 60
<210> 33
   <211> 60
   <212> DNA
   <213> Artificial sequence
<220>
   <223> mutagenesis primer
<400> 33
   ttttccatgg cctgactccg gaccaagtgg tggctatcgc cagcggcaag caagcgctcg 60
<210> 34
   <211> 60
   <212> DNA
   <213> Artificial sequence
<220>
   <223> mutagenesis primer
<400> 34
   ttttctcgag ggtctcctgc accgtttcga gcgcttgctt gccgctggcg atagccacca 60
<210> 35
   <211> 60
   <212> DNA
   <213> Artificial sequence
<220>
   <223> mutagenesis primer
<400> 35
   ttttccatgg cctgactccg gaccaagtgg tggctatcgc cagcaagcaa gcgctcgaaa 60
<210> 36
   <211> 60
   <212> DNA
   <213> Artificial sequence
<220>
   <223> mutagenesis primer
<400> 36
   ttttctcgag ggtctcctgc accgtttcga gcgcttgctt gctggcgata gccaccactt 60
<210> 37
   <211> 60
   <212> DNA
   <213> Artificial sequence
<220>
   <223> mutagenesis primer
<400> 37
   ttttccatgg cctgactccg gaccaagtgg tggctatcgc caataagcaa gcgctcgaaa 60
<210> 38
   <211> 60
   <212> DNA
   <213> Artificial sequence
<220>
   <223> mutagenesis primer
<400> 38
   ttttctcgag ggtctcctgc accgtttcga gcgcttgctt attggcgata gccaccactt 60
<210> 39
   <211> 45
   <212> DNA
   <213> Artificial sequence
<220>
   <223> primer
<400> 39
   cttcctcttc cgtctctttc cttttacgtc atccgggggc agact 45
<210> 40
   <211> 45
   <212> DNA
   <213> Artificial sequence
<220>
   <223> primer
<220>
   <221> modified_base
   <222> (27)..(27)
   <223> mC
<400> 40
   cttcctcttc cgtctctttc cttttacgtc atccgggggc agact 45
<210> 41
   <211> 45
   <212> DNA
   <213> Artificial sequence
<220>
   <223> primer
<220>
   <221> modified_base
   <222> (11)..(11)
   <223> hmC
<400> 41
   cttcctcttc cgtctctttc cttttacgtc atccgggggc agact 45
<210> 42
   <211> 45
   <212> DNA
   <213> Artificial sequence
<220>
   <223> primer
<220>
   <221> modified_base
   <222> (27)..(27)
   <223> hmC
<400> 42
   cttcctcttc cgtctctttc cttttacgtc atccgggggc agact 45
<210> 43
   <211> 45
   <212> DNA
   <213> Artificial sequence
<220>
   <223> primer
<220>
   <221> modified_base
   <222> (27)..(27)
   <223> fC
<400> 43
   cttcctcttc cgtctctttc cttttacgtc atccgggggc agact 45
<210> 44
   <211> 45
   <212> DNA
   <213> Artificial sequence
<220>
   <223> primer
<220>
   <221> modified_base
   <222> (27)..(27)
   <223> caC
<400> 44
   cttcctcttc cgtctctttc cttttacgtc atccgggggc agact 45
<210> 45
   <211> 45
   <212> DNA
   <213> Artificial sequence
<220>
   <223> primer
<400> 45
   agtctgcccc cggatgacgt aaaaggaaag agacggaaga ggaag 45
<210> 46
   <211> 46
   <212> DNA
   <213> Artificial sequence
<220>
   <223> primer
<400> 46
   ggccagccag tcagccgaag gctccatgct gctccccgcc gccggc 46
<210> 47
   <211> 46
   <212> DNA
   <213> Artificial sequence
<220>
   <223> primer
<220>
   <221> modified_base
   <222> (16)..(16)
   <223> mC
<400> 47
   ggccagccag tcagccgaag gctccatgct gctccccgcc gccggc 46
<210> 48
   <211> 46
   <212> DNA
   <213> Artificial sequence
<220>
   <223> primer
<220>
   <221> modified_base
   <222> (16)..(16)
   <223> hmC
<400> 48
   ggccagccag tcagccgaag gctccatgct gctccccgcc gccggc 46
<210> 49
   <211> 46
   <212> DNA
   <213> Artificial sequence
<220>
   <223> primer
<220>
   <221> modified_base
   <222> (16)..(16)
   <223> fC
<400> 49
   ggccagccag tcagccgaag gctccatgct gctccccgcc gccggc 46
<210> 50
   <211> 46
   <212> DNA
   <213> Artificial sequence
<220>
   <223> primer
<220>
   <221> modified_base
   <222> (16)..(16)
   <223> caC
<400> 50
   ggccagccag tcagccgaag gctccatgct gctccccgcc gccggc 46
<210> 51
   <211> 46
   <212> DNA
   <213> Artificial sequence
<220>
   <223> primer
<400> 51
   agggcggtgt ggggggcagg tggggaggag cccagtcctc cttcct 46
<210> 52
   <211> 28
   <212> DNA
   <213> Artificial sequence
<220>
   <223> primer for radiolabeling
<400> 52
   gcccccggat gacgtaaaag gaaagaga 28

## Description

The present invention relates to methods for determining the presence or absence of a particular cytosine nucleobase, selected from the group consisting of cytosine (C), 5-methylcytosine (mC), 5-hydroxymethylcytosine (hmC), 5-formylcytosine (fC), and 5-carboxylcytosine (caC), in a DNA molecule. Said method employs specifically selected and/or tailored transcription-activator-like effector (TALE) proteins in combination with specific modification reactions for the conversion of the cytosine nucleobase. Further, the present invention relates to related methods for the enrichment of DNA molecules in which a cytosine nucleobase of interest is present or absent, as well as for the differential digestion of DNA molecules in which a cytosine nucleobase of interest is present or absent.

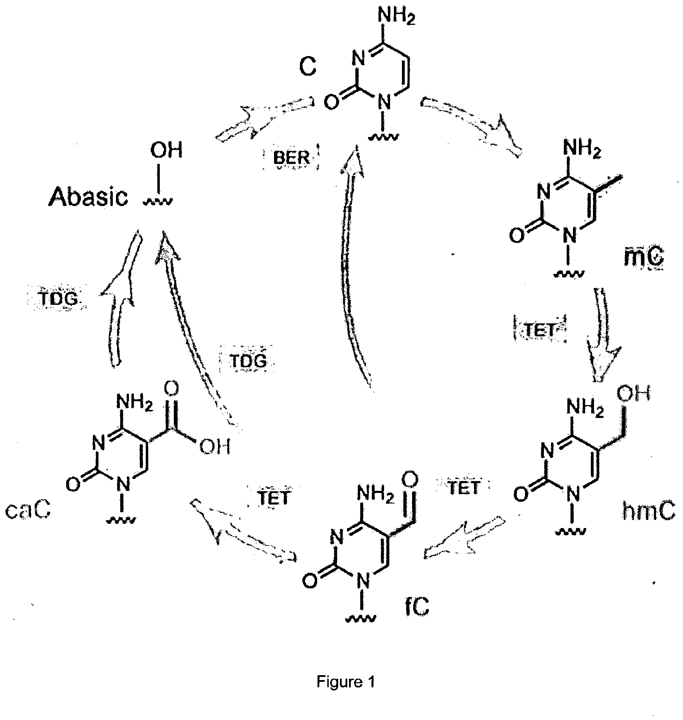

The phenotypic diversity of cells is determined by differences in gene expression profiles. Central regulatory elements of gene expression are epigenetic DNA modifications that affect local transcriptional activity of the genome by controlling the chromatin structure. Among these, methylation of DNA cytosine (C) nucleobases at the carbon 5-position (5-methylcytosine or mC, the "fifth base", Fig. 1) is the most common modification in vertebrates.

Key to the epigenetic regulation of gene expression is the introduction and removal of 5-methyl-groups. However, while the process of C-methylation is relatively well characterized, a potential process of active mC-demethylation has long remained elusive. Recently, ten-eleven-translocation (TET) family dioxygenases have been discovered to iteratively oxidize mC to 5-hydroxymethylcytosine (hmC, the "sixth base"), 5-formylcytosine (fC) and 5-carboxylcytosine (caC). fC and caC can be excised and replaced with C via thymine DNA glycosylase (TDG)-mediated base excision repair (BER, Fig. 1). C-methylation, oxidation and repair now offer a model for the long searched, complete cycle of dynamic cytosine modification, with paramount importance for gene expression regulation and cell fate (Fig. 1).

Importantly, besides being mC-demethylation intermediates, hmC, fC, and caC are increasingly recognized to have inherent biological functions, and thus considerably augment the biologically instructive information potential of the human genome: all three nucleobases exhibit unique cell-type specific occurrences, genomic distribution patterns and ability to recruit DNA-binding proteins. fC has been shown to be present at high densities at specific loci and to physically distort the DNA duplex structure in this setting. Both fC and caC have been shown to control eukaryotic RNA polymerase *in vitro.* Finally, hmC is particularly stable and abundant, and exhibits strong, wide-spread differences between cancer and non-cancer tissue, making it a novel biomarker for various cancers.

The aforementioned findings have created a pressing need for simple and effective methods that can delineate both regional patterns (typing) and broader profiles (profiling) of hmC, fC, caC, as well as other epigenetically modified cytosine nucleobases, in genomes with maximal resolution. These will help to address important scientific questions regarding the involvement of hmC and others in gene expression regulation and disease development:
1) How is the presence of hmC at specific genomic loci correlated with the presence of DNA-binding proteins that may serve as readers of hmC? This will help to understand if and in what way hmC is actively involved in transcription control and what exact mechanisms underlie the active demethylation pathway.
2) What specific TET proteins are involved in the oxidation of mC to hmC in different cell types, developmental stages, disease processes and promoter contexts? This will elucidate the individual functions of TET proteins and reveal their potential use as drugs or drug targets (in a similar way as modulation of DNA methyl transferase (DNMT) activity is used in cancer therapy).
3) If a global reduction of genomic hmC content is a hallmark of cancer, is this also true for specific cancer-related loci, and are there specific patterns of hmC that can serve as biomarkers in a similar way as mC patterns do? This will have important implications for the potential development of diagnostic applications based on hmC as cancer biomarker.

The key component of current approaches for the *in vitro* typing and profiling of hmC is the strategy used for the actual differentiation (e.g. the selective recognition or conversion) of hmC from other nucleobases in the genome. This step dictates the analytical value of the approach in terms of nucleobase selectivity, sequence resolution, level quantitativeness and technical ease. Ideal characteristics of this step are:
1) Full nucleobase selectivity (requiring atomic resolution, since C, mC, hmC, fC, and caC differ in the presence of only one atom in several cases),
2) single nucleotide sequence resolution (the ability to assess the presence of a nucleobase at only one, user-defined position in the genome),
3) fully quantitative, unbiased level analysis (*i.e.,* the ability to quantitatively assess the presence of multiple, different nucleobases at the position), and
4) maximal technical ease, *i.e.,* an assay setup that is as direct as possible, is scalable in multiplexing, and can easily be combined with effective approaches of DNA analysis.

However, though a variety of typing and profiling approaches for hmC have been introduced in recent years, none of these ideally meets these requirements. A central reason for this is a long-standing dilemma of DNA recognition: Canonical nucleobases can be recognized in a fully programmable manner (by Watson-Crick hybridization), and this simplicity has enabled the development of various powerful approaches for canonical nucleobase analysis that all fully meet the aforementioned characteristics.

In contrast, epigenetically modified nucleobases cannot be recognized in a programmable manner, since they are not revealed by Watson-Crick-hybridization and, thus, current routine methods for epigenetic nucleobase analysis are only modification-specific, but not directly sequence specific. This prevents the satisfactory fulfillment of the aforementioned characteristics and results in comparably complicated and/or poorly resolved approaches.

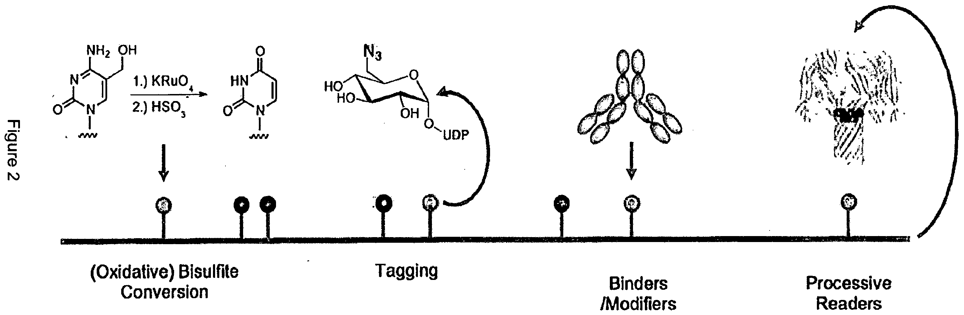

A major group of hmC differentiation approaches is based on chemical conversion (Fig. 2), that is, on the selective deamination of C (but not mC/hmC) with bisulfite and subsequent analysis of the resulting C to uridine (U) mutation (e.g. bisulfite sequencing, BS). This can be coupled with oxidation by KRuO₄ (used in "oxBS") that converts hmC to fC, whereas mC remains unchanged. fC is converted to U in bisulfite treatments, and thus hmC and mC can be indirectly differentiated in comparative assays. Alternatively, hmC can be glucosylated to β-glucosyl-hmC (ghmC) by T4 beta-glucosyltransferase (T4 BGT) and then reads as C after bisulfite treatment. If this is coupled with an oxidation step using a TET dioxygenase, as in the TAB-Seq assay (TET-assisted bisulfite sequencing), all other C-modifications will be oxidized to caC and then read as U after bisulfite treatment. Though bisulfite-based approaches offer single nucleotide resolution and quantitative level analysis, they are laborious, time-consuming (i.e. > 4-16 h), require harsh, DNA destructing reaction conditions, and reduce the sequence complexity of the sample, which interferes with downstream processing and bioinformatic mapping. These drawbacks prevent the development of direct and simple *in vitro* assays.

A second group of hmC differentiation approaches relies on affinity enrichment, for example, via T4 BGT-catalyzed glucosylation of hmC with azide-containing substrates (tagging, Fig. 2) and subsequent genome-wide enrichment via Huisgen 1,3-dipolar azide-alkyne cycloaddition with biotin-reagents and streptavidine bead binding, *i.e.,* in the hMe-Seal assay. Though relatively simple, this approach suffers from a poor resolution that is defined by the fragment size of the DNA sample (typically >200-300 nt), since any hmC in the fragment results in a positive signal. Similarly, current proteins that selectively recognize hmC directly or modify DNA conditionally on its presence (binders/modifiers, Fig. 2), exhibit no or constraint sequence-selectivity and can be biased in their affinity (e.g. antibodies in the hMe-DIP assay, restriction enzymes). This again results in poor resolution and non-quantitative data.

Finally, protein nanopores and DNA polymerases have been shown to be able to sense hmC (processive readers, Fig. 2), and, thus, in principle offer a direct and combined analysis of canonical and epigenetic nucleobases. However, though highly appealing for sequencing, these approaches are restricted to technically demanding single molecule techniques, and their routine applicability for genomic hmC analysis remains to be demonstrated.

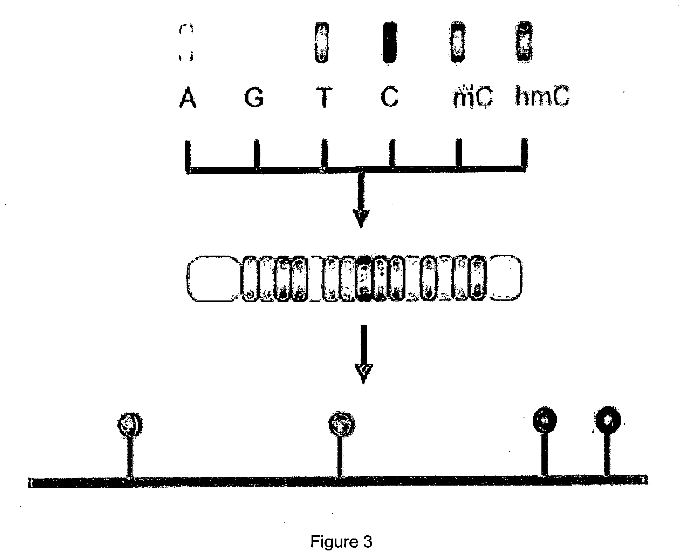

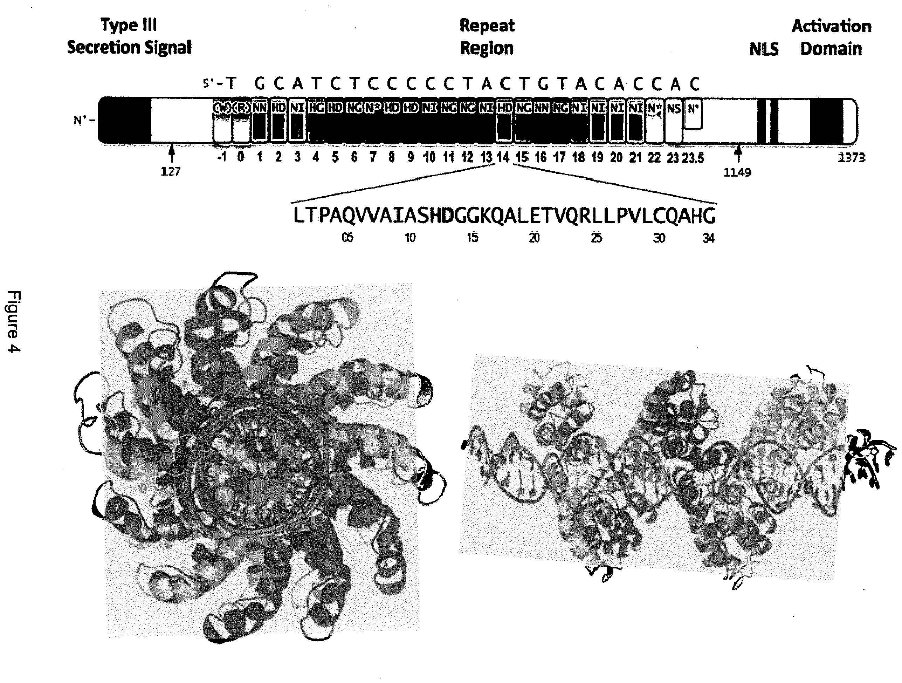

To overcome the aforementioned dilemma and to provide a strategy for epigenetic nucleobase analysis that meets the ideal characteristics of differentiation, the concept of an "expanded programmability of DNA recognition" has been recently introduced. This allows for the direct, programmable recognition of both canonical and epigenetic nucleobases using a single molecular scaffold (Fig. 3), and thus provides a universal and flexible platform for epigenetic nucleobase analysis. The concept is based on transcription-activator-like effectors (TALEs). TALEs feature a central DNA-binding domain that consists of multiple concatenated and interchangeable repeats, each of which recognizes one of the four canonical DNA nucleotides. This recognition mode is fully programmable. Fig. 4 shows an exemplary representation of the several structural components of TALEs.

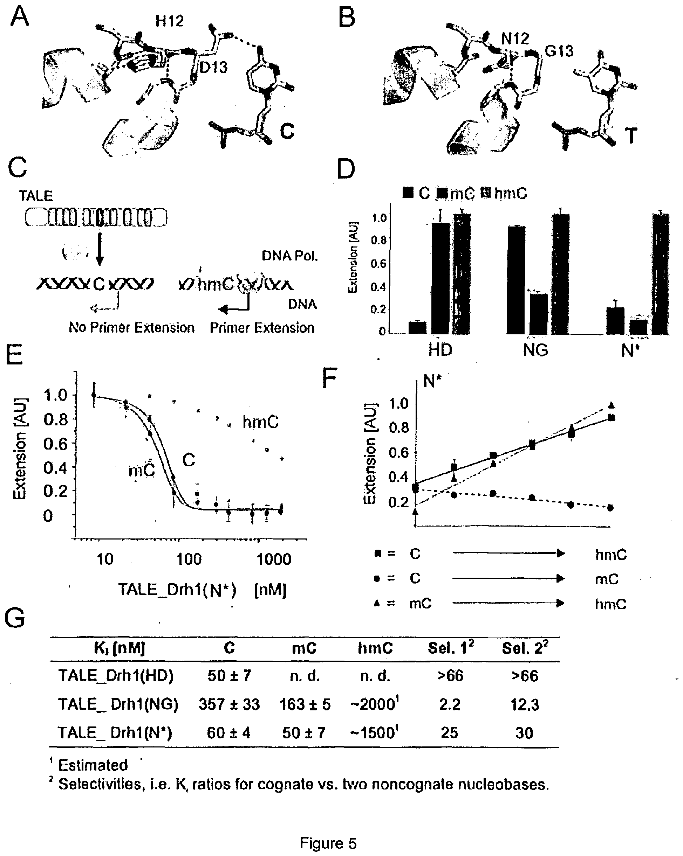

TALE repeats consist of 34 amino acids (aa) arranged in two α-helices connected by a small loop. Most of these aa are highly conserved, whereas two aa of the loop are variable (repeat variable diresidue, RVD), and these are responsible for selective nucleobase binding. In the majority of natural TALEs, aa 12 (numbering within the repeat) is an H or N residue (one-letter aa code) that makes a stabilizing intra-loop hydrogen bond. The second residue (aa 13) makes a specific contact to the nucleobase. This recognition follows a simple code, with the RVDs NI, NN (NH), HD and NG binding the nucleotides A, G, C, and T, respectively (Figs. 5A and 5B show binding of C by RVD HD, and of T by RVD NG, respectively). TALEs thereby wind into the DNA major groove, where consecutive repeats bind all nucleotides of the forward DNA strand. The programmability of TALEs by simple concatenation of individual repeats has extensively been proven in various sequence contexts and does not exhibit pronounced context dependences or sequence constraints.

The first genomic *in vitro* assay for the direct, programmable detection of single mC positions in a large eukaryotic genome (zebrafish) has recently been introduced, based on the sensitivity of RVD HD to mC. This assay is based on an mC-dependent, competitive inhibition of DNA polymerase-catalyzed primer extension by the TALE (Fig. 5C). Very high selectivities, e.g. up to a >66-fold difference in the assay read-out have been observed for only a single mC in a 17 nt target DNA sequence. Moreover, a highly robust differentiation ability by RVD HD was observed, *i.e.,* with little dependence on the target sequence composition, sequence length (16-20 nt), the position within the TALE-DNA complex (for central and N-terminal repeats) and on the direct sequence context around the mC.

As a further step towards an expanded programmability of DNA recognition, RVDs HD, NG, and N* (* = aa deletion) have recently been identified to provide individual selectivities for C, mC, and hmC (Fig. 5D). This approach was based on exploiting the increasing steric demand of the 5-modifications of C, mC, and hmC for selective recognition by RVDs with gradually decreasing size (decreasing size = deletion of an aa side chain from HD to NG, and deletion of the whole aa from NG to N*). TALEs bearing repeats with RVD N* thereby provided high selectivities for single hmC in the target DNA (Figs. 5E and G, 25- and 30-fold selectivity on basis of Ki; towards C and mC, respectively). Moreover, hmC could be selectively and quantitatively detected even in mixtures of DNAs containing hmC and C or mC at single positions (Fig. 5F). This is important, since biological samples often exhibit heterogeneous C modification. These data show for the first time that TALE repeats can be designed that enable differentiation not only between C and mC, but between all three targeted C-modifications, including the fully selective sensing of hmC.

Nevertheless, despite this successful proof of principle in model DNAs, a fully selective typing/profiling of hmC and other epigenetically modified cytosine nucleobases in genomic DNA requires maximal selectivity in the context of all modifications, e.g. including fC and caC that have similar steric demands.

WO2014206568 discloses methods of detecting and distinguishing a 5-hydroxymethyl modification from a 5-methyl modification of cytosine in a DNA molecule using TALE proteins that specifically bind the different forms of cytosine.

Dong Deng et al (Cell Research, vol.22, no.10, pp.1502-1504, 2012) discloses differentiation of mC from C by using a TALE protein with the diresidue NG instead of HD.

Accordingly, the technical problem underlying the present invention is to provide methods for the selective typing/profiling of cytosine nucleobases in DNA molecules. These methods should offer full nucleobase selectivity at atomic resolution, single nucleotide sequence resolution, *i.e.,* the ability to assess the presence of a nucleobase at only one, user-defined position in the genome, fully quantitative, unbiased level analysis, *i.e*., the ability to quantitatively assess the presence of multiple, different nucleobases at the position, and maximal technical ease, *i.e.,* an assay setup that is as direct as possible, is scalable in multiplexing, and can easily be combined with effective approaches of DNA analysis.

The solution to the above technical problem is achieved by the embodiments characterized in the claims.

In particular, in a first aspect, the present invention relates to a method for directly determining the presence or absence of a particular cytosine nucleobase of interest, selected from the group consisting of cytosine (C), 5-methylcytosine (mC), 5-hydroxymethylcytosine (hmC), 5-formylcytosine (fC), and 5-carboxylcytosine (caC), in a DNA molecule, comprising the steps of:
(a) providing a transcription-activator-like effector (TALE) protein, said TALE protein being capable of binding in a sequence-specific manner to a region of said DNA molecule that includes said cytosine nucleobase of interest;
(b) providing a first aliquot (aliquot 1) of said DNA molecule and subjecting said first aliquot to a modification reaction that selectively modifies the cytosine nucleobase of interest at the 5-position, wherein the presence of said chemical modification at the 5-position of the cytosine nucleobase of interest alters the binding affinity of said TALE protein to the DNA molecule; and
(c) subsequently determining the binding affinity of said TALE protein to said region of said DNA molecule in the first aliquot;
   wherein
   (i) the cytosine nucleobase of interest is present when the binding affinity of said TALE protein to said region of said DNA molecule in the first aliquot (first binding affinity) is different from the binding affinity of said TALE protein to said region of a DNA molecule that has not been subjected to said modification reaction (second binding affinity); and
   (ii) the cytosine nucleobase of interest is not present when the first binding affinity is not different from the second binding affinity.

As used herein, the terms "directly determining" or "direct determination" relate to the fact that with the method of the present invention, a cytosine nucleobase of interest in a DNA molecule can be detected without the need for any pretreatment of the DNA molecule in addition to the modification reaction performed in step (b) of the method of the present invention. Accordingly, said DNA molecule originally is preferably in its native state. Thus, the method of the present invention is significantly less time-, labor- and cost-intensive compared to methods known in the art relying on pretreatments of the nucleic acid molecule. Moreover, the method of the present invention is highly sensitive and thus allows the detection of cytosine nucleobases with high resolution, *i.e.* at single nucleobase sites. Finally, the method allows the detection of cytosine nucleobases at user-defined sites, owing to the programmability of sequence recognition of TALE proteins that exceeds the programmability of any other known protein.

As used herein, the term "cytosine nucleobase" includes cytosine (C), 5-methylcytosine (mC), 5-hydroxymethylcytosine (hmC), 5-formylcytosine (fC), and 5-carboxylcytosine (caC).

According to the present invention, the DNA molecule may be single-stranded or double-stranded, wherein double-stranded DNA is preferred.

In preferred embodiments of the method of the present invention, the repeat variable diresidue (RVD) of said TALE protein whose position corresponds to the position of the cytosine nucleobase of interest is an RVD having one or more amino acid deletions. In particular, size reduced RVDs can have a deletion of 1, 2, 3, 4, 5, 6, 7, 8, 9, or 10 amino acids. Preferably, size reduced RVDs comprise RVDs X*, wherein X is any proteinogenic amino acid, and * is an amino acid deletion. Other preferred size reduced RVDs are X**, e.g. G**, and RVDs wherein the amino acids at positions 12, 13, and 14 are deleted (3*), e.g. an RVD wherein amino acids 11 to 14 read S***G.

The term "cytosine nucleobase of interest" as used herein points to the fact that with the method of the present invention, any particular position in a given DNA molecule can be analyzed for determining the presence or absence of said nucleobase. This is achieved by providing a TALE protein in step (a) of said method which is capable of binding in a sequence-specific manner to a region of said DNA molecule that includes said cytosine nucleobase of interest. This binding capability is irrespective of whether the particular cytosine nucleobase is present or absent.

In this context, TALE proteins (TALEs) to date represent the protein scaffold with the highest modularity and flexibility of sequence-specific nucleic acid recognition. They consist of concatenated single modules that each recognize one nucleotide in the target nucleic acid. These modules can be flexibly connected to recognize any arbitrary nucleic acid sequence of interest. The recognition is achieved by only two amino acid residues (repeat variable diresidue, RVD) within each module, providing a "code" of recognition. The most widely used TALE codes are NG for T, NI for A, NN, NK, NH and HN for G and HD for C.

As indicated above, TALEs consist of concatenated modules, each of which recognizes a canonical nucleobase in DNA and this recognition mode is fully programmable. This makes TALEs the first molecules that hold potential for the direct, fully programmable differentiation of individual epigenetic modifications both *in vitro* and *in vivo.* This offers considerable simplifications and performance improvements for typing/profiling *in vitro* as compared to current approaches.

TALE proteins have recently been discovered as a new scaffold for the design of DNA binding domains with programmable sequence selectivity. Besides fixed N- and C-terminal domains, TALEs feature a central DNA-binding domain that consists of concatenated, interchangeable modules each of which recognizes one of the four canonical DNA nucleotides. These modules consist of 34 amino acids (aa) arranged in two α-helices connected by a small loop. Most of these aa are highly conserved, whereas two aa of the loop are variable (the repeat variable diresidue, RVD) and are responsible for selective nucleobase binding. In the majority of natural TALEs, aa 12 (numbering in the module) is an H or N residue (one-letter aa code) that makes a stabilizing intra-loop hydrogen bond. The second residue (aa 13) makes a specific contact to the edge of the nucleobase through the major groove of the DNA duplex. This results in a winding of the TALE into the DNA groove, forming a super-helical structure, where consecutive TALE modules bind all nucleotides of the forward DNA strand. The nucleotide recognition by TALE RVDs follows a simple code, with the RVDs NI, NN, HD and NG binding the nucleotides A, G, C, and T, respectively. The programmability of TALEs by facile concatenation of individual modules has extensively been proven in various sequence contexts and does not exhibit pronounced context dependencies or sequence constraints.

Interestingly, RVD NG binds mC better than C, and RVDs having one or more amino acid deletions, in particular RVD N* (* = aa deletion), binds C and mC with comparable affinities. This demonstrates that it is possible to design universal TALE modules that are able to ignore certain C-modifications at user-defined positions of a target. The present invention further expands this concept to cytosine nucleobases in general. In particular, RVDs that bind to a cytosine nucleobase irrespective of the modification status of said nucleobase have been discovered.

Thus, in the method of the present invention, TALE proteins can be designed (or suitable naturally occurring TALE proteins provided) that are capable of binding in a sequence-specific manner to a region of said DNA molecule that includes said cytosine nucleobase of interest. In this context, the term "capable of binding in a sequence-specific manner to a region of said DNA molecule that includes said cytosine nucleobase of interest" as used herein relates to the fact that the TALE protein is capable of binding to said region irrespective of which particular cytosine nucleobase is present. In other words, said term relates to the fact that the TALE protein's sequence specificity is directed to the nucleotide sequence of said region wherein the precise nature of the cytosine nucleobase of interest is not (yet) known. In a preferred embodiment, the region to which said TALE protein is capable of binding in a sequence-specific manner is from 10 to 40 nucleotides in length, more preferably from 15 to 30 nucleotides in length, and most preferably from 18 to 26 nucleotides in length. Methods for the design and expression of TALE proteins are not particularly limited and are known in the art.

In step (b) of the method of the present invention, a first aliquot of the DNA molecule is provided and subjected to a modification reaction that selectively modifies the cytosine nucleobase of interest at the 5-position, wherein the presence of said chemical modification at the 5-position of the cytosine nucleobase of interest alters the binding affinity of said TALE protein to said DNA molecule.

In this context, the term "modification reaction that selectively modifies the cytosine nucleobase of interest at the 5-position" as used herein relates to any reaction that changes the chemical structure of the substituent at the C5 carbon atom.

According to the present invention, the modification reaction is selective for the cytosine nucleobase of interest, *i.e.,* the modification reaction specifically accepts only the cytosine nucleobase of interest as substrate, starting material or reactant, but not any other cytosine nucleobase. Respective modification reactions for a given cytosine nucleobase are not particularly limited and are known in the art.

According to the present invention, the chemical modification introduced into the 5-position of the cytosine nucleobase of interest alters the binding affinity of the TALE protein to the DNA molecule. This can be an alteration in the binding affinity ranging from a complete abolishment of binding over a mere weakening of the binding affinity to a strengthening of the binding affinity. Suitable chemical modifications in this respect are not particularly limited and are known in the art.

According to the present invention, TALE proteins and RVDs thereof are suitably selected with respect to suitable modification reactions to provide the above alteration of binding affinity in case the cytosine nucleobase of interest is present, *i.e.,* in case the selective modification reaction occurs.

In preferred embodiments, the alteration of the binding affinity is an abolishment of binding or a significant weakening of the binding affinity. In such a case, determining the first and second binding affinities in step (c) of the methods of the present invention can comprise a mere qualitative determination, *i.e.,* a determination of whether the TALE proteins binds at all or does not bind to the DNA molecule. In this context, cytosine nucleobases, respective TALE proteins that bind a DNA molecule irrespective of what particular cytosine nucleobase is present, and respective modification reactions selectively modifying the cytosine nucleobase of interest at the 5-postion, wherein the presence of said chemical modification at the 5-position of the cytosine nucleobase of interest alters the binding affinity of said TALE protein to said DNA molecule, are indicated hereinafter.

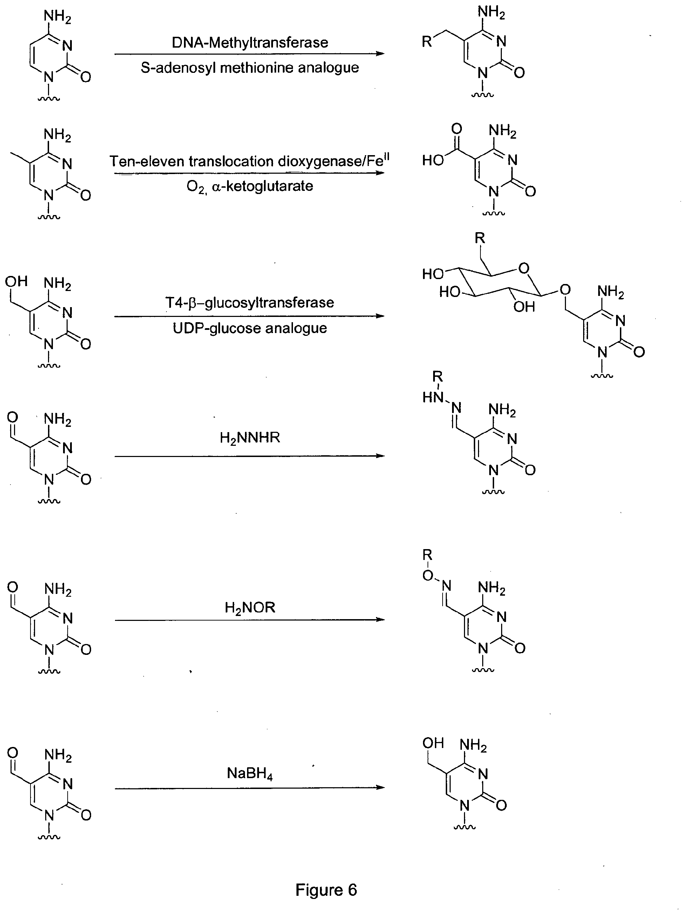

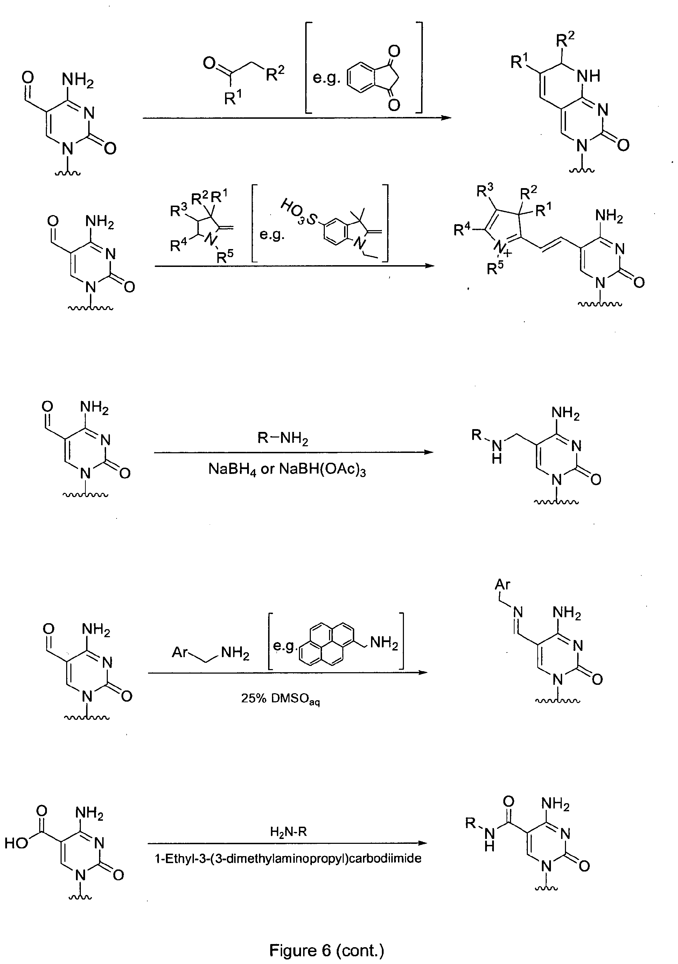

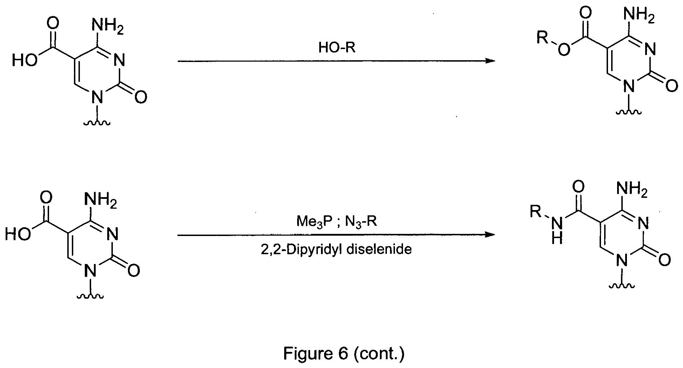

In particular, the cytosine nucleobase of interest can be C, the RVD of said TALE protein whose position corresponds to the position of the cytosine nucleobase of interest is selected from the group of HD or ND, and the modification reaction is the enzymatic conversion of C to a 5-modified C using a natural or engineered DNA-methyltransferase (Dnmt) in the presence of a S-adenosylmethionine (SAM, Adomet) or SAM analog as cofactor that donates a bulky alkyl-group to the 5-position of C or transfers the cofactor to the 5-position as known from aziridine analogs or mustard analogs (Fig. 6). Suitable SAM analogs are not particularly limited and are known in the art. Examples of suitable SAM analogs include SAM analogs wherein the methyl group at the sulfur atom is exchanged against larger saturated or unsaturated alkyl groups, e.g. the groups -CH₂CH₃, -CH₂CH₂CH₃, -CH₂CH=CH₂ or -CH₂C≡CCH₃. Further suitable SAM analogs include SAM analogs wherein the methylsulfonium moiety is replaced by an aziridine ring.

Further, the cytosine nucleobase of interest can be mC, the RVD of said TALE protein whose position corresponds to the position of the cytosine nucleobase of interest is selected from the group consisting of N*, S*, NG, HG, Q*, H*, K*, R*, L*, G*, E*, I*, F*, A*, C*, V*, Y*, D*, M*, P*, T*, and G**; and the modification reaction is the enzymatic conversion of mC to caC via hmC and fC as intermediates (Fig. 1), using ten-eleven-translocation (TET)-dioxygenase in the presence of α-ketoglutarate, Fe²⁺ and oxygen (Fig. 6).

Further, the cytosine nucleobase of interest can be hmC, the RVD of said TALE protein whose position corresponds to the position of the cytosine nucleobase of interest is selected from the group consisting of N*, G*, and 3*, wherein 3* is particularly preferred; and the modification reaction is the enzymatic conversion of hmC to 5-glucosyl-hmC (ghmC) using T4 β-glucosyl-transferase (T4 BGT) in the presence of uridine diphosphate (UDP)-glucose or an analog thereof (Fig. 6). Suitable UDP-glucose analogs are not particular limited and are known in the art. One specific UDP-glucose in this respect is azidoglucose that can be modified post-transfer by Copper Click chemistry.

Furthermore, the cytosine nucleobase of interest can be fC, the RVD of said TALE protein whose position corresponds to the position of the cytosine nucleobase of interest is selected from the group consisting of NG, N*, G*, and 3*, wherein 3* is particularly preferred; and the modification reaction is the conversion of fC to an oxime-modified cytosine using a hydroxylamine optionally in the presence of a suitable activator or catalyst such as anilines, to a hydrazone-modified cytosine using a hydrazine, to a substituted alkene using methylene components that react via aldol-addition/condensation-type reactions, or to an amine by reductive amination using an amine and a reductant (Fig. 6). Preferably, the hydroxylamine has the formula H₂NOR, and/or the hydrazine has the formula H₂NNHR and/or the amine has the formula R-NH₂, wherein R is selected from the group consisting of any residue that is directly connected to the stated groups H₂NO- or H₂NNH- via a carbon atom. This includes e.g. saturated and unsaturated alkyl or aryl groups with or without heteroatoms. The reductant is preferably a hydride donor, e.g. NaBH₄.

Moreover, the cytosine nucleobase of interest can be fC, the RVD of said TALE protein whose position corresponds to the position of the cytosine nucleobase of interest is selected from the group consisting of NG, N*, S*, Q*, H*, K*, R*, L*, E*, F*, G*, A*, C*, D*, M*, T*; and the modification reaction is the conversion, *i.e.,* reduction, of fC to hmC using a reductant. The reductant is preferably a hydride donor, e.g. NaBH₄ (Fig. 6).

Finally, the cytosine nucleobase of interest can be caC, the RVD of said TALE protein whose position corresponds to the position of the cytosine nucleobase of interest is selected from the group consisting of N*, G*, and 3*, wherein 3* is particularly preferred; and the modification reaction is the conversion of caC to an amide-modified cytosine using an amine or the conversion of caC to an ester-modified cytosine using an alcohol in the presence of a suitable activator or catalyst such as 1-ethyl-3-(3-dimethylaminopropyl)carbodiimid (EDC) (Fig. 6). Preferably, the amine has the formula H₂NR, wherein R is selected from the group consisting of any residue that is directly connected to the stated groups H₂NO- or H₂NNH- via a carbon atom. This includes e.g. saturated and unsaturated alkyl or aryl groups with or without heteroatoms.

In particularly preferred embodiments, the modification reactions as described above are the modification reactions depicted in Fig. 6, wherein R, R¹, R², R³, R⁴, R⁵ are independently selected from the group consisting of any residue that is directly connected to the stated groups via a carbon atom, and Ar is any aryl unit. This includes e.g. saturated and unsaturated alkyl or aryl groups with or without heteroatoms.

In the context of TALE protein RVDs as indicated above, * signifies an amino acid deletion. For sake of clarity, RVDs in the form of X*, wherein X is any proteinogenic amino acid are still designated as RVDs (repeat variable diresidues), although actually only one amino acid is present. Similarly, RVD G** as indicated above signifies a situation wherein amino acid 12 (numbering within the TALE module) is G, amino acid 13 is deleted, and in addition amino acid 14 is deleted. Although only one amino acid is present, and also the deletion of amino acid 14 is defined, this is still referred to as an RVD herein. Further, RVD 3* as indicated above signifies a situation wherein amino acids 12, 13, and 14 are deleted. Although no amino acid is present, and the deletion of three amino acids is defined, this is still referred to as an RVD herein.

In step (c) of the method of the present invention, the binding affinity of said TALE protein to said region of said DNA molecule in the first aliquot is determined, wherein
(i) the cytosine nucleobase of interest is present when the binding affinity of said TALE protein to said region of said DNA molecule in the first aliquot (first binding affinity) is different from the binding affinity of said TALE protein to said region of a DNA molecule that has not been subjected to said modification reaction (second binding affinity); and
(ii) the cytosine nucleobase of interest is not present when the first binding affinity is not different from the second binding affinity.

In this context, the above second binding affinity can be a reference value for the particular DNA molecule and TALE protein that has been determined independently prior to performing the method of the present invention. Alternatively, said second binding affinity can be determined concurrently with the method of the present invention, e.g. by providing a second aliquot (aliquot 2) of said DNA molecule that is not subjected to the modification reaction in step (b) of the method, and subsequently determining the binding affinity of said TALE protein to said region of said DNA molecule in the second aliquot in addition to determining the binding affinity of said TALE protein to said region of said DNA molecule in the first aliquot in step (c) of the method.

Thus, in an embodiment wherein said second binding affinity is determined concurrently with the method of the present invention, said method comprises the steps of
(a) providing a transcription-activator-like effector (TALE) protein, said TALE protein being capable of binding in a sequence-specific manner to a region of said DNA molecule that includes said cytosine nucleobase of interest;
(b) providing a first aliquot (aliquot 1) and a second aliquot (aliquot 2) of said DNA molecule and subjecting said first aliquot to a modification reaction that selectively modifies the cytosine nucleobase of interest at the 5-position, wherein the presence of said chemical modification at the 5-position of the cytosine nucleobase of interest alters the binding affinity of said TALE protein to the DNA molecule; and
(c) subsequently determining the binding affinity of said TALE protein to said region of said DNA molecule in the first aliquot and in the second aliquot;
   wherein
   (iii) the cytosine nucleobase of interest is present when the binding affinity of said TALE protein to said region of said DNA molecule in the first aliquot (first binding affinity) is different from the binding affinity of said TALE protein to said region of said DNA molecule in the second aliquot (second binding affinity); and
   (iv) the cytosine nucleobase of interest is not present when the first binding affinity is not different from the second binding affinity.

In a preferred embodiment, step (c) of the method of the present invention is performed under conditions that allow the maximizing of the difference between the first and second binding affinity. Respective conditions are known and can be chosen by the person skilled in the art.

Further, the first binding affinity is considered to be different from the second binding affinity in the sense of the present invention in case said affinities differ by at least 5%, more preferably by at least 10%, at least 20%, at least 30%, at least 50% or more. Vice versa, the first binding affinity is considered to be not different from the second binding affinity in case said affinities differ by at most 5%, more preferably at most 3%, at most 2%, at most 1% or less.

As indicated above, in preferred embodiments, the alteration of the binding affinity is an abolishment of binding or a significant weakening of the binding affinity. In this context, the first binding affinity as defined above is preferably 50%, more preferably 40%, more preferably 30%, more preferably 20%, more preferably 10%, more preferably 5%, more preferably 2%, and most preferably 1% or less of the second binding affinity.

Methods for determining the binding affinity of a TALE protein to a DNA molecule are not particular limited and are known in the art. Suitable methods include affinity enrichment and primer extension assays with subsequent quantification of the generated DNA, e.g. via qPCR, next generation sequencing, microarrays, rolling circle amplification, or thyramid signal amplification.

In cases wherein the alteration of the binding affinity is an abolishment of binding or a significant weakening of the binding affinity, step (c) of the method of the present invention can comprise the step of determining whether a specific DNA polymerase reaction using a primer that is capable of binding to the region of said DNA molecule that includes said cytosine nucleobase of interest is inhibited or not, thus indicating binding of said TALE protein to said region in case of inhibition. In this context, binding of the TALE protein to a DNA molecule with a strongly reduced affinity does not inhibit the above specific DNA polymerase reaction using a primer that is capable of binding to the region of said DNA molecule that includes said nucleobase residue of interest.

In this embodiment, step (c) of the method of the present invention preferably comprises the steps of:
(c1) providing a primer that is capable of binding to the region of said DNA molecule that includes said nucleobase of interest;
(c2) providing a first aliquot of the first aliquot provided in step (b) (aliquot 1-1), and adding said TALE protein to said aliquot;
(c3) performing a specific DNA polymerase reaction with said primer using said aliquot of the DNA molecule (aliquot 1-1) as template;
(c4) determining at least one parameter, selected from the group consisting of the concentration, size, and sequence of the products of said specific DNA polymerase reaction; and
(c5) determining whether said TALE protein binds to said region, wherein
   (i) said TALE protein does not bind to said region or does bind to said region with a strongly reduced affinity when the parameters detected in step (c4) for the specific DNA polymerase reaction do not differ significantly from respective parameters for a specific DNA polymerase reaction performed with an aliquot of the DNA molecule as template to which no TALE protein has been added, and
   (ii) said TALE protein binds to said region when the parameters detected in step (c4) for the specific DNA polymerase reaction differ significantly from respective parameters detected in step (c4) for a specific DNA polymerase reaction performed with an aliquot of the DNA molecule as template to which no TALE protein has been added.

In the above step (c3) of the method of the present invention, a specific DNA polymerase reaction is performed with the primer of step (c1), using said DNA molecule to which said TALE protein has been added as template. In this context, it has been found that binding of a TALE protein to the region of DNA to which the primer binds abolishes the ability of a DNA polymerase to bind to the DNA-primer complex, thus inhibiting the elongation of the primer. Therefore, in case the cytosine nucleobase of interest is present, the TALE protein does not bind to the DNA molecule in the specific DNA polymerase reaction, thus allowing primer elongation. Accordingly, in case the cytosine nucleobase of interest is not present, the TALE protein does bind to the DNA molecule in the specific DNA polymerase reaction, thus inhibiting primer extension. Suitable DNA polymerase reactions are not particularly limited and are known in the art. They include for example primer extension reaction, arrayed primer extension (APEX), polymerase chain reaction (PCR), rolling circle amplification (RCA) and strand displacement amplification (SDA).

In order to detect whether primer elongation has occurred or not, at least one parameter, selected from the group consisting of the concentration, size (expressed as molecular weight or as chain-length), and sequence, of the products of said specific DNA polymerase reaction is determined in step (c4) of the method of the present invention. Methods for the detection of the above parameters are not particularly limited and are known in the art. In a preferred embodiment, in step (c4) of the method of the present invention, (i) the concentration of the products of said specific DNA polymerase reaction is determined using a method, selected from the group consisting of quantitative polymerase chain reaction (qPCR), fluorescence measurements using nucleic acid binding fluorescent probes, and photometry; and/or (ii) the size of the products of said specific DNA polymerase reaction is determined using a method, selected from the group consisting of polymerase chain reaction (PCR), and gel electrophoresis; and/or (iii) the sequence of the products of said specific DNA polymerase reaction is determined using a method, selected from the group consisting of Sanger sequencing, pyrosequencing, microarray hybridization, next-generation sequencing methods, and next-next-generation sequencing methods, including sequence-by-synthesis, sequencing-by-ligation, single molecule sequencing in zero-mode waveguides and nanopore sequencing.

In a particularly preferred embodiment, in step (c4) of the method of the present invention, the concentration of the products of said specific DNA polymerase reaction is determined by qPCR, the primer of step (c1) is biotinylated, and said products are isolated prior to said step (c4) by a method, comprising the steps of:
(i) incubating said products with beads that are coupled to streptavidin;
(ii) removing non-biotinylated template by washing in a buffer that denatures DNA duplexes; and
(iii) using said beads having the biotinylated products bound thereto in said qPCR.

Respective methods of generating biotinylated DNA polymerase reaction products by using biotinylated primers, of isolating biotinylated products using beads that are coupled to streptavidin, and of removing DNA duplexes are not particularly limited and are known in the art. In preferred embodiments, said primer is 5'-biotinylated, and said beads are streptavidin-agarose beads.

In the above step (c5) of the method of the present invention, the presence or absence of the cytosine nucleobase of interest is actually determined. This is based on the above finding that primer elongation in the specific DNA polymerase reaction can only occur in case the TALE protein does not bind to the DNA molecule or does bind to said molecule with a strongly reduced affinity, i.e. in case the cytosine nucleobase of interest is present. Thus, in this case the parameter determined in step (c4) will not differ from respective parameters for a specific DNA polymerase reaction performed with an aliquot of the DNA molecule as template to which no TALE protein has been added. In case the cytosine nucleobase of interest is not present, the TALE protein will bind to the DNA molecule, thus inhibiting primer elongation, so that the parameters determined in step (c4) will differ from respective parameters for a specific DNA polymerase reaction performed with an aliquot of the DNA molecule as template to which no TALE protein has been added. In this context, the term "differ significantly" as used herein relates to a difference of at least 10%, preferably at least 20%, at least 30% or at least 50%.

In this context, said respective parameters for a specific DNA polymerase reaction performed with an aliquot of the DNA molecule as template to which no TALE protein has been added can be reference values that have been determined independently prior to performing the method of the present invention. Alternatively, said parameters can be determined concurrently with the method of the present invention, e.g. by (i) providing a second aliquot of the first aliquot provided in step (b) (aliquot 1-2) to which no TALE protein is added in step (c2) of the method, (ii) performing said specific DNA polymerase reaction with said primer using said second aliquot of the DNA molecule (aliquot 1-2) as template, in addition to performing said specific DNA polymerase reaction with said primer using said first aliquot of the DNA molecule (aliquot 1-1) as template in step (c3), and (iii) determining said parameters in step (c4) of the method.

Thus, in an embodiment wherein said respective parameters for a specific DNA polymerase reaction performed with an aliquot of the DNA molecule as template to which no TALE protein has been added are determined concurrently with the method of the present invention, step (c) of said method comprises the steps of
(c1) providing a primer that is capable of binding to the region of said DNA molecule that includes said nucleobase of interest;
(c2) providing a first aliquot and a second aliquot of the first aliquot provided in step (b) (aliquots 1-1 and 1-2), and adding said TALE protein to said first aliquot of the DNA molecule (aliquot 1-1);
(c3) performing a first specific DNA polymerase reaction with said primer using said first aliquot of the DNA molecule (aliquot 1-1) as template, and performing a second specific DNA polymerase reaction with said primer using said second aliquot of the DNA molecule (aliquot 1-2) as template to which no TALE protein has been added;
(c4) determining at least one parameter, selected from the group consisting of the concentration, size, and sequence of each of the products of said first and second specific DNA polymerase reactions; and
(c5) determining whether said TALE protein binds to said region, wherein
   (i) said TALE protein does not bind to said region or does bind to said region with a strongly reduced affinity when the parameters detected in step (c4) for the first specific DNA polymerase reaction do not differ significantly from the parameters detected in step (c4) for the second specific DNA polymerase reaction, and
   (ii) said TALE protein binds to said region when the parameters detected in step (c4) for the first specific DNA polymerase reaction differ significantly from the parameters detected in step (c4) for the second specific DNA polymerase reaction.

Further, in embodiments wherein a second aliquot (aliquot 2) of said DNA molecule that is not subjected to the modification reaction in step (b) of the method is used, the above method steps (c1) to (c5) can be performed in an analogous manner, e.g. by providing a first and a second aliquot of said second aliquot (aliquots 2-1 and 2-2) in step (c2), wherein no TALE protein is added to aliquot (2-2), performing respective specific DNA polymerase reactions in step (c3), and determining respective parameters in step (c4).

Thus, in a respective embodiment, step (c) of the method of the present invention comprises the steps of
(c1) providing a primer that is capable of binding to the region of said DNA molecule that includes said nucleobase of interest;
(c2) providing a first aliquot and a second aliquot of the first aliquot provided in step (b) (aliquots 1-1 and 1-2), and a first aliquot and a second aliquot of the second aliquot provided in step (b) (aliquots 2-1 and 2-2), and adding said TALE protein to said first aliquots of the DNA molecule (aliquots 1-1 and 2-1);
(c3) performing first specific DNA polymerase reactions with said primer using said first aliquots of the DNA molecule (aliquots 1-1 and 2-1) as template, and performing second specific DNA polymerase reactions with said primer using said second aliquots of the DNA molecule (aliquots 1-2 and 2-2) as template to which no TALE protein has been added;
(c4) determining at least one parameter, selected from the group consisting of the concentration, size, and sequence of each of the products of said first and second specific DNA polymerase reactions; and
(c5) determining whether said TALE protein binds to said region, wherein
   (i) said TALE protein does not bind to said region or does bind to said region with a strongly reduced affinity when the parameters detected in step (c4) for the first specific DNA polymerase reactions do not differ significantly from the parameters detected in step (c4) for the second specific DNA polymerase reactions, and
   (ii) said TALE protein binds to said region when the parameters detected in step (c4) for the first specific DNA polymerase reactions differ significantly from the parameters detected in step (c4) for the second specific DNA polymerase reactions.

In particular embodiments, the region of the DNA molecule to which the TALE protein is capable of binding in a sequence-specific manner contains more than one cytosine nucleobase of interest. In these embodiments, step (b) of the method of the present invention can comprise the use of more than one suitable modification reactions as defined above, and at least one cytosine nucleobase of interest is present when the binding affinity of said TALE protein to said region of said DNA molecule in the first aliquot (first binding affinity) is different from a binding affinity of said TALE protein to said region of said DNA molecule that has not been subjected to said modification reaction(s) (second binding affinity).

The method of the present invention can not only be used to gain a qualitative result concerning the presence or absence of a cytosine nucleobase of interest, but also to gain a quantitative result concerning the degree of presence of the cytosine nucleobase of interest in a given DNA preparation. To this end, a calibration curve using DNA molecules having a known degree of presence of the cytosine nucleobase of interest can be established, and the degree of presence of the cytosine nucleobase of interest can be determined by linear regression. Accordingly, in a preferred embodiment, the method of the present invention further comprises the steps of:
(d) comparing the parameters detected in step (c4) for the specific DNA polymerase reaction to one or more respective parameters obtained from DNA molecules having a known degree of presence of the cytosine nucleobase of interest; and
(e) thereby determining the degree of presence of the cytosine nucleobase of interest in the DNA molecule.

In this context, the term "degree of presence of the cytosine nucleobase of interest" relates to the proportion of DNA molecules in a given DNA molecule preparation that have the cytosine nucleobase of interest at a given position as opposed to DNA molecules that have any other cytosine nucleobase at the position. By way of example, a DNA molecule preparation wherein 10% of the individual DNA molecules have a mC at a given position whereas the remaining individual DNA molecules have a C at that position, the degree of presence of the cytosine nucleobase of interest would be 10%.

The TALE proteins employed in the present invention do not only allow for the determination of the presence or absence of a particular cytosine nucleobase of interest in a DNA molecule, but can also be used for a wide range of applications for the detection, manipulation, purification, and analysis of epigenetically modified nucleobases in DNAs. To this end, said TALE proteins can be coupled to a variety of compounds, proteins, affinity ligands, and other moieties. Such "coupling moieties" are not particularly limited and can be easily chosen by a person skilled in the art in accordance with the particular application of interest. Examples of such moieties include fluorescent dyes, fluorescent proteins such as e.g. green fluorescent protein (GFP), affinity ligands such as e.g. biotin, digoxigenin, dinitrophenol, magnetic particles, antibodies, including antibody fragments and antibody mimetics known in the art, protein tags, and proteins having enzymatic activities such as nuclease activities. Suitable protein tags in this respect include 18A-Tag, ACP-Tag, Avi-Tag, BCCP-Tag, Calmodulin-Tag (CaM-Tag), Chitin-binding-Protein-Tag (CBP-Tag), E-Tag, ELK16-Tag, ELP-Tag, FLAG-Tag, Flash-Tag, poly-glutamic acid-Tag, Glutathion-S-Transferase-Tag (GST-Tag), Green fluorescent protein-Tag (GFP-Tag), Hemagglutinin-Tag (HA-Tag), poly-Histidin-Tag (His-Tag), Isopeptag, Maltose binding protein-Tag (MBP-Tag), Myc-Tag, Nus-Tag, ProtA-Tag, ProtC-Tag, S-Tag, SBP-Tag, Snap-Tag, SpyTag, SofTag 1 and 3, Streptavidin-Tag (Strep-Tag), Strep-II-Tag, Tandem Affinity Purification-Tag (TAP-Tag), TC-Tag, Thioredoxin-Tag (TRX-Tag), Ty-Tag, V5-Tag, VSV-Tag, and Xpress-Tag, which are known in the art. Such "coupling moieties" can be coupled directly or indirectly to the TALE protein, or can be expressed as part of conjugate proteins comprising also the TALE protein. Methods for the coupling of respective "coupling moieties" and for the expression of respective conjugates are not particularly limited and are known in the art. They include methods employing a His-Tag on the expressed conjugates and purification via NiNTA as known in the art.

In this context, TALE proteins can include non-natural amino acids with reactive groups. The coupling of the above compounds can be effected e.g. via click chemistry at said reactive groups. Suitable reactive groups in this context are not particularly limited and include e.g. azides, alkynes, alkenes, including strained alkynes and alkenes that have been genetically encoded in the form of non-natural amino acids.

Applications of respectively coupled TALE proteins are not particularly limited and can be easily devised by the person skilled in the art in accordance with the respective application or scientific problem to be addressed.

In accordance with the above, in a preferred embodiment of the method of the present invention for cases wherein the alteration of the binding affinity is an abolishment of binding or a significant weakening of the binding affinity, the TALE protein is coupled directly or indirectly to an affinity ligand, said method further comprising the step of:
(d) depleting DNA molecules in which the cytosine nucleobase of interest is absent via affinity capture using said affinity ligand;
said method allowing for the enrichment of DNA molecules in which the cytosine nucleobase of interest is present.

Alternatively, in a preferred embodiment of the method of the present invention for cases wherein the alteration of the binding affinity is a strengthening of the binding affinity, the TALE protein is coupled directly or indirectly to an affinity ligand, set method further comprising the step of:
(d) depleting DNA molecules in which the cytosine nucleobase of interest is present via affinity capture using said affinity ligand;
said method allowing for the enrichment of DNA molecules in which the cytosine nucleobase of interest is absent.

In this context, the term "affinity capture" as used herein relates to any methods wherein an affinity ligand is bound by a suitable binding partner. This includes binding of the affinity ligand to a binding partner in solution and subsequent immobilization of the binding partner, as well as binding of the affinity ligand to an already immobilized binding partner (e.g. in affinity chromatography).

Affinity ligands for use according to these embodiments are not particularly limited and are known in the art. They include for example biotin, digoxigenin, dinitrophenol, magnetic particles, antibodies, including antibody fragments and antibody mimetics, and protein tags, e.g. as indicated above. Methods for the direct or indirect coupling of affinity ligands to TALE proteins are not particularly limited and are known in the art. In this context, the term "coupled directly or indirectly" expressly includes the expression of conjugate molecules of TALE proteins and any particular proteinaceous affinity ligands. Further, the term "coupled indirectly" includes any coupling strategies employing linkers, binding via further affinity partners, and the like, which are known in the art. Moreover, methods for the depletion of DNA molecules in which an epigenetic modification of said nucleobase of interest is absent via affinity capture using said affinity ligands are not particularly limited and are known in the art. Particular methods include the use of His-tags as affinity ligand and depletion of DNAs bound to respective TALE proteins via NiNTA-beads, as well as the use of other protein tags as indicated above in connection with the respective affinity binding partners.

According to these embodiments of the method of the present invention, DNA molecules in which the cytosine nucleobase of interest is absent (or present) are depleted from a mixture containing DNA molecules in which the cytosine nucleobase of interest is present, as well as DNA molecules in which the cytosine nucleobase of interest is absent. In this manner, DNA molecules in which the cytosine nucleobase of interest is present (or absent) are enriched in the mixture.

Also in accordance with the above, in a further preferred embodiment of the method of the present invention for cases wherein the alteration of the binding affinity is an abolishment of binding or a significant weakening of the binding affinity, the TALE protein is coupled directly or indirectly to a protein having nuclease activity, said method further comprising the step of:
(d) digesting DNA molecules in which the cytosine nucleobase of interest is absent using said protein having nuclease activity;
said method allowing for the differential digestion of DNA molecules in which the cytosine nucleobase of interest is absent, wherein DNA molecules in which an epigenetic modification of said nucleobase of interest is present remain undigested.

Proteins having nuclease activity for use according to this embodiment are not particularly limited and are known in the art. They include for example restriction enzymes and cleavage domains of restriction enzymes, e.g. the cleavage domain of Fokl. Methods for the direct or indirect coupling of proteins having nuclease activity to TALE proteins are not particularly limited and are known in the art. In this context, the term "coupled directly or indirectly" expressly includes the expression of conjugate molecules of TALE proteins and any particular proteins having nuclease activity. Further, the term "coupled indirectly" includes any coupling strategies employing linkers, binding via further affinity partners, and the like, which are known in the art. Moreover, methods for the digestion of DNA molecules in which an epigenetic modification of said nucleobase of interest is absent using said proteins having nuclease activity are not particularly limited and are known in the art.

According to this embodiment of the method of the present invention, DNA molecules in which the cytosine nucleobase of interest is absent are digested in a mixture containing DNA molecules in which the cytosine nucleobase of interest is present, as well as DNA molecules in which the cytosine nucleobase of interest is absent. In this manner, DNA molecules in which the cytosine nucleobase of interest is present are enriched in the mixture.

The enriched DNA molecules in which the cytosine nucleobase of interest is present, obtained according to the above preferred embodiments of the method of the present invention, can be further used for any particular application of interest. For examples, said DNA molecules can be used for sequencing applications known in the art, including PCR and so-called Next Generation Sequencing methods, microarray analyses, or any methods for the analysis of nucleic acids known in the art.

The methods of the present invention can be used for the concurrent determination of the presence or absence of more than one particular cytosine nucleobase of interest in a given DNA molecule. In particular, several aliquots of a given DNA molecule can be provided, and the presence of different cytosine nucleobases of interest can be determined, respectively. As an example, the method of the present invention can be performed with respect to the detection of C, mC, hmC, fC, and caC, respectively, for a given position with the DNA molecule.

In a second aspect, the present invention relates to a method for the enrichment of DNA molecules in which a particular cytosine nucleobase of interest is present, said method comprising the steps of:
(a) providing a transcription-activator-like effector (TALE) protein, said TALE protein being capable of binding in a sequence-specific manner to a region of said DNA molecule that includes said nucleobase of interest, wherein said TALE protein is coupled directly or indirectly to an affinity ligand;
(b) subjecting the DNA molecule to a modification reaction that selectively modifies the cytosine nucleobase of interest at the 5-position, wherein the presence of said chemical modification at the 5-position of the cytosine nucleobase of interest abolishes or significantly weakens binding of said TALE protein to the DNA molecule;
(c) incubating the DNA molecules with said TALE protein; and
(d) depleting DNA molecules in which the cytosine nucleobase of interest is absent via affinity capture using said affinity ligand.

In a related third aspect, the present invention relates to a method for the enrichment of DNA molecules in which a particular cytosine nucleobase of interest is absent, said method comprising the steps of:
(a) providing a transcription-activator-like effector (TALE) protein, said TALE protein being capable of binding in a sequence-specific manner to a region of said DNA molecule that includes said nucleobase of interest, wherein said TALE protein is coupled directly or indirectly to an affinity ligand;
(b) subjecting the DNA molecule to a modification reaction that selectively modifies the cytosine nucleobase of interest at the 5-position, wherein the presence of said chemical modification at the 5-position of the cytosine nucleobase of interest strengthens binding of said TALE protein to the DNA molecule;
(c) incubating the DNA molecules with said TALE protein; and
(d) depleting DNA molecules in which the cytosine nucleobase of interest is present via affinity capture using said affinity ligand.

In a fourth aspect, the present invention relates to a method for the differential digestion of DNA molecules in which a particular cytosine nucleobase of interest is absent, wherein DNA molecules in which the cytosine nucleobase of interest is present remain undigested, said method comprising the steps of:
(a) providing a transcription-activator-like effector (TALE) protein, said TALE protein being capable of binding in a sequence-specific manner to a region of said DNA molecule that includes said nucleobase of interest, wherein the TALE protein is coupled directly or indirectly to a protein having nuclease activity;
(b) subjecting the DNA molecule to a modification reaction that selectively modifies the cytosine nucleobase of interest at the 5-position, wherein the presence of said chemical modification at the 5-position of the cytosine nucleobase of interest abolishes or significantly weakens binding of said TALE protein to the DNA molecule;
(c) incubating the DNA molecules with said TALE protein; and
(d) digesting DNA molecules in which the cytosine nucleobase of interest is absent using said protein having nuclease activity.

In a related fifth aspect, the present invention relates to a method for the differential digestion of DNA molecules in which a particular cytosine nucleobase of interest is present, wherein DNA molecules in which the cytosine nucleobase of interest is absent remain undigested, said method comprising the steps of:
(a) providing a transcription-activator-like effector (TALE) protein, said TALE protein being capable of binding in a sequence-specific manner to a region of said DNA molecule that includes said nucleobase of interest, wherein the TALE protein is coupled directly or indirectly to a protein having nuclease activity;
(b) subjecting the DNA molecule to a modification reaction that selectively modifies the cytosine nucleobase of interest at the 5-position, wherein the presence of said chemical modification at the 5-position of the cytosine nucleobase of interest strengthens binding of said TALE protein to the DNA molecule;
(c) incubating the DNA molecules with said TALE protein; and
(d) digesting DNA molecules in which the cytosine nucleobase of interest is present using said protein having nuclease activity.

In a sixth aspect, the present invention relates to a method for the differential digestion of DNA molecules in which a particular cytosine nucleobase of interest is absent, wherein DNA molecules in which the cytosine nucleobase of interest is present remain undigested, said method comprising the steps of:
(a) providing a transcription-activator-like effector (TALE) protein, said TALE protein being capable of binding in a sequence-specific manner to a region of said DNA molecule that includes said nucleobase of interest;
(b) subjecting the DNA molecule to a modification reaction that selectively modifies the cytosine nucleobase of interest at the 5-position, wherein the presence of said chemical modification at the 5-position of the cytosine nucleobase of interest strengthens binding of said TALE protein to the DNA molecule;
(c) incubating the DNA molecules with said TALE protein; and
(d) incubating the DNA molecules with a protein having nuclease activity;
wherein DNA molecules in which the cytosine nucleobase of interest is absent are digested using said protein having nuclease activity, and DNA molecules in which the cytosine nucleobase of interest is present are protected from digestion by the TALE protein bound to the DNA molecule.

In a related seventh aspect, the present invention relates to a method for the differential digestion of DNA molecules in which a particular cytosine nucleobase of interest is present, wherein DNA molecules in which the cytosine nucleobase of interest is absent remain undigested, said method comprising the steps of:
(a) providing a transcription-activator-like effector (TALE) protein, said TALE protein being capable of binding in a sequence-specific manner to a region of said DNA molecule that includes said nucleobase of interest;
(b) subjecting the DNA molecule to a modification reaction that selectively modifies the cytosine nucleobase of interest at the 5-position, wherein the presence of said chemical modification at the 5-position of the cytosine nucleobase of interest abolishes or significantly weakens binding of said TALE protein to the DNA molecule;
(c) incubating the DNA molecules with said TALE protein; and
(d) incubating the DNA molecules with a protein having nuclease activity;
wherein DNA molecules in which the cytosine nucleobase of interest is present are digested using said protein having nuclease activity, and DNA molecules in which the cytosine nucleobase of interest is absent are protected from digestion by the TALE protein bound to the DNA molecule.

In this sixth and seventh aspects of the present invention, the presence or absence of digestion or differences in the degree of digestion can be determined e.g. by qPCR or next generation sequencing, wherein (i) in the sixth aspect the absence of digestion or a relatively lesser degree of digestion indicates the presence of the nucleobase of interest, and presence of digestion or a relatively higher degree of digestion indicates the absence of the nucleobase of interest, and (ii) in the seventh aspect the absence of digestion or a relatively lesser degree of digestion indicates the absence of the nucleobase of interest, and presence of digestion or a relatively higher degree of digestion indicates the presence of the nucleobase of interest

All of the relevant definitions, explanations, and preferred embodiments defined above for the method according to the first aspect of the present invention expressly apply in an analogous manner also to the methods according to the second to sixth aspect of the present invention.

In particular, in preferred and independent embodiments, (i) the DNA molecule is preferably a double-stranded DNA molecule, (ii) the region to which said TALE protein is capable of binding in a sequence- and locus-specific manner is from 10 to 40 nucleotides in length, preferably from 15 to 30 nucleotides in length, more preferably from 18 to 26 nucleotides in length, (iii) said DNA molecule is in its native state and is not subjected to any pretreatment in addition to the modification reaction of step (b), (iv) the affinity ligands include for example biotin, digoxigenin, dinitrophenol, magnetic particles, antibodies, including antibody fragments and antibody mimetics, and protein tags, and (v) the proteins having nuclease activity include for example restriction enzymes and cleavage domains of restriction enzymes, e.g. the cleavage domain of Fokl. Moreover, the preferred cytosine nucleobases, the respective TALE proteins and their RVDs, as well as the respective modification reactions are preferably as defined for the first aspect of the present invention.

The methods of the present invention according to all aspects are *in vitro* methods, *i.e*., said methods are not performed on the human or animal body.

The methods of the present invention advantageously combine assay simplicity with high analytical accuracy, *i.e.,* level quantification at single sites, strand specificity, and no density bias. The selective modification response strongly facilitates the identification of selective TALE nucleobase interactions and is also expected to strongly enhance selectivity, since chemical difference between the cytosine nucleobase of interest and all others can be increased in an almost arbitrary manner, whereas differentiation between natural cytosine nucleobases requires selectivity between subtle chemical differences (e.g. formyl group vs. hydroxymethyl group etc.

The figure show:
Figure 1:
   Current model for the complete cycle of dynamic cytosine modification as central mechanism for the epigenetic regulation of gene expression. TET: ten-eleven translocation dioxygenase. TDG: Thymine DNA glycosylase. BER: base excision repair.
Figure 2:
   Current approaches for hmC analysis. C, mC and hmC are depicted as black, red, and blue spheres, respectively.
Figure 3:
   Principle of an expanded programmability of DNA recognition. A molecular scaffold consisting of interchangeable repeats with individual selectivities not only for the canonical nucleobases A, G, T, and C, but also for epigenetic nucleobases provides expanded programmable DNA recognition. C, mC and hmC are depicted as black, red, and blue spheres, respectively.
Figure 4:
   Exemplary representation of the several structural components of transcription activator like effectors (TALEs). a) TAL effectors have various domains of which the repeat region binds to the DNA. The highly conserved primary structure of a single TALE repeat contains different repeat variable residues (RVD) depending on the targeted nucleotide. b) Frontal and lateral view of the TALE binding domain/DNA.
Figure 5:
   Principle of TALE-DNA binding and its use for an expanded programmability of DNA recognition. A: Interaction of RVD HD (aa 12 and 13) with C in DNA. Hydrogen bonds are shown as green dashed lines. B: Structure of the interaction of RVD NG (aa 12 and 13) with T in DNA. Hydrogen bonds are shown as green dashed lines. PDB entry 3V6T. C: Principle of replication assay. TALE binds conditionally on presence of C, mC, or hmC and competitively with a DNA polymerase to a primer/template complex and thus controls primer extension. Extension product is quantified and used as a measure of inhibitive binding of TALE. Scheme shows an example with C and hmC. D: Selectivities of three TALE repeats for C, mC and hmC. Selectivity of TALEs with the TALE repeat with RVD as shown in the diagram opposite a single C, mC, or hmC in the target DNA duplex was analyzed in the replication assay shown in Fig. 5C. E: Inhibition profile with DNAs as shown in Fig. 5D and TALE_Drh1(N*). F: Data of replication assay analogous to Fig. 5D with TALE_Drh1(N*) and mixtures of two nucleotides at the single position of the primer/template complex as shown in the figure between 0 and 100 % in increments of 20 %. G: Inhibition constants obtained in replication assays for TALEs with TALE repeats with RVDs as shown opposite a single C, mC, or hmC in the primer/template complex.
Figure 6:
   Nucleobase modification reactions employed in the present invention. Dnmt: DNA-methyltransferase; SAM: S-Adenosyl-methionine; TET: Ten-Eleven-translocation dioxygenase; T4 BGT: T4 beta-glucosyl-transferase; NaBH4: sodium borohydride; EDC: 1-Ethyl-3-(3-dimethylaminopropyl)carbodiimid.
Figure 7:
   Concept of TALE repeat engineering to achieve universal binding to cytosine and all of its carbon-5-modifications. a) First row of the table contains the amino acid sequence of a repeat of the DNA binding domain of a TALE, the second row shows the different RVDs that are representatively shown in row three in a short excerpt from position 11 to 15. The 4th row gives the name of the TALEs that bear the variants. b) Model of a TALE with special N- and C-terminal domains (light blue; GFP: green; His-Tag: dark blue) flanking the DNA binding region with HD (dark grey), NG (black), NI (light grey), NN (white) and the single engineered mutant RVD (red).
Figure 8:
   a) CDKN2A and b) BRCA1 target sequence with the marked xCpG dinucleotide (red) that can contain C, mC, hmC, fC or caC. Below the target sequences are the corresponding canonical TALE repeats (abbreviated by the RVDs they bear). The numbers stand for the position in the respective pFUS A30A, A30B or B4 vector. LR is the last repeat.
Figure 9:
   Principle of the primer extension assay. TALEs that bind cytosine variants universally or selectively will compete with KF (exo-) to bind to the single strand DNA template which is paired with a shorter radiolabeled primer. The inhibitive binding of TALEs can be used to identify their unique binding efficiencies by quantifying the abundance of extension product that results from the polymerase activity for example by denaturing PAGE.
Figure 10:
   Direct, universal recognition of epigenetic cytosine derivatives by engineered TALEs with mutated repeats. Selectivities of TALEs bearing one of the listed engineered repeats with respective alternated RVDs and loops (c.f. color code) for DNA binding at a DNA:TALE ratio of 1:5 (solid fill, 41.65 nM) and 1:20 (diagonal pattern, 166.6 nM).
Figure 11:
   Determination of T4 β-GT glycosylation efficiency for an hmC-bearing dsDNA oligonucleotide at two different incubation times. a) Reaction scheme for the glycosylation of hmC bearing dsDNA using T4 β-GT at 37°C. b) Table with reaction conditions, determined masses, yields and excerpts of the ESI-TOF mass spectra. mpre: detected mass of starting material (o1520_hmCpG) mpost: detected mass after 1 h or 18 h of glycosylation (o1520_ghmCpG).
Figure 12:
   Selective modification response by enzymatic glycosylation of hmC. a) Denaturing PAGE after primer extension reveals a significant decrease of KF(exo-) inhibition for all TALEs tested on tCDKN2A_C6 → ghmC. Analysis shows that the 3* RVD in TALE 134 provides the most selective molecular recognition of glycosylated hmC. b) No other tested cytosine variant is significantly affected by the action of T4 β-GT or presence of UDP-Glucose.
Figure 13:
   Inhibitory constants for TALE 134 reveal the kinetic differences for cytosine and its 5-modifications. a) Variable dose response as four-parameter logistic equation. A₁: initial extension value; A₂: final extension value; p: hill slope; xo: inflection point, IC50; x: variable concentration. b) Denaturing urea PAGE analysis of primer extensions containing 25 mU KF (exo-) and 8.33 nM of tCDKN2A_C6 → hmC or tCDKN2A_C6 → ghmC. c) Glycosylation of hmC has an effect on TALE 134 concentration dependent inhibition of KF (exo-) activity, revealing quantitative insights in the selective modification response. tCDKN2A (red squares), tCDKN2A_C6 → hmC (black triangles) are included for reference. d) Inhibition of 25 mU KF (exo-) on 8.33 nM target CDKN2A sequences at varying concentrations of TALE 134. The various styles of the fitted sigmoidal curves represent the binding curves of the TALE to DNA targets with different tested Cytosine derivatives at the 6th position: black universal: C; dash-dot: mC; red universal: hmC; dot: fC ;dash: caC. e) Table of estimated IC50 or Kᵢ values calculated with the formula of the fitted dose response function.
Figure 14:
   Selective reduction of fC to hmC by NaBH₄. B: Selectivity of TALE repeat N* for hmC and fC. DNA-binding before and after reduction of fC to hmC by NaBH₄ was analyzed in primer extension assay containing 8.3 nM primer-template complex in presence of 416 nM TALE T-drh107 and 25 mU KF(exo-) were analyzed by denaturing PAGE. Full length extension product was quantified and shown in column diagram. AU = arbitrary units.
Figure 15:
   Binding of TALEs having the indicated RVDs to C, mC, hmC, fC, and caC in primer extension assay (A to C).

The present invention will be further illustrated in the following examples without being limited thereto.

### Examples

### Material and experimental procedures:

### Bacterial strains

In the present invention, *E. coli* strains BL21 (DE3), BL21 (DE3) Gold, and GH371 were used.

**Buffers and media**

| Name | Components |
|---|---|
| CutSmart buffer | 50 mM KOAc, 20 mM Tris-acetate, 10 mM Mg(OAc)₂, 100 µg/mL BSA, pH 7.9 |
| 5xBGrK1 (TAL Binding Buffer G1) | 100 mM Tris-HCl (pH = 8.0), 250 mM NaCl, 25 mM MgCl₂, 0.5 mg/ml BSA, 25% Glycerol |
| KF (exo⁻) storage buffer | 25 mM Tris-HCI, 1 mM DTT, 0.1 mM EDTA, 50% Glycerol, pH 7.4 |
| Glyco buffer 1¹¹⁸ | 150 mM NaCl, 20 mM Tris-HCl (pH 8.0), 25 mM CaCl₂, 1 mM DTT |
| Glyco buffer 2⁵⁷ | 50 mM HEPES (pH 7.9), 25 mM MgCl2 |
| LB Medium (Sterile) | 10 g/l Tryptone, 5 g/l yeast extract, 10 g/l NaCl, pH 7 |
| NEB1 | 10 mM Bis-Tris-Propane-HCl, 10 mM MgCl₂, 1 mM DTT, pH 7 |
| NEB2 | 50 mM NaCl, 10 mM Tris-HCl, 10 mM MgCl₂, 1 mM DTT, pH 7.9 |
| NEB3 | 100 mM NaCl, 50 mM Tris-HCl, 10 mM MgCl2, 1 mM DTT, pH 7.9 |
| NEB4 | 50 mM KOAc, 20 mM Tris-acetate, 10 mM Mg(OAc)₂, 1 mM DTT, pH 7.9 |
| 4xPBS | 548 mM NaCl, 43 mM KCl, 69 mM Na₂HPO₄, 3.2 g/l KH₂PO₄, pH 8 |
| Qiagen Lysis buffer | 50 mM NaH₂PO₄ monohydrate, 300 mM NaCl, pH 8 |
| Qiagen Lysis buffer 20 | Qiagen Lysis buffer + 20 mM Imidazole |
| Qiagen Lysis buffer 50 | Qiagen Lysis buffer + 50 mM Imidazole |
| Qiagen Lysis buffer 500 | Qiagen Lysis buffer + 500 mM Imidazole |
| SOB⁺⁺ (Sterile) | 20 g/l Tryptone, 5 g/L yeast extract, 0.5 g/l NaCl, 0.186 g/L KCl, 10 mmol/l MgCl₂, 10 mmol/l MgSO₄ |
| SOC medium (Sterile) | 0.58 g/l NaCl, 2.03 g/l MgCl₂ hexahydrate, 2.46 g/l MgSO₄ heptahydrate, 5 g/l Yeast Extract, 20 g/l Trypton, 1 M Glucose, pH 7.5 |
| T4 DNA ligase buffer | 50 mM Tris-HCl, 10 mM MgCl₂, 1 mM ATP, 10 mM DTT, pH 7.5 |
| T4 PNK buffer A | 350 mM Tris-HCl (pH 7.6), 50 mM MgCl₂ , 500 mM KCl, 5 mM 2-mercaptoethanol |
| TALE storage buffer | 200 mM NaCl, 20 mM Tris-HCI (pH 7.5), 10% glycerol, 1 mM DTT |
| Taq-lysis buffer | 10 mM Tris-HCl, 300 mM NaCl, 2.5 mM MgCl₂, 0.1% Triton X-100, pH 9 |
| TB buffer (sterile) | 10 mM HEPES, 15 mM CaCl₂, 250 mM KCl, 55 mM MnCl₂, pH 6.7 |
| TBE buffer | 89 mM Tris, 89 mM Boric acid, 2 mM EDTA (pH 8) |

**Chemicals, reagents, formulations**

| Name | Supplier | Grade | Abbreviation/ Molecular formula |
|---|---|---|---|
| 2-Log DNA Ladder | NEB | | |
| (4-(2-hydroxyethyl)-1-piperazineethanesulfonic acid) | Roth | ≥99.5 % | HEPES |
| [y-³²P]ATP | Hartmann Analytic | | |
| Ammonium persulfate | Roth | ≥98 % | APS |
| Boric acid | Roth | ≥99.8 % | |
| Bovine Serum Albumin | Cell Signaling | | BSA |
| Brilliant blue G250 | Roth | | |
| Bromophenol blue | Roth | | |
| Calcium chloride | Fisher | | |
| Carbenicilin | Roth | ≥98 % | |
| Disodiumhydrogen phosphate | Sigma Aldrich | ≥985 % | Na₂HPO₄ |
| Dithiothreitol | Roth | ≥99 % | DTT |
| Ethanol | Sigma Aldrich | ≥998 % | EtOH |
| Ethidium bromide | Sigma Aldrich | | |
| Ethylene diamine tetraacetic acid | Roth | ≥99 % | EDTA |
| Glycerol | Roth | | |
| N-2-Hydroxyethyl piperazine-N'-2-ethane sulphonic acid | Roth | ≥99,9 | HEPES |
| Hydrochloric acid | VWR | | HCI |
| Imidazol | ABCR | 99 % | |
| Isopropanol | Fisher | ≥99.9 % | |
| Isopropyl β-D-1-thiogalactopyranoside | Roth | ≥99 % | IPTG |
| LB-Agar | Roth | | |
| LB-Medium | Roth | | |
| LE Agarose | Roth | | |
| Magnesium acetate | Merck | | Mg(OAc)₂ |
| Magnesium chloride hexahydrate | Acros | ≥99 % | MgCl₂ |
| Magnesium sulfate heptahydrate | Merck | ≥99.5 % | MgSO₄ |
| Nickel Nitrilotriacetic acid | Thermo Scientific | | Ni-NTA |
| Phenylmethanesulfonylfluoride | Roth | ≥99 % | PMSF |
| Potassium chloride | Roth | ≥99.5 % | KCI |
| Potassium dihydrogenphosphate monohydrate | Sigma Aldrich | ≥99.5 % | KH₂PO₄ |
| Protein Marker, Broad Range (2-212 kDa) | NEB | | |
| Rotiphorese Gel40 | Roth | | |
| Roti®-Chloroform/Phenol/ Isoamylalcohol | Roth | | |
| Sequencing gel concentrate | Roth | | |
| Sodium chloride | Roth | ≥99.5 % | NaCl |
| Sodium dodecyl sulfate | Roth | ≥99 % | SDS |
| Sodium hydroxide | Fisher | | NaOH |
| Spectinomycin | Alfa Aeser | | Spec |
| Streptavidin-Agarose | Sigma Aldrich | | |
| Tetracycline | Roth | | Tet |
| Tetramethylethylenediamine | Roth | ≥99 % | TMEDA |
| Tris(hydroxymethyl)amino methane | Sigma Aldrich | ≥99.9 % | Tris |
| Triton X-100 | Fluka | | |
| Urea | Roth | ≥99.5 % | |
| Uridine diphosphate glucose | NEB | | UDP-Glc |
| Xylene cyanol | Roth | | |
| Yeast Extract | Roth | | |

**Enzymes**

| Name | Supplier | Conc. (Units/µl) | Application |
|---|---|---|---|
| Bsal | NEB | 10 | Restriction |
| BsmBI | NEB | 10 | Restriction |
| Xhol | NEB | 20 | Restriction |
| Ncol | NEB | 50 | Restriction |
| Plasmid-Safe DNase | Biozym | 10 | Restriction |
| Sacl | NEB | 20 | Restriction |
| AbaSI | NEB | 10 | Restriction |
| Klenow Fragment (exo-) | NEB | 5 | Primer extension |
| Phusion | NEB/Self-made | 2 | PCR |
| Taq | NEB/Self-made | 5 | PCR |
| Lysozyme | Fluka | | Lysis |
| T4-DNA ligase | NEB | 400 | Ligation |
| T4 Polynucleotidekinase | ThermoFisher Scientific | 10 | 5'-Phosphorylation |
| T4-β-glucosyltransferase | NEB | 10 | Glucosylation |

**Kits**

| Name | Supplier | Application |
|---|---|---|
| GeneJet Gel Extraction | Thermo Fisher Scientific | Gel extraction |
| GeneJet Plasmid Miniprep | Thermo Fisher Scientific | Plasmid isolation |
| Pierce BCA Protein Assay Kit - Reducing Agent Compatible | Thermo Fisher Scientific | BCA |
| QiaAmp DNA Mini Kit | Qiagen | Purification of human genomic DNA |

### Basic bacterial cultivation and cloning techniques - Liquid culture

A single bacterial colony was inoculated in LB medium and incubated at 37 °C at 180 rpm shaking for overnight in glass Erlenmeyer flasks or conical 15 and 50 ml plastic tubes. Depending on the antibiotic resistance of the organisms, the sterile LB medium was supplemented with 50 µg/mL Carbenicillin (Roth), 12.5 µg/mL Tetracycline (Alfa Aesar) and 100 µg/mL Spectinomycine (Roth), respectively.

### Basic bacterial cultivation and cloning techniques - Plate culture

Sterile LB-Agar was liquefied by heating to the boiling point in a microwave at 600 W and then cooled down to around 40 °C under constant stirring. The LB-Agar was supplemented with the respective antibiotic (cf. Liquid culture, *supra*) and swiftly poured into a plastic petri dish. Bacterial cultures inoculated in either water or SOC medium were pipetted onto the LB-Agar and spread out using glass beads close to the flame.

### Basic bacterial cultivation and cloning techniques - Preparation of chemical competent GH317 E. coli bacteria

A colony of the GH317 strain was inoculated in 15ml LB medium and incubated overnight as described above. 8 ml of bacterial overnight culture were added to 800 ml of LB medium and incubated at 37 °C whilst shaking at 180 rpm until an OD₆₀₀ of 0.5 was reached. The culture was cooled on ice and subsequently centrifuged at 4 °C for 10 min at 4000 rpm. The supernatant was promptly discarded afterwards and the pellet was resuspended in 80 ml of precooled, sterile 100 mM MgCl₂ on ice. Culture was again centrifuged (4 °C, 15 min, 4000 rpm), the pellet recovered, kept on ice and resuspended in 80 ml of ice-cold, sterile 50 mM CaCl₂. After repeating the previous step of centrifugation, the pellet was resuspended in 4 ml ice cold, sterile 50 mM CaCl₂ supplemented with 15 % glycerol. Finally, the suspension was distributed in volumes of 50 µL to 0.2 ml PCR tubes on ice, immediately frozen in liquid Nitrogen and stored at -80 °C.

### Basic bacterial cultivation and cloning techniques - Procedure for BL21(DE3) and BL21(DE3)Gold strain

800 ml of SOB⁺⁺ medium were inoculated with 5 ml of bacterial overnight culture and incubated at 200 rpm shaking and 25-30 °C till an OD₆₀₀ = 0.5 was reached. The culture was chilled on ice for at least 10 min. In the further procedure the cultures were kept on ice as much as possible. Culture was centrifuged at 4 °C for 10 min at 4000 rpm. Supernatant was discarded and the pellet was resuspended in 200 ml of cold TB-Buffer with a Pipette on Ice and incubated on ice for 10 min. After centrifugation at 4 °C (Eppendorf 5804R) for 10 min at 4000 rpm the supernatant was discarded and the pellet was resuspended in 30 ml of cold TB-Buffer and 2.24 ml cold DMSO were added and incubated for 10 min on ice. A volume of 200 µL was transferred to 0.2 ml PCR tubes on ice and immediately frozen in liquid Nitrogen. Storage was performed at -80 °C.

### Basic bacterial cultivation and cloning techniques - Transformation by heat shock

Depending on purity and concentration up to 5 µl of the plasmid solution (usually 1 µl if purified and concentrated, 2.5 µl if crude product was used) were very carefully suspended in a solution with 25-50 µl GH317 bacterial cells (for plasmid enrichment) or 100-200 µl BL21 bacterial cells (for protein expression) in a 1.5 ml micro centrifuge tube and cooled on ice for 30 minutes. After a heat shock at 42 °C for 30-45 sec the tube was immediately mixed with 1 ml pre-warmed SOC medium (37 °C). Afterwards the suspension was incubated at 37 °C and shook at 600 rpm for 1h. Finally up to 800 µl of the suspension (usually 250 µl if purified and concentrated, 500 µl if crude product was used) was plated on LB-Agar plates with corresponding antibiotic using glass beads and incubated at 37 °C overnight.

### DNA hybridization, purification, mutagenesis and modification - Hybridization of oligonucleotides

Pairs of selected complimentary oligonucleotides were hybridized in a 1:1 ratio in the most convenient 1xbuffer for 5 minutes at 95 °C, depending on the downstream application. A list of all used oligonucleotide sequences can be found in Table 1 below.

| | Mutagenesis (NEB2) | | | |
|---|---|---|---|---|
| | (3 pmol of each oligonucleotide) | | | |
| | pHD2 | | pHD5 | |
| | fw | rv | fw | rv |
| | o1494 | o1495 | o1504 | o1505 |
| | o1496 | o1495 | o1506 | o1507 |
| | o1498 | o1499 | o1508 | o1509 |
| | o1500 | o1501 | o1510 | o1511 |
| | o1502 | o1503 | o1512 | o1513 |

| | Mass spectrometry (Glyco buffer 1 or Glyco buffer 2 or NEB4) | | | |
|---|---|---|---|---|
| | (200 pmol of each oligonucleotide) | | | |
| hybridized oligonucleotides | fw | | rv | |
| | o1520 | | o1529 | |

| | PEX Assay (NEB4 or BGrK1) | | | |
|---|---|---|---|---|
| | (0.21 pmol of forward template, 0.2 pmol of reverse primer) | | | |
| | CDKN2A | | BRCA1 | |
| | fw | rv | fw | rv |
| | o1591 | o1082 | o1516 | o1798 |
| | o1592 | o1082 | o1518 | o1798 |
| | o1593 | o1082 | o1521 | o1798 |
| | o1594 | o1082 | o1524 | o1798 |
| | o1595 | o1082 | o1527 | o1798 |

**Table 1: List of oligonucleotides**

| Name SEQ ID NO. | Application | Sequence |
|---|---|---|
| o2471 | rev Sequencing (Seq) primer for C-terminal repeats | GGGTTATGCTAGTTATTGCTCAG |
| o3152 | Seq primer pET32_before_ T7_downstream | CGTAGAGGATCGAGATC |
| o4103 | primer for N-terminal repeats | GAGTAACAGCGGTAGAG |
| o4654 | 1297_C6-→mC | |
| o4665 | 1297_Primer_re v | ggatgtggaaacGgaaga |
| o4766 | 1297 | |
| o5207 | 1297_C6→hmC | |
| o7348 | fw Seq primer for pFUS A and B vectors | TTGATGCCTGGCAGTTCCCT |
| o7359 | rev Seq primer for pFUS A and B vectors | CGAACCGAACAGGCTTATGT |
| o81410 | fw Seq primer for pHD2 and pHD5 | AATGCGCAAACCAACCC |
| o89011 | fw primer for N-terminal domain and repeats | AGATATGATTGCGGCCCTG |
| o108212 | tCDKN2A primer for radiolabeling | GGGAGCAGCATGGAGCCTTCGGCTGA |
| o119113 | QC NNK*/NNK** rev | GGCGATAGCCACCACTTGGTCCGGAGTCAGGCCATG |
| o119214 | QC NNK* fw | |
| | | |
| o119315 | QC NNK** fw | |
| o140816 | Seq primer for N-terminal GFP-domain of TALEs | AGAAGCGCGATCACATG |
| o142217 | 1297_C6→caC | |
| o142318 | 1297_C6→fC | |
| o149419 | mutpHD2 fw G* | |
| o149520 | mutpHD2 rev G* | |
| o149621 | mutpHD2 fw 2* | |
| o149722 | mutpHD2 rev 2* | |
| o149823 | mutpHD2 fw 3* | |
| o149924 | mutpHD2 rev 3* | |
| o150025 | mutpHD2 fw 4* | |
| o150126 | mutpHD2 rev 4* | |
| o150227 | mutpHD2 fw N4* | |
| o150328 | mutpHD2 rev N4* | |
| o150429 | mutpHD5 fw G* | |
| o150530 | mutpHD5 rev G* | |
| o150631 | mutpHD5 fw 2* | |
| o150732 | mutpHD5 rev 2* | |
| o150833 | mutpHD5 fw 3* | |
| o150934 | mutpHD5 rev 3* | |
| o151035 | mutpHD5 fw 4* | |
| o151136 | mutpHD5 rev 4* | |
| o151237 | mutpHD5 fw N4* | |
| o151338 | mutpHD5 rev N4* | |
| o151639 | sense tBRCA1_C13 | CTTCCTCTTCCGTCTCTTTCCTTTT ACGTCA TCCGGGGGCAGACT |
| o153840 | sense tBRCA1_C13→mC | |
| o152041 | sense tBRCA1_C-4→hmC | |
| o152142 | sense tBRCA1_C13→hmC | |
| o152443 | sense tBRCA1_C13→fC | CTTCCTCTTCCGTCTCTTTCCTTTTAfCGTCATCCGGGGGCAGACT |
| o152744 | sense tBRCA1_C13→caC | |
| o152945 | antisense tBRCA1 for MS | |
| o159146 | sense tCDKN2_C6 | |
| o159247 | sense tCDKN2_C6→mC | |
| o159348 | sense tCDKN2_C6→hmC | |
| o159449 | sense tCDKN2_C6→fC | |
| o159550 | sense tCDKN2_C6→caC | |
| o159651 | antisense tCDKN2A for MS | |
| o179852 | tBRCA1 primer for radiolabeling | GCCCCCGGATGACGTAAAAGGAAAGAGA |

The alterations in the sequence (*=deletion) which will result in mutations of the respective module, are highlighted in bold. Underlined parts of the tBRCA1 and tCDKN2A sequences display the target of the 27-repeat long DNA binding domain of the various TALEs.

In cases where primer/template combinations were used, the concentration of the template was individually higher than the concentration of the primer, which is mentioned in the appropriate section.

### DNA hybridization, purification, mutagenesis and modification - Extension of oligonucleotides

3 µl of a 10 mM dNTP-solution and 5U of KF (exo⁻) were added to 2 µM dsDNA oligonucleotides with overhangs at both ends in 50 µl 1xNEB2. Extension of oligonucleotides was done in 2 h at 37 °C. dsDNA oligonucleotides were isolated from the reaction mixture by using the GeneJET Gel extraction kit.

### DNA hybridization, purification, mutagenesis and modification - Purification of DNA, plasmid purification

Overnight cultures of GH317 with the amplified plasmid copies were pelleted for 10 min at 4000 rpm/RT and the supernatant was discarded. The lysis, purification and elution was carried out using the GeneJET Plasmid Miniprep Kit (Thermo Scientific) and following the manufacturer's instructions. After plasmids were captured on the column and washed sufficiently, the product was eluted with 60 µl milliQ H₂O. To potentially increase the concentration of plasmid, the column was incubated at 37 °C for 5 minutes or kept at RT for 30 min before elution.

### DNA hybridization, purification, mutagenesis and modification - Purification of DNA, purification after PCR, complement strand synthesis, labeling or gel electrophoresis

For purification of DNA that was mixed with enzymes (polymerase, glycosylase, restriction enzyme) of single nucleotides (dNTPs, UDP-Glc) the GeneJET gel extraction kit (Thermo Scientific) was used according to the manufacturer's protocol. In cases of purifications without preceding Agarose gel electrophoresis, the volume of binding buffer and Isopropanol was set equally according to the volume of the reaction mixture. Desired dsDNA products were again eluted in 10 to 60 µl milliQ H₂O, depending on the downstream application and amount of recovered dsDNA.

### DNA hybridization, purification, mutagenesis and modification - Purification of DNA, phenol/chloroform extraction

Crude reaction samples with low concentration of modified DNA were often insufficiently concentrated for being used in the GeneJET gel extraction kit. In that case the solution was mixed with an equivalent volume (1 eq.) of Phenol/Chloroform/Isoamyl alcohol (Roth). The sample was thoroughly vortexed for 15 s and subsequently centrifuged for 1 min at 14000 rpm at room temperature. The upper aqueous phase was carefully taken from the lower phase containing the organic solvents and again mixed with 1 eq. volume of Phenol/Chloroform/Isoamyl alcohol to repeat the previous step. After the aqueous phase was put into a fresh tube, 0.1 eq. volume of a 3 M NaOAc (pH 5.2) was added together with 2.5 eq. volume of ice cold Ethanol (EtOH) to precipitate the DNA at -20°C overnight. The next day the DNA was pelleted by centrifugation at 4 °C/14000 rpm for 30 min. The supernatant was carefully decanted and the pellet was let to dry. After all EtOH evaporated, the pellet was resuspended in an appropriate amount of milliQ H₂O.

### DNA hybridization, purification, mutagenesis and modification - Cassette Mutagenesis of pHD2 and pHD5

In order to obtain plasmids with alternated repeat-domain sequences, 2.5 pmol of vectors carrying regular domains (pHD2 or pHD5, respectively) were digested in 1x CutSmart buffer (NEB) by adding 15 U of Ncol and Xhol (both NEB) and incubating the mixture for 1 hour at 37 °C before inactivating the enzymes at 80 °C for 20 minutes. The same procedure was used for 3 pmol of hybridized oligonucleotides carrying the alternated sequence fragment, thus creating repeat-domain sequence fragments with sticky ends. After purifying vectors and oligonucleotides as previously described, both fragments were ligated in 1x T4 DNA ligase reaction buffer (NEB) using 400 U T4 DNA ligase (NEB) over the course of 4 hours at 16 °C. The reaction was stopped by heating the mixture to 65 °C for 10 minutes.

5 µl of the reaction mixture was transformed into GH317 and plated onto LB-Agar supplemented with.

From there single colonies were picked, grown in 5 ml LB-Medium + 12.5 µg/ml Tetracycline and plasmids were isolated using the GeneJET Plasmid Miniprep kit (Thermo Scientific). After the validity of the sequence was confirmed by sequencing, samples with correct plasmids were stored at -20°C.

### DNA hybridization, purification, mutagenesis and modification - Labeling of DNA with [γ-32P]-ATP

10 pmol DNA in 1x T4 PNK buffer A was combined with 1 µl of [γ-³²P]- ATP (0.37 MBq) (Hartman Analytic) and 10 U of T4 Polynucleotide Kinase (PNK) (Thermo Scientific) in a safe environment, suitable for working with radioactive substances. The DNA was labeled for 1 h at 37 °C, before the PNK was inactivated for 10 min at 65 °C. The reaction mixture was purified using the Illustra G-25 column (GE Healthcare) according to the manufacturer's protocol and the pure radiolabeled DNA was stored securely at -20 °C.

### DNA hybridization, purification, mutagenesis and modification - Glycosylation of DNA

1 µg of dsDNA was glycosylated with 1 U T4 β-GT (NEB) and 800 nmol UDP-Glucose with various reaction times and in different buffers (cf. Example 2, infra, for condition specifications). The reaction was terminated by heat inactivation of the glucosyltransferase at 65 °C for 10 min and the glycosylated DNA was recovered by Phenol/Chloroform extraction, if required.

### Engineering of TALE repeats and isolation of the full length TALEs - TALE assembly through Golden Gate reaction

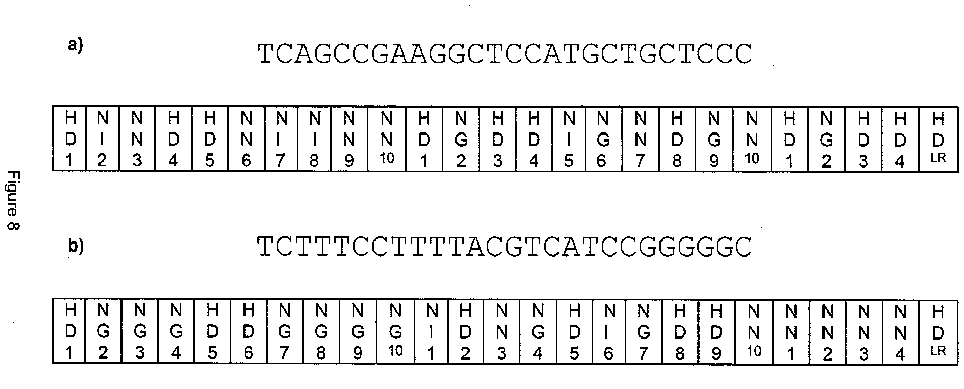

The composition of the TALE DNA binding domain was planned according to the DNA target Sequences for tBRCA1 and tCDKN2a (Fig. 8). The assembly of the TALE proteins was carried out as known in the art with small adaptations. Plasmids with the single canonical TALE repeats (cf. Fig. 8 for relevant sequences for any of the four canonical RVDs) with unique cleavage sites for integration in a pFUS vector at position 1 to 10 were kindly provided, while the plasmids with mutated sequences for HD2 and HD5 were ready to use from previous cloning and purification steps. In the first step of the two-step TALE assembly, the 37.5 ng pFUS vectors were digested and ligated with 37.5 ng of each module vector that provided a single TALE repeat fragments according to Fig. 8 in a 10 µl reaction mixture containing 1x T4 DNA ligase buffer, 40 U T4 DNA ligase (NEB) and 10 U Bsal (NEB). To ensure optimal digestion at 37 °C for 5 min and ligation at 16 °C for 10 min both steps were successively repeated 10 times. After heat inactivation of the enzymes at 50 °C and 80 °C for 5 min each, a plasmid-safe nuclease treatment was performed to remove all non-circular plasmid DNA by adding 5 nmol ATP and 5 U Plasmid-safe Nuclease (Epicentre) and incubating the mixture for 1h at 37°C and subsequently at 70 °C for 20 min.

After the Golden Gate reaction, different tubes contained the first ten repeats integrated in pFUS A30A, the next ten repeats were in pFUS A30B, and the 5^{th} to 2^{nd} last were put into the pFUS B4 vector. All plasmids were transformed into GH317 and plated onto LB-Agar plates supplemented with 50 µg/ml x-Gal and 50 µg/ml Spectinomycine for blue-white screening and selection for the correct antibiotic resistance. Four white colonies from the previous step were used to perform a colony PCR to check for the correct insert length by 1% Agarose GE. One correct colony per different pFUS vector was used for an overnight culture, which was harvested for plasmid isolation by using the GeneJET Plasmid Miniprep kit.

For the second Golden Gate reaction, 75 ng of pFUS vectors A30A, A30B, B4 and the vector for the HD last repeat were combined with 200 U T4 DNA Ligase (NEB), 5 U BsmBI (NEB) and 37.5 ng of the expression/backbone vector with the N- and C-terminal domain (pAnl521) in a reaction mixture of 10 µl T4 DNA ligase buffer. This solution was incubated for 10 min at 37 °C, then cooled to 16 °C and kept for another 15 min. This cycle was repeated 5 times, before a final step at 37 °C for 15 min was carried out. After heat inactivation for 5 min at 80°C, 5 µl of the crude solution was transformed into GH317 cells which were plated onto LB-Agar plates supplemented with 50 µg/ml x-Gal and 50 µg/ml Carbenicillin as written above.

Again, white colonies were picked and a colony PCR was performed and the result monitored on a 1% Agarose gel after electrophoresis. Two of colonies with the correct insert length were inoculated in 5 ml LB-Medium + 50 µg/ml Carbenicillin, incubated overnight at 37 °C and plasmids were isolated as usual. To confirm the DNA sequence of assembled TALE proteins, plasmids were send for sequencing using appropriate primers listed in Table 1, *supra.*

### Engineering of TALE repeats and isolation of the full length TALEs - Expression of TALE proteins

After transformation of *E. coli* BL21 (DE3) Gold or BL21 (DE3) with the respective TALE expression plasmid, a single clone was inoculated and grown in 25 µl LB media supplemented with 50 µg/ml carbenicilin as previously described. The next day, 12.5 µl of the bacterial suspension was diluted 50-fold into LB medium supplemented with 50 µg/ml carbenicilin and the culture was incubated at 37 °C until an OD₆₀₀ between 0.4-0.6 was reached. At that point, expression of proteins was induced by adding 0.2 mM IPTG (Invitrogen). The culture was kept at 28°C for 5 h and finally harvested by centrifugation (15 min, 4000 rpm, RT). The supernatant was discarded without disturbing the greenly pellet. Lysis was performed in Taq-lysis-buffer supplemented with 1 mM PMSF and 50 µg/ml lysozyme by shaking at room temperature at 1400 rpm for 30 min or by sonification (pulse 30s on/30s off, 20 cycles).

### Engineering of TALE repeats and isolation of the full length TALEs - NI-NTA Affinity chromatography for isolation of TALEs

After lysis, the homogenous suspension was centrifuged (20 min, 9600 rpm, room temperature) and the white-grey pellet was discarded. From this point on, it was crucial that all solutions were supplemented with 1 mM DTT. TALE proteins with functional His-Tag domains were bound to 1 ml HisPur Ni-NTA Resin (Thermo Scientific) suspension and extracted from the green-colored supernatant by strong shaking at room temperature for 2 h or overnight. The resin beads were washed with 20 ml 4xPBS three times, 50 ml Qiagen lysis buffer 20 and finally 20 ml Qiagen lysis buffer 50. Fractions of the flow-through were kept for subsequent analysis by SDS-PAGE. TALE proteins were eluted by adding 1 ml Qiagen lysis buffer 500 and shaking the suspension vigorously with the resin beads overnight. The following day, the solution was collected and checked for purity by performing an 8% SDS PAGE. If the resin beads appeared greenly after the first elution, another 1 ml of Qiagen lysis buffer 500 was added and the procedure to recover still bound TALE proteins repeated. Fractions that showed sufficient purity were combined and dialyzed in 1x TALE storage buffer in 5 subsequent steps to dilute the imidazole sufficiently. Concentrations of TALE proteins (CDKN2A→MW: 131.49 kDa, BRCA1→MW: 132.92 kDa) were determined by using the Microplate BCA Protein assay kit (Thermo Scientific) according to manufactures guidelines.

### Methods of analysis - Mass spectrometry

For ESI-TOF mass spectroscopy 200 pmol DNA solubilized in 10 µl miliQ H₂O were sent to metabion. Samples were analyzed in negative mode with a fragmentor voltage of 200 V. Yields were determined from the resulting spectra as ratio of the intensities at the m/z value for o1520_ghmCpG divided by the sum of o1520_ghmCpG and o1520_hmCpG.

### Methods of analysis - Polyacrylamide gel electrophoresis

8% SDS PAGE was used for analysis of purity of expressed TALEs. The resolving gel was prepared from 3808 µl MilliQ-water, 1092 µl acrylamide concentrate (40 %), 420 µl Tris (pH = 8.8), 66.7 µl SDS solution (10 %), 66.7 µl APS solution (10 %) and 6.67 µl TEMED (Roth). Shortly after the addition of TEMED it was swiftly poured into the assembled casting chamber and covered with isopropanol. After polymerization of the resolving gel the isopropanol was taken off and replaced with stacking gel prepared from 1000 µl MilliQ-water, 208 µl Acrylamide concentrate (Roth) (40 %), 420 µl Tris (pH = 6.8), 16.7 µl SDS solution (10 %), 16.7 µl APS solution (10 %) and 1.67 µl TEMED. Samples were diluted with 1/3 volume of SDS-PAGE loading buffer, denatured at 95 °C for 5 min and subsequently loaded on the gel for electrophoresis. The gel was run as described at 80 mA, stained with brilliant blue for 15 minutes and destained in water overnight whilst being gently shaken. For analysis of PEX denaturing gels were prepared by adding 0.4 ‰ (v/v) TEMED and 0.08 % APS to a solution of 5.4 M Urea and 16 % Acrylamide in 1x TBE buffer.

### Methods of analysis - Agarose gel electrophoresis

First, agarose was dissolved in 0.5x TBE Buffer. When the solution became lucid it was poured into the gel casting chamber. A comb was added to create cavities for putting the samples later on. After polymerization the gel was transferred to the running chamber filled with 0.5x TBE buffer. Then, samples diluted with 1/5 volume of 6x loading dye (50% glycerol supplemented with bromophenol blue and xylenecyanol) were loaded into the pockets and the electrophoresis was performed at 120 V, and 30 mA for 1 h 30 min. Afterwards the gel was stained with ethidium bromide and intercalated DNA bands were visualized by UV light.

### Methods of analysis - Primer extension assay

0.2 pmol of hybridized DNA template and primer pairs in 6 µl 1xBGrK1 were incubated with TALE proteins in 6 µl TALE storage buffer at varying concentrations for 30 minutes at room temperature. 12 µl of a mixture of 25 mU Klenow fragment (3'→ 5' exo⁻) of *E. coli* DNA polymerase I (New England Biolabs) and 200 µM of each dNTP in binding buffer consisting of 1x TALE storage and 1x BGrK1 buffer were added. KF (exo⁻) activity was allowed at room temperature for additional 15 min before the reaction was terminated by adding 12 µl of PAGE loading buffer (80 % formamide, 2 mM EDTA) to the mixture and incubating it at 95 °C for 5 min.

Mixtures were loaded onto a pre-run, Urea denaturing PAGE gel and various products separated through electrophoresis in 1x TBE at 60 mA/30 W. For analysis the gel was dried under reduced pressure at 90 °C, subsequently applied on a phosphor screen and read out with a phosphorimager the next day.

### Design of DNA oligonucleotides for cassette mutagenesis of TALE repeats

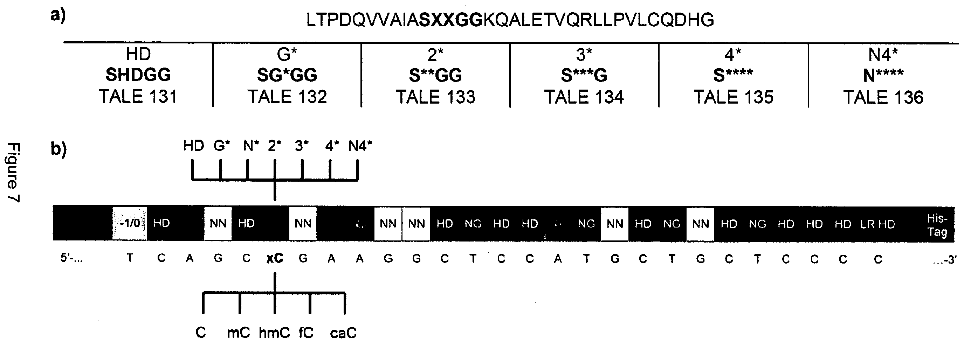

In the target sequence for CDKN2A TALEs (Fig. 8) the 6^{th} nucleotide was the cytosine that was substituted with the respective C5-modification. TALE repeats had a highly conserved sequence, except for aa 12 and 13, which represent the RVD. For the TALE repeat targeting the modified cytosines, mutations were introduced as displayed (Fig. 7) by designing overlapping oligonucleotide pairs complementary to the repeat sequence but with triplet codon deletions or substitutions.

At both ends these oligonucleotides were given unique restriction enzyme cleavage sites, which are required for later integration in the plasmid. For cutting the original pHD2 plasmid at the determined sites NcoI (CCATGG) and XhoI (CTCGAG) were suitable. Respective recognition sequences were added either to the 3' or 5' end of oligonucleotide pairs which were protected by polyA (sense) or polyT (antisense) sequences to rule out unspecific pairing of the oligonucleotide with the plasmid in the mutagenesis.

### DNA sequencing

5 µl of an 80-100 ng/ µl DNA solution were combined with 5 µl of an aqueous solution containing 5 µM primer (cf. Table 1 for primer sequences) and sent to GATC for sequencing. Sequence traces were analyzed with Finch TV software. Sequencing results were copied from Finch TV and pasted to Serial Cloner for editing and sequence alignment.

### Analysis of PEX assays

Phosphoimaging pictures were analyzed and adjusted in Quantity One. The raw data was transferred to Microsoft Excel and subsequently processed by calculating standard deviations and plotting the mean values on bar graphs or in dose-response curves.

### Design of figures, charts and graphs

Figures were designed using Microsoft Word, Paint and CorelDRAW X6. Charts were created in Microsoft Excel and Origin 2015. Data of TALE repeat structures was obtained from PDB entry 3UGM and modelled in PyMOL.

### Example 1:

### Universal detection of epigenetic cytosine derivatives by engineered TALE repeats

Cytosine is selectively recognized by the HD RVD of a DNA binding domain repeat in a TALE. Previous experiments revealed that if this unit is substituted by a N* RVD, not only cytosine but also 5-methylcytosine can be accommodated.

A recent study showed that incorporation of small amino acids at or even the deletion of the 13^{th} position leads to the recognition of mC. Although the ability for selective sensing decreases by reducing the loop structure, the repeat does discriminate between mC, C and hmC, caC (unpublished data).

Going forward from that proof of principle, reducing the structure of repeat loops even further could remove hindering RVD-nucleobase interactions and lower the steric demand of the repeat units which would consequently lead to non-selective binding of cytosine, all of the other mentioned epigenetic variants and potentially other canonical nucleobases. Therefore several promising candidates for a promiscuous repeat were conceived and used to assemble six TALEs targeting a 27 nt long sequence of CDKN2A (tCDKN2A) of which each contained a different size reduced repeat (Fig. 7, Fig. 8).

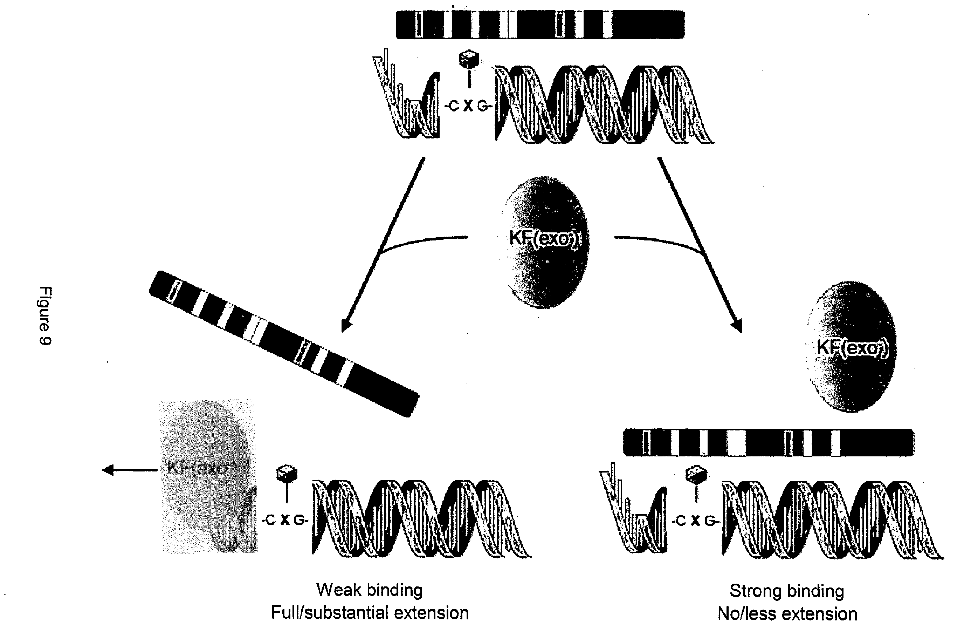

To analyze binding to the template DNA, TALEs were tested in a primer extension (PEX) assay for their ability to prevent polymerase activity at the target. Oligonucleotides containing C, mC, hmC, fC or caC at the 6^{th} nucleotide of tCDKN2A were hybridized with ³²P-labeled primer and incubated with both TALE and the Klenow Fragment (3'→5' exo⁻) of the DNA polymerase I (KF (exo⁻)), which will extend the primer to create blunt end dsDNA oligonucleotides (Fig. 9). Firm TALE-DNA interaction will interfere with docking of KF (exo⁻) to the primer-template complex and thus inhibit polymerase activity. The resulting amount of extended radioactive oligonucleotide will be reverse proportional to the inhibitory effect of the TALE which allows qualitative and quantitative analysis of binding efficiency of TALEs to the DNA and therefore permit a conclusion of the ability of the mutant TALE repeat to bind Cytosine and tested derivatives.

All in Fig. 7 a) listed TALEs were separately tested twice and at two DNA:TALE ratios of 1:5 (8,33:41.65 nM) and 1:20 (8.33:166.66 nM) to rule out false-positive hits and to identify to what extent TALEs would bind to the different nucleobases.

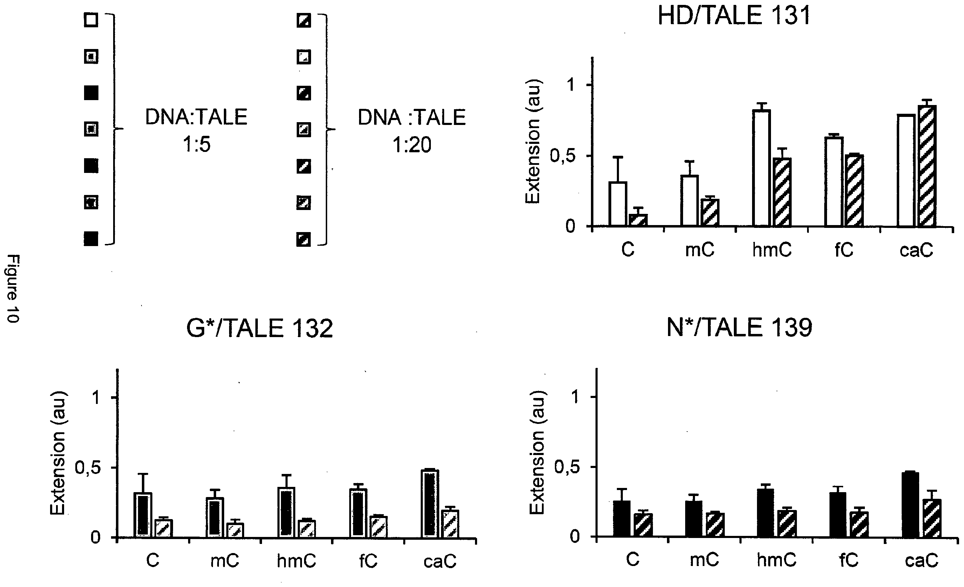

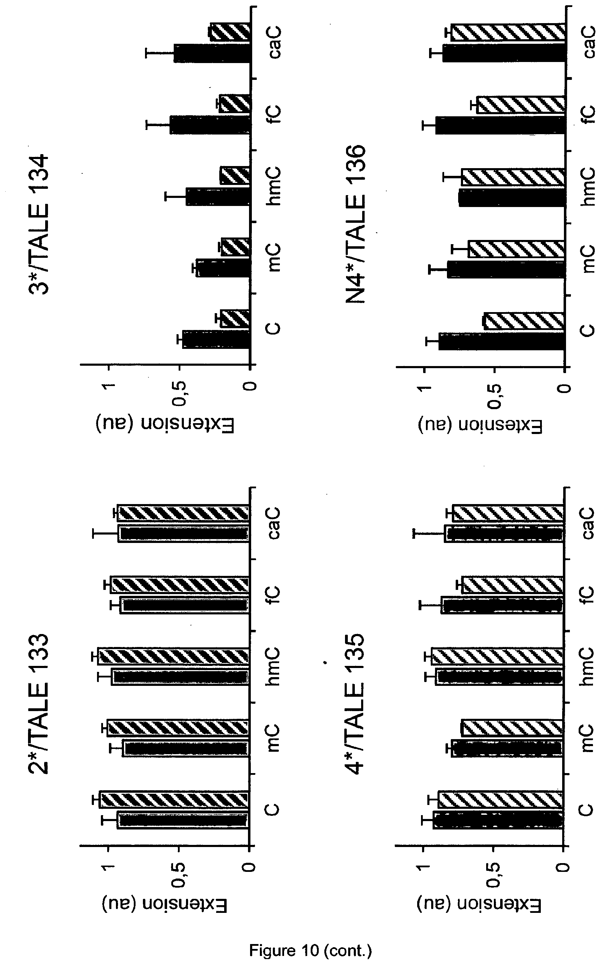

At a ratio of 1:5 and 1:20 TALE 131 (white bars, Fig. 10) with the canonical HD RVD in the 5^{th} repeat shows strong binding to cytosine and also mC but only weak binding to the oxidized mC derivatives (Fig. 10). Whereas the results for hmC, fC and caC were expected, the interaction with mC could be explained by TALE length effects, because longer TALEs are more susceptible for compensation of mismatches in TALE-DNA pairing. If the remaining 26 nt are tightly bound, the weak binding of the HD RVD to mC could be enough to result in an overall stable interaction of the TALE 131 with tCDKN2A_C6 → mC that can overcome the 2-3 fold reduction in sensitivity.

TALE 133 (2*, yellow bars, Fig. 10), 135 (4*, green bars, Fig. 10) and 136 (N4*, blue bars, Fig. 10) show no to little overall potential to inhibit polymerase activity. The high extension rates caused by the weak binding of TALEs are probably due to the deletion of the RVD (2*) or even the whole loop (4* and N4*) (Fig. 10). However, there is a substantial decrease in primer extension for N4* for C and fC which is not observable for the TALEs with the size reduced 4* or 2* repeat. This could indicate a noteworthy effect of the substitution of Serine (Ser, S) with Asparagine at the 10^{th} amino acid of the repeat.

Another explanation for the observed effect of TALEs not binding to their target could be a partial (loss of the RVD; 2*, TALE 133) or total deletion of the loop (residue 12 to 15; 4*, TALE 135, N4*, TALE 136). The reduction or lack thereof may result in an altered tertiary repeat structure which could go as far as refolding to regain a loop structure or planarization of the repeat by horizontally converging orientation of the α-helices that exhibits repulsive effects.

Another noteworthy observation is that no detectable difference in TALE binding for the absence or presence of two Gly residues in the smaller loop of the engineered TALE repeat can be seen. This is interesting because G14 and G15 usually coordinate the phosphate of the sense strand DNA through water-mediated H-bonds with their backbone carbonyl oxygen and amide groups which contributes to nucleobase-unspecific DNA-TALE interaction.

While the first Gly is reportedly essential because the appearance of an amino acid with a functional group at this position may cause steric clash with the DNA backbone, the second Gly is replaceable. This indicates that either the presence of the Glycine residues does not provide an effect which would be proficient enough to cover the missing of the RVD or that they are shifted to a different orientation where they cannot contact the backbone (2*, TALE 133, grey bars, Fig. 10).

In general, transposition of amino acids to atypical positions could potentially prevent DNA-TALE interactions. For example, Lysine16 (K16) and Glutamine17 (Q17) are directly adjacent to the loop and form a shallow surface pocket that allows interaction with the phosphate groups of the DNA backbone through hydrogen bonds of the amino moieties. It is reasonable that if the loop structure is modified, K16 and Q17 would be reallocated to a different site from which they could either not contact the phosphate group anymore or might interact repulsively with the DNA.

This inability of the TALE to bind to the targeted DNA could be probably enhanced the absence of the Histidine12, which is known for stabilizing the canonical repeat structure. The structural instability in 2*, 4*, N4* repeats might even affect neighboring canonical repeats which could partially alter the structure of the TALE in this region.

Contrary to the TALEs 132 (2*), 135 (4*) and 136 (N4*), three TALEs showed overall high inhibition of KF (exo⁻) and thus display promiscuous interaction of the engineered repeat with the epigenetic nucleobases. Most surprisingly, repeats with the N* RVD displayed very low primer extension for tCDKN2A templates with cytosine and each of its 5-modifications (black bars, Fig. 10), whereas this repeat has been previously reported to only bind C, mC and fC to some extent in a different sequence context (unpublished results).

This could be an array-length dependent effect or the result of the position of the module in question. Additionally, it would be of great interest to obtain structural data for all size reduced, engineered repeats but computational modelling could as well give valuable insights in the way those repeats are organized so that reason for the various efficiencies and selectivities in binding could be explained.

Because the results of N* are contradictory to published results, the following data for the two remaining TALEs 132 (G*, gray, Fig. 10) and 134 (3*, red, Fig. 10) might be conditional and could only be applicable to this context.

As implied, both TALEs did efficiently block polymerase activity comparable to TALE 139. The repeat with the G* RVD had the highest overall affinity towards the target, not differentiating if C or any of the modifications were present. 3* had strong binding tendencies as well, but those were always slightly but significantly less efficient compared to G*. The strong inhibitory effect of the TALEs bearing the G* RVD or mutated 3* repeat could for example be explained by a decreased steric demand of the loop that exhibits lower obstructive effects than the HD RVD for hmC, fC and caC, thus enables universal TALE binding to tCDKN2A with each tested nucleobase.

Through the deletion of H12 and a substitution of D13 with a glycine, a loop of 3 glycines is introduced in the G* RVD of TALE 132 that in contrast to the 4* or N4* repeat seem to facilitate non-specific binding to DNA with epigenetic cytosine modifications. A similar observation can be made for TALEs with a repeat in which the RVD and G14 (TALE 134) are deleted so that only a single glycine remains between the residues Serine11 (S11) and K13 (K16 in the HD repeat). In combination with the aforementioned findings, there is a possibility that the observation for G* and 3* are in accordance to findings of the involvement of Gly at residue 13 and 14 (G12 for 3*) for DNA backbone binding and thus provide a scaffold to accommodate cytosines mainly based on van-der-Waals interactions as it was suggested for the N* repeat. Contrary to 2*, 4* and N4* this would indicate that repeats with the G* RVD or a deletion of 3 amino acids in the loop region display a significantly different organization in their structure that either promotes TALE binding or reduces obstructive effects introduced by RVD (2*) or loop deletion (4*, N4*). This could include a favorable orientation or conformation of K15 and Q16 (K13, Q14 for 3*) side chains which could establish or restore attraction but might as well remove potential repulsive interactions.

Additionally, it has been reported that TALE repeats of unusual amino-acid sequence length can introduce a local flexibility to TALE-DNA binding. This could be facilitated by either one of the three or in case of TALE 134 the only remaining glycine residue which could potentially interact through van-der-Waals forces or through water-mediated H-bonds with their respective 5-modification of the cytosines, but it could also just be an unsophisticated element that accept nucleobases despite their different molecular properties and steric effects.

It is also unclear, how TALEs with an ability to universally bind the cytosine and the 5-modifications will respond to size-increased derivatives. If size reduced repeats wouldn't be able to cope with bulky moieties, the drop in affinity for a certain nucleobase would allow identification of the epigenetic cytosine derivative based on a highly chemoselective tagging method.

Glycosylation of hmC is such a highly selective modification method used in typing and profiling approaches. Furthermore, because of its abundance in the human genome and implied importance in biological processes, hmC seemed the most appropriate derivative to be tested. Investigating the effect of an hmC-conjugate with increased steric demands on recognition by the G* and 3* repeat in TALE 132 and 134, respectively, might enable highly resolved, programmable identification of hmC in a user-defined context.

### Example 2:

### Impact of glycosylation on TALE binding - Glycosylation of hmC by T4 β-glucosyltranserfase

The enzymatic glycosylation with T4 β-glucosyltransferase (T4 β-GT) is well established and has been used for over 50 years. More recently, this reaction was also used to quantify hmC in genomic DNA analysis assays because of all epigenetic cytosine modifications only hmC is labelled.

For the purpose of the present invention, the chemoselective glycosylation was used to increase steric demands between hmC and all other cytosines. Through the addition of structural elements to the nucleobase, the nucleotide will increase in size which will be likely affecting the interaction of TALE and DNA thus possibly induce steric repulsion based on the size and other molecular properties of the additional moiety.

The reaction also has the advantages in that it works both *in vivo* and *in vitro,* is inexpensive and fast, as conversions in as low as twenty minutes at room temperature (RT) with good yields have been reported.

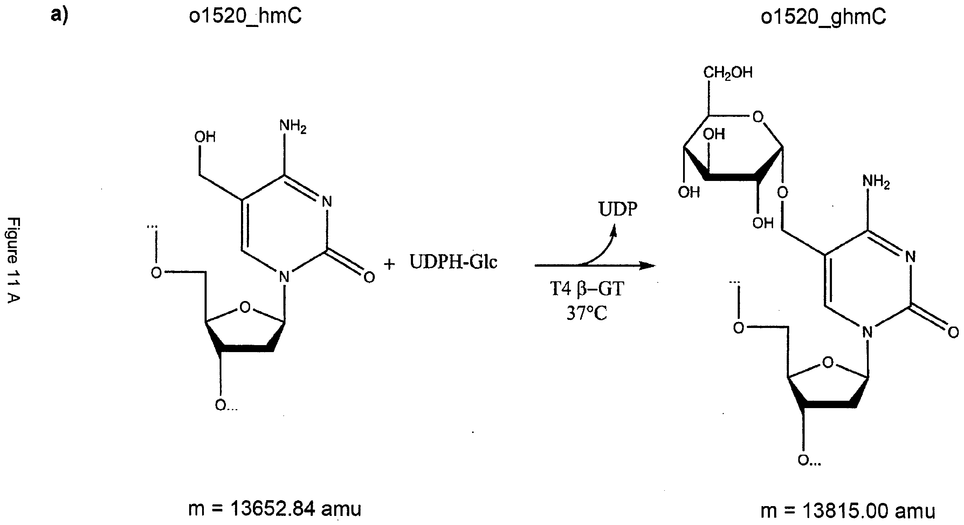

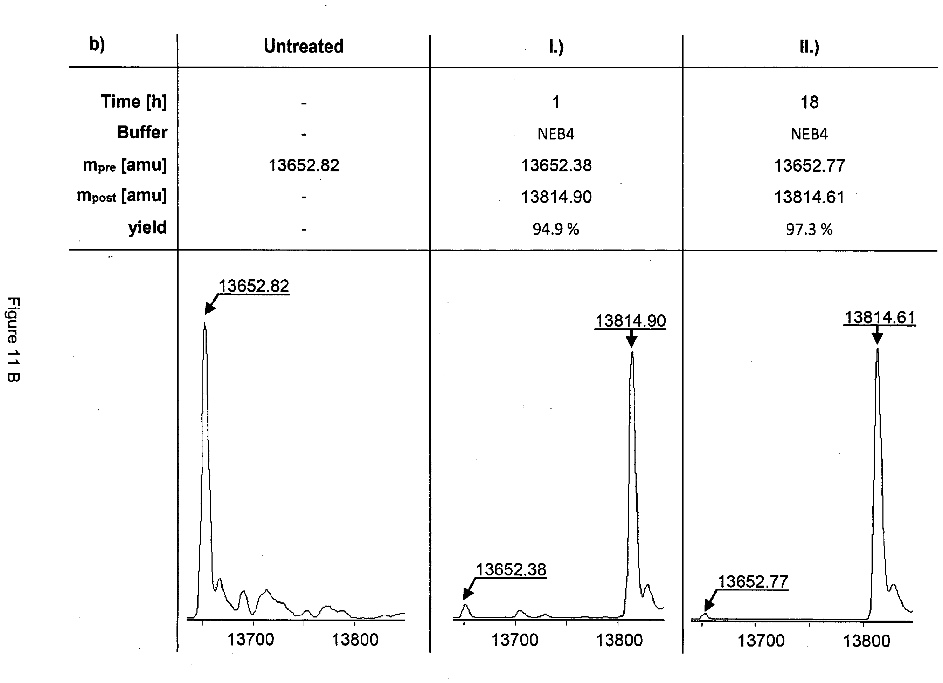

However, two different reaction conditions for the glycosylation of dsDNA were available to work for the commercially available T4 β-GT. To determine which of those would convert more hmC to the glycosylated product, 1 µg DNA in 50 mM KOAc, 20 mM Tris-OAc, 10 mM Mg(OAc)₂, 1mM DTT (NEB4) were incubated with 1 U T4 β-GT and 40 µM Uridinediphosphate Glucose (UDP-Glc) for I.) 1 h or II.) 18 h, respectively (Fig. 11 a)). Independent of the conditions, glycosylation reaction were terminated by heat inactivation of T4 β-GT at 65 °C for 20 min.

Purified glycosylated DNA oligonucleotides were then analyzed by electrospray ionization-time of flight (ESI-TOF) mass spectrometry (Fig. 11 b), c), d)). The calculated difference of 162 amu (atomic mass units) for the glucosyl moiety was found in both spectra and comparison of the relative abundances of peaks around 13652 (o1520_hmCpG) and 13815 amu (o1520_ghmCpG) revealed different conversion efficiencies for each reaction condition (Fig. 11 c), d), e)).

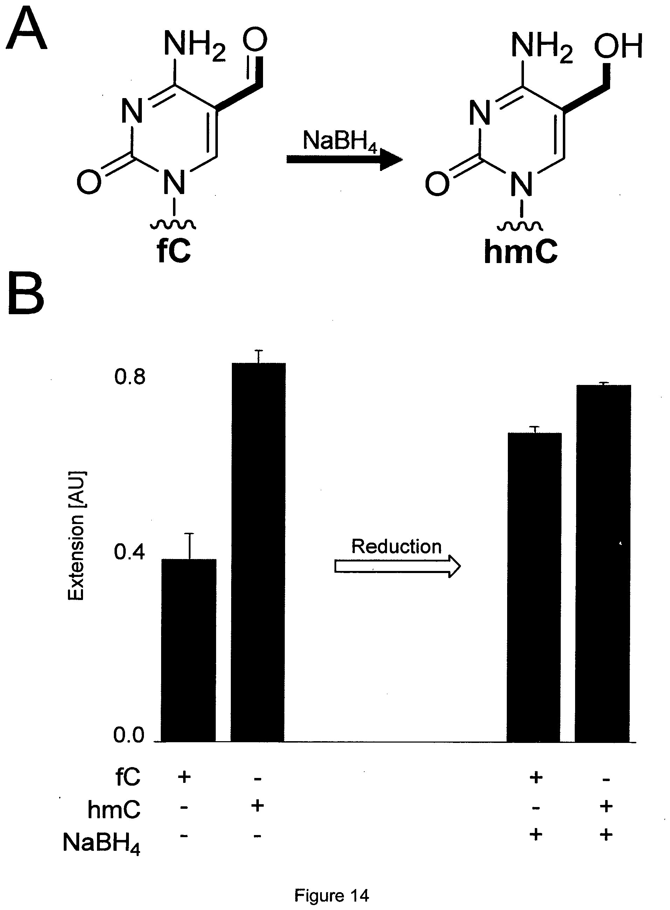

After 1 h already 94.9 % of the starting material were glycosylated (Fig. 11 c)), whereas 97.3% of the remaining starting material was converted after 18 h (Fig. 14 d)), which confirmed the applicability of this reaction for efficient glycosylation.

### Example 3:

### Impact of glycosylation on TALE binding - TALEs respond selectively to glycosylation of hmC

After performing glycosylation of tCDKN2A_C6 → hmC, 166.6 nM (1:20) TALEs with the canonical HD RVD (TALE 131), G* RVD (TALE 132), N* RVD (TALE 139) and 3* (TALE 134) repeat were tested for their response to β-glucosyl-hmC (ghmC) in the DNA target with tagged hmC.

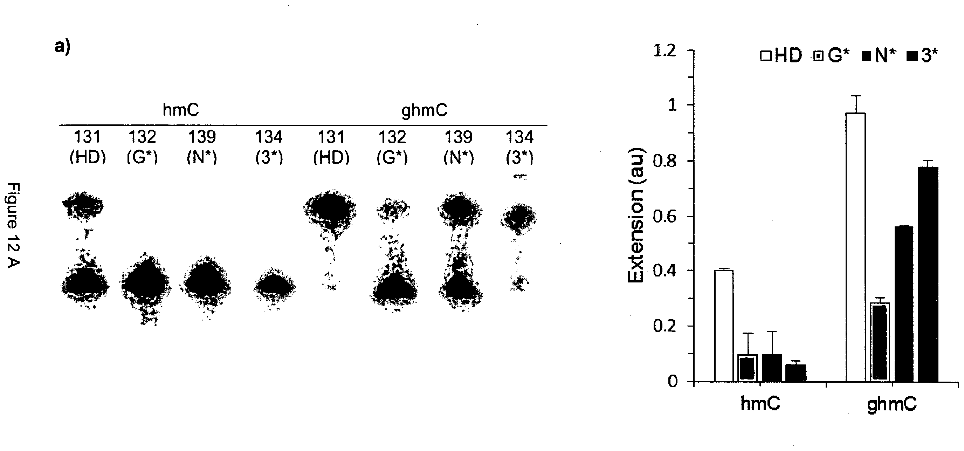

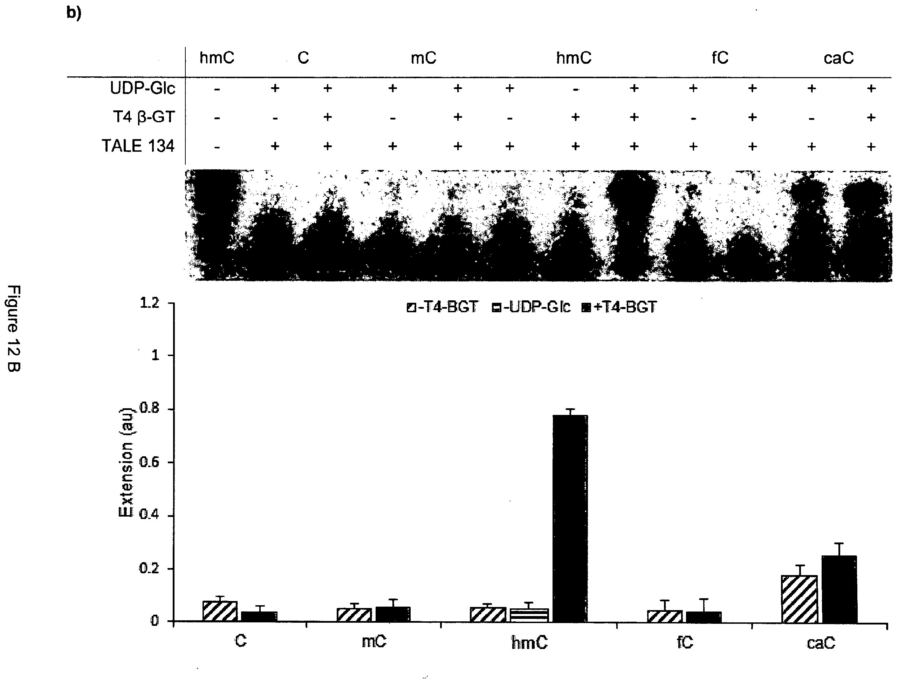

As previously demonstrated, the binding of the canonical HD repeat is highly selective due to its steric demand and will lead to steric clashes with oxidized mC-derivatives (Fig. 10). Expectedly, full primer extension was shown for TALE 131 with tCDKN2A_C6 → ghmC (Fig. 12 a), white bars, 0.972 ± 0.064 au). Additionally, the value was raised by more than 2-fold compared to hmC, which emphasizes the impact of glycosylation on DNA binding of TALEs despite potential constructive TALE-length and -composition effects.

An approximate 2-fold increase in primer extension can also be observed for the G* repeat. However, TALE 132 displayed much lower values compared to TALE 131 thus presented also elevated KF (exo⁻) inhibition for the target sequence with ghmC (Fig. 12 a), grey bars, 0.285 ± 0.002 au). These values demonstrate the ability of the G* repeat to accept the glucosyl residue with only minor additional repulsive consequences compared to caC (0.198 ± 0,027 au, Fig. 10).

This assumption is supported by the presence of asparagine (Asn, N) instead of glycine, which leads to an increase in steric repulsion as demonstrated by an increase in primer extension (Fig. 12 a), black bars, 0.561 ± 0.003 au). This might be because the carboxamide of asparagine has been reported to stabilize the canonical repeat structure, just like in the imidazole group of the histidine residue. Thus, H12G might add a certain flexibility to the G* repeat structure as G12 presumably cannot bond with the carbonyl group of position 8. This leads to a wobbly structure of the loop that could bear the glucosyl moiety whereas in case of the N* repeat this structure is more compact and displays a higher steric demand. However, in regard to HD, both G* and N* repeats have a deletion of amino acid 13 which would explain why the steric hindrance for ghmC is lower.

One could expect that there is even less hindrance in case of the 3* repeat. But surprisingly, amongst all tested TALEs with engineered repeats, TALE 134 (3*) showed the strongest selective modification response which comes closest to the levels of extension observed for the canonical TALE with the HD RVD (Fig. 12 a), red bars, 0.778 ± 0.027 au).

Compared to N* and G*, the decrease in the ability of the 3* repeat to accommodate ghmC might be a consequence of inductive effects that were induced through the different position of the repeat-typical α-helices that are theoretically just separated by a single Gly at position 12. This could lead to reorientation of G12 neighboring amino acids, which might induce a repulsive force between the amino acid(s) and the ghmC.

Remarkably, because of the effectiveness in binding to hmC, the response of TALE 134 is over 5-fold stronger than for TALE 131. This indicates the successful establishment of a selective modification response for 3* which now allows precise identification of hmC.

It remains elusive however, if and to what extent this would be applicable in a heterogeneous mixture of multiple cytosine derivatives and how sensitive the detection would be in genomic analysis for example. Therefore it is essential to determine first the uniqueness of this effect being purely dependent on the glycosylation and second to evaluate binding affinities of TALE 134 to the different DNA templates.

To test the previous, T4 β-GT and UDP-Glc were added to the full set of cytosine derivatives and were kept at 37 °C for 18h. Additionally, two samples were prepared, which contained besides tCDKN2A_C6 → hmC either only UDP-Glc or only T4-glucosyltransferase to demonstrate that the decrease in binding is not caused by prevention of TALE binding by UDP-Glc or because of unspecific interactions of T4 β-GT with KF (exo⁻), the TALE or the DNA.

Indeed, only for hmC containing DNA a significant increase in extension could be observed but only if substrate and enzyme are present (0.784 ± 0.057 au) (Fig. 12 b)). This shows that neither C, mC, fC nor caC was affected by T4 β-GT and therefore the glycosylation of hmC is an unbiased, highly selective labelling method that could be used for determination of hmC contents at user-defined target sequences.

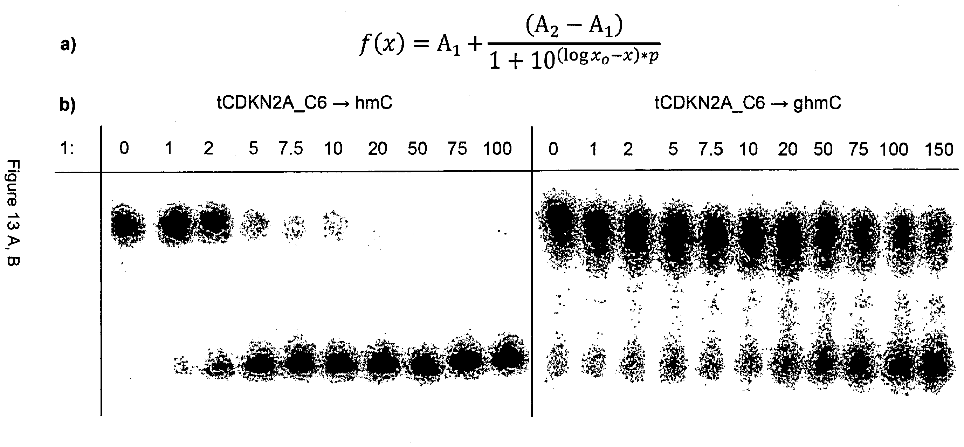

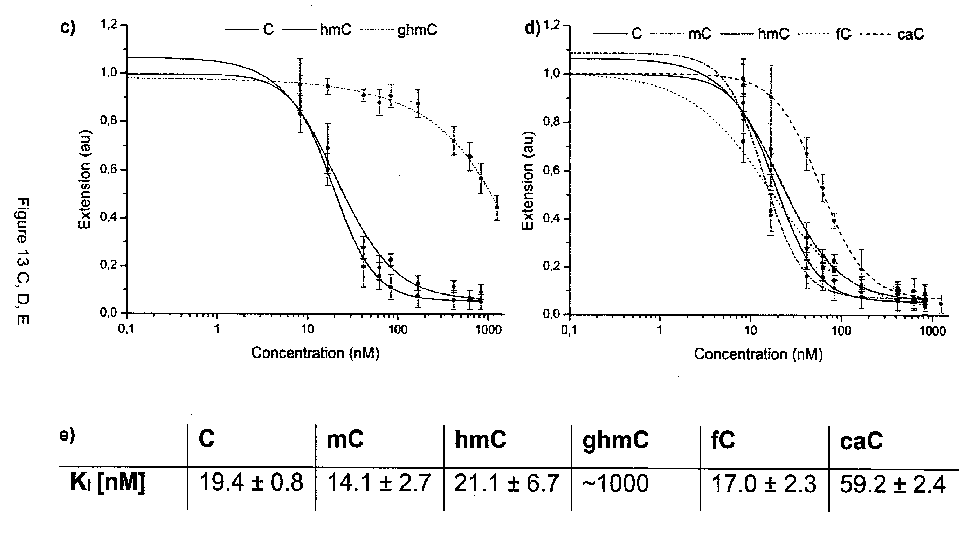

To gain quantitative insights on the influence of all functional groups of the different cytosines on the binding affinity, a dose response of 8.3 nM labeled dsDNA was plotted accordingly for an increasing concentration of TALE 134 (Fig. 13 b), c)). This enabled the determination of inhibitory constant (Kᵢ, also referred to as IC50 or xo, Fig. 13 a)) which represents the point at which the concentration of TALE 134 results in 50% obstruction of 25 mU KF(exo⁻)-mediated primer extension activity in regard to monitor the effect of C5-Cytosine modification on TALE binding.

Analysis by denaturing PAGE illustrates the distinct impact of ghmC on TALE 134-DNA interaction (Fig. 13 b)), while plotting of the obtained values reveal a shifted course of the KF (exo-) mediated primer extension curve and inflection point compared to CDKN2A target sequences with the 5-hydroxylmethyl derivative (Fig. 13 c)).

Because of this weakened binding, even at the highest applied concentration of TALE 134 (1.25 µM) the corresponding extension curve has not passed half of its course towards its lower boundary. This makes the exact calculation of an inhibitory constant of TALE 134 in the PEX assay with tCDKN2A_C6 → ghmC impossible but with the given trend the estimated IC50 value would presumably range around ∼1 µM. In contrast, Kᵢ values for C (19.4 ± 0.8 nM), mC (14.1 ± 2.7 nM), hmC (21.1 ± 6.7 nM) and fC (17.0 ± 2.3 nM) are approximately 50-fold lower (Fig. 13 b), d)), whereas the curve for extension of a primer hybridized to a caC embodying oligonucleotide differs significantly from the aforementioned unlabeled 5-modifications (Kᵢ=59.2 ± 2.4 nM) but still equals more than a 15-fold reduction compared to Kᵢ(ghmC) (Fig. 13 c), d), e)).

These results elucidate that the different reactivity of C, mC, hmC, fC and caC can be used to selectively introduce a glucose-moiety into hmC which extensively alternates the TALE affinity to its target sequence and would presumably allow precise typing of hmC at high resolution in genomic analysis.

Interestingly, because of the significant difference of TALE binding to tCDKN2A_C6 → caC, there might be a possibility to differentiate caC from other 5-modifications and cytosine without the need for a selective modification response based on the low, but still meaningful steric demand of the 3* repeat.

The repulsion of nucleobases with increased steric effects like in the case of 5-carboxylated cytosine might be caused by interaction of the functional groups at the C5-position of cytosine derivatives with amino acid residues or the position of thereof. The methyl group of mC could interact through van-der-Waals-interaction with the G12 in the 3* repeat, while the flexible orientation of the hydroxyl group of hmC, which can freely rotate but likely faces away from the repeat towards the 3'-end of the DNA strand, would allow a favorable conformation of this group that supports or at least does not disturb interaction of the repeat with the nucleobase. Finally, the 5-formyl group has been shown to engage a hydrogen bond with the 4-amino group of 5-formylcytosine. If this bond persists under the conditions tested, the formyl group would not be oriented towards the repeat, thus the steric effect should be reduced. For the 5-carboxyl moiety of caC the orientation of the oxo group is similar to the formyl residue of fC, however the additional hydroxyl group of the 5-carboxyl unit would be oriented towards the 3* TALE repeat. This could be a potential cause for a steric clash of the structures which then could explain the -3-fold lower binding affinity of TALE 134 for caC.

In contrast, the enormous increase of obstruction in TALE binding to the target bearing a bulky glycosylated hmC might be the effect of just the sheer size of the glucosyl moiety which could lead to separation of the target from the TALE. Therefore it should be of interest to examine the response for other selective labeling methods, but for fc and caC, in order to explore the ability of the 3* repeat to precisely type hmC, fC and caC with three highly chemoselective, preferably biocompatible labeling methods *in vitro* and even *in vivo* assays.

In summary, the presented data demonstrate a glucosyl-dependent effect for DNA binding of TALE 134 (3* repeat) that allows differentiation of 5-hydroxylmethylcytosine from every other cytosine modification. For other TALEs this effect might be empowered or weakened by the composition and structure of the RVD containing loop of TALE repeats, which is displayed for example by the G* repeat through TALE-dependent inhibition of polymerase activity related to substantial TALE-nucleobase interaction with ghmC.

Different labeling reagents might on the one hand lead to even stronger responses of the 3* repeat for hmC and might then also potentially enable the G* RVD as another promising candidate, which has been shown to visibly bind better to CDKN2A targets.

### Example 4:

### Molecular Recognition of fC and caC

Next, the sensitivity of TALEs to the two remaining oxidized cytosine variants fC and caC was investigated. For this, TALEs T-drh103, T-drh106, T-drh107 and T-drh108 bearing RVDs HD, NG, N* and S*, respectively, in repeat 5, were incubated with target DNA bearing fC or caC opposite the investigated RVD. Then, primer extension reactions were performed as above and analyzed by denaturing PAGE. The same was performed for Sequence context S-drh2 using TALEs T-drh204 and T-drh205 bearing RVDs N* and S*, respectively.

The results revealed that fC is bound by RVDs N* and S* efficiently, leading to inhibition of primer extension. These results are in agreement with reported structural data where fC forms intramolecular hydrogen bonds with the amine function at the 4 position of cytosine, with the aldehyde oxygen being fixed to point into the direction of the amino moiety, providing a similar face to the major groove as 5mC. On the other hand, caC is not bound by any of the RVDs, interestingly in S-drh2 where some binding to hmC was observed a clear decreased affinity to caC has been observed, when compared to hmC. This may be caused by an overall higher steric demand of caC or by the difference in polarity. This data mean, that hmC and caC and fC, 5mC and C cannot be reliably distinguished by RVDs N* and S*. Due to the different sensitivity of N* and S* to fC and caC, this approach was coupled to selective chemical reduction of fC to hmC using sodium borohydride (Fig. 14). When comparing the primer extension assay results of untreated and reduced DNA, fC can be distinguished from 5mC. As shown in Figure 14, after treatment of fC containing DNA with sodium borohydride an increased primer extension product formation, similar to hmC containing DNA, can be observed. This shows a decreased binding affinity of TALE protein, bearing RVD N* opposite the investigated cytosine. This indicates successful and selective reduction. Coupling of the method to reported selective oxidation of hmC to fC with potassium perruthenate is not suitable due to the denaturing reaction conditions, making the application to genomic DNA inconvenient.

In conclusion, employing a simple chemical conversion of fC to hmC makes it possible to differentiate between C, 5mC, hmC/caC and fC using TALE proteins. Differentiation ability between hmC and caC has still to be achieved, but due to the much lesser occurrence of caC, compared to hmC, the applicability of a purely binding based assay may be challenging.

### Example 5:

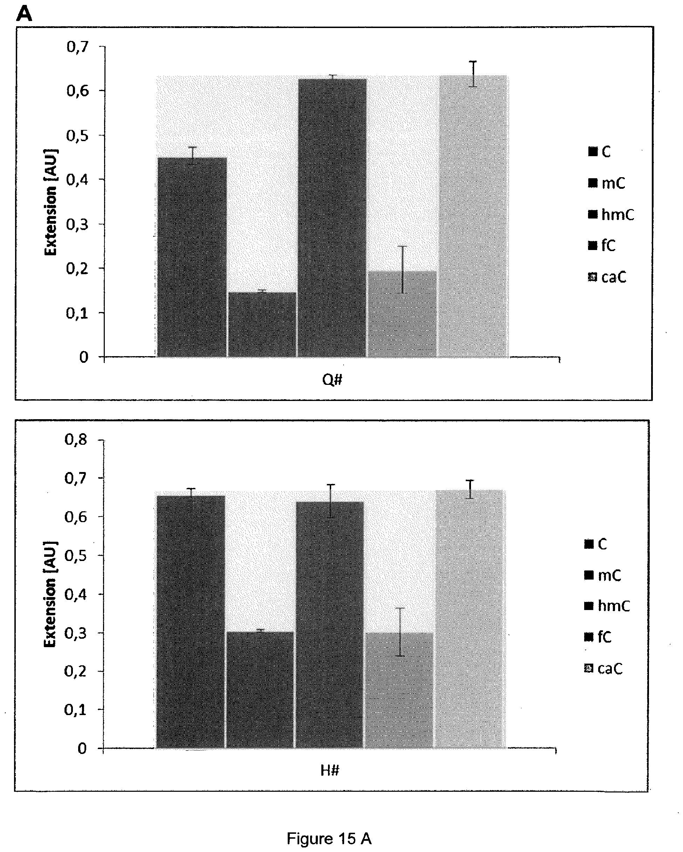

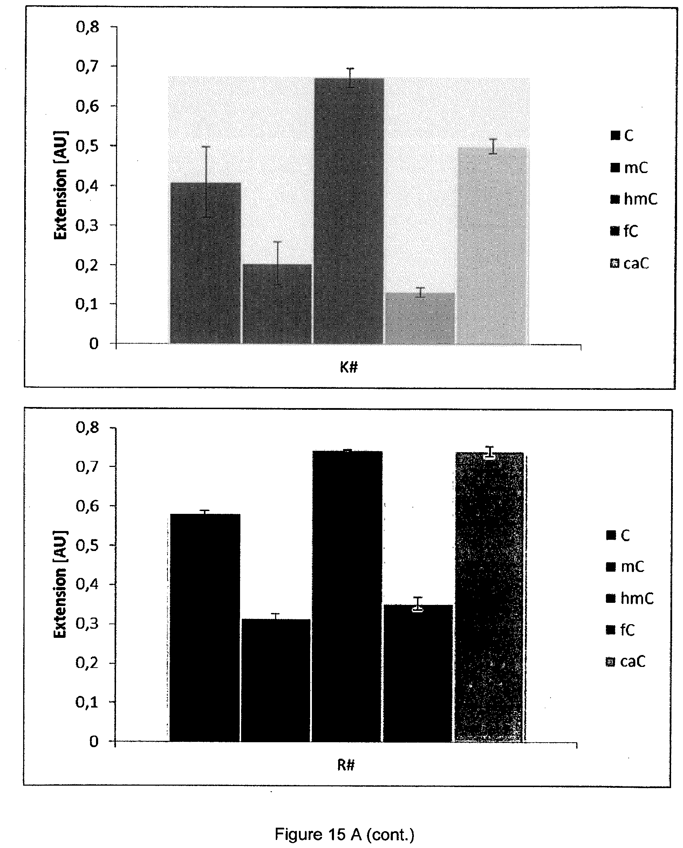

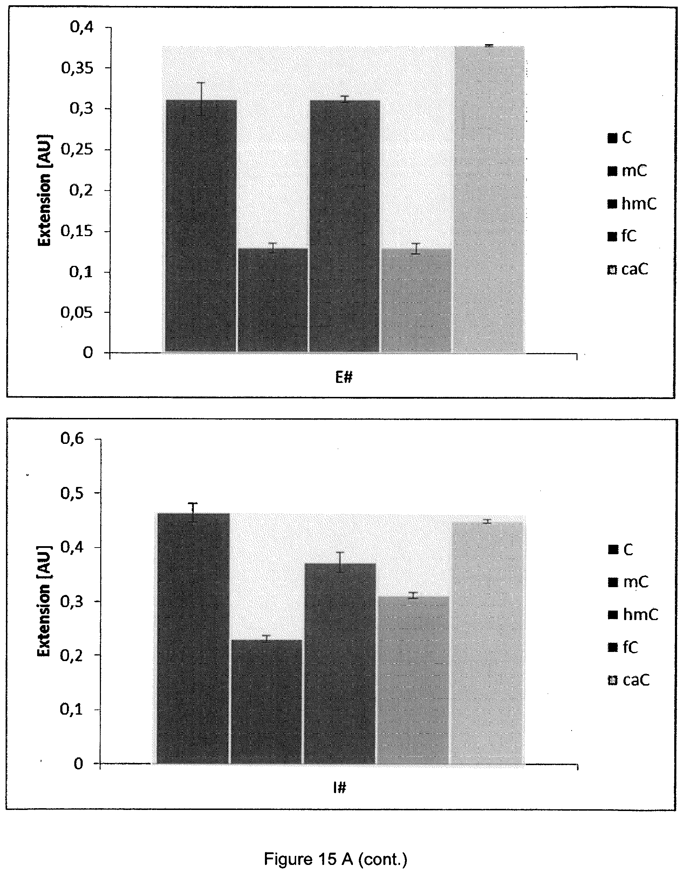

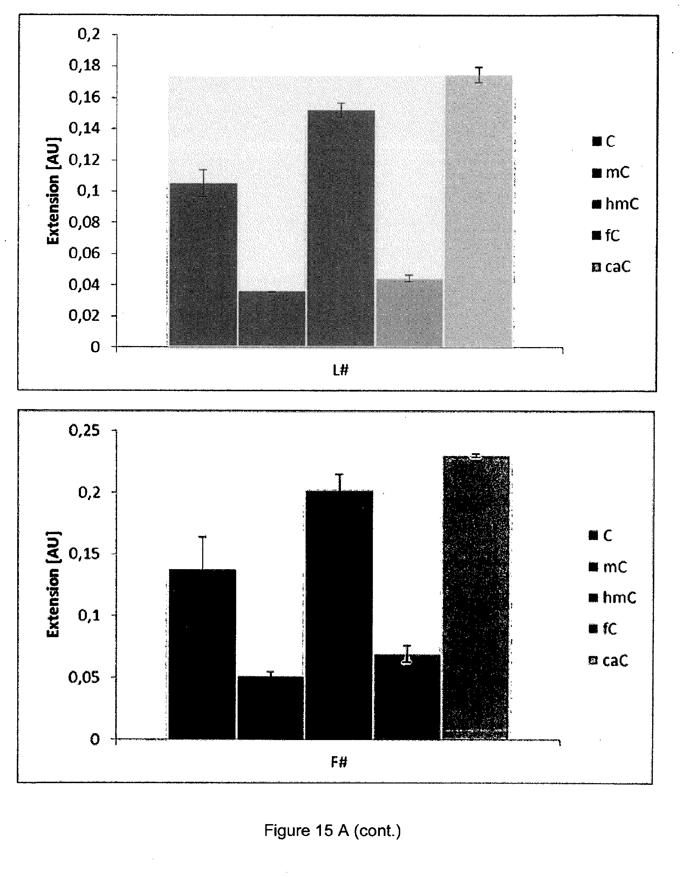

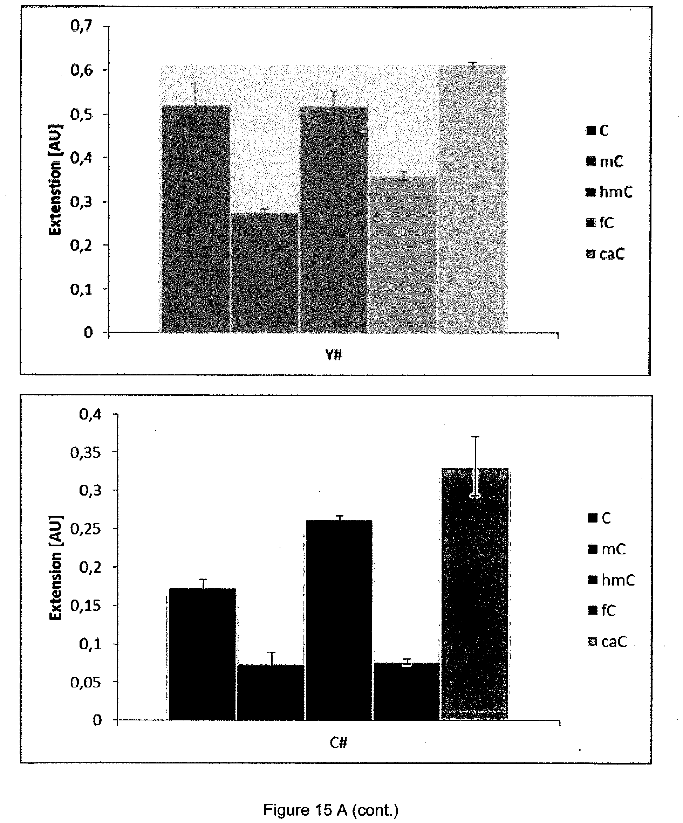

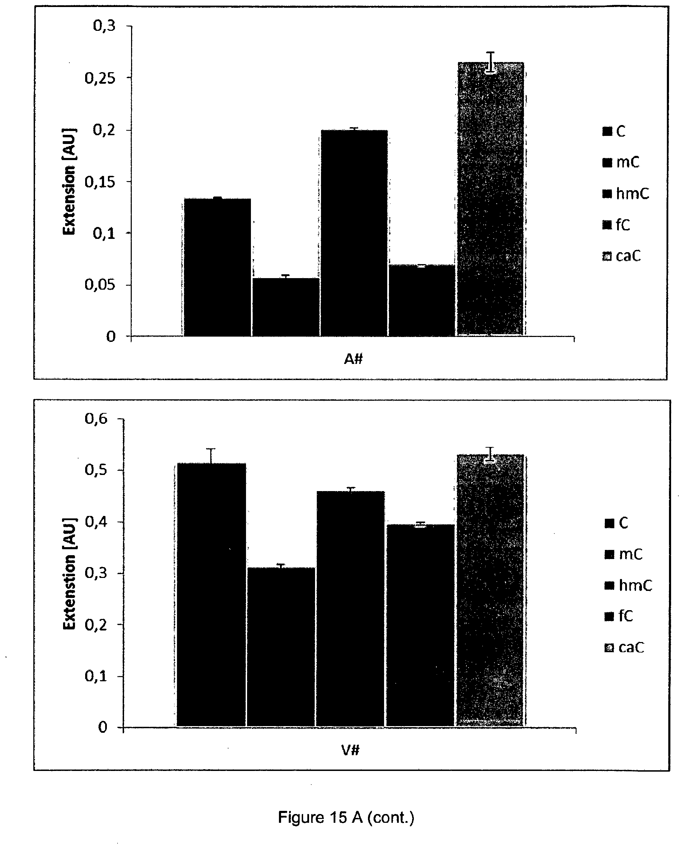

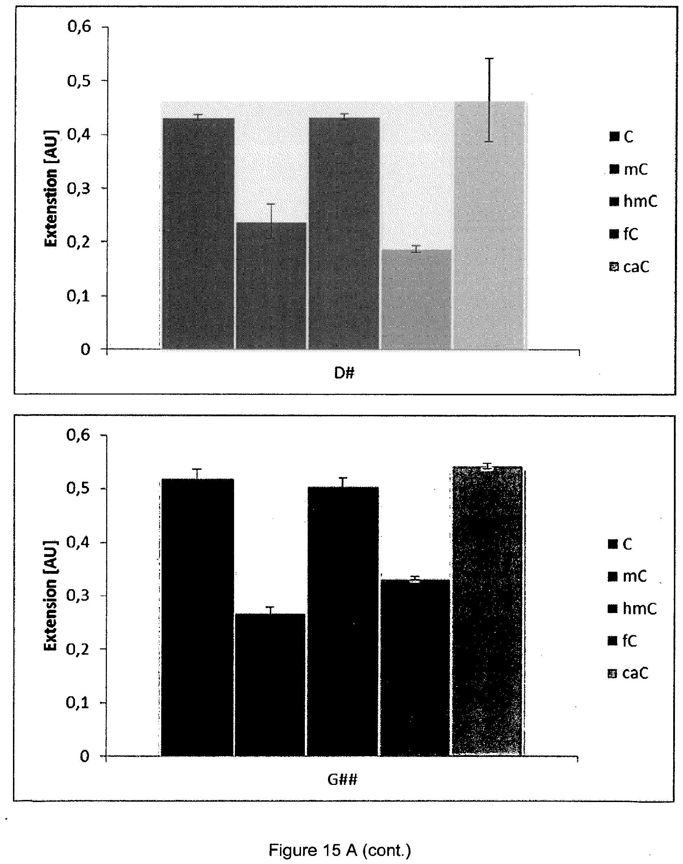

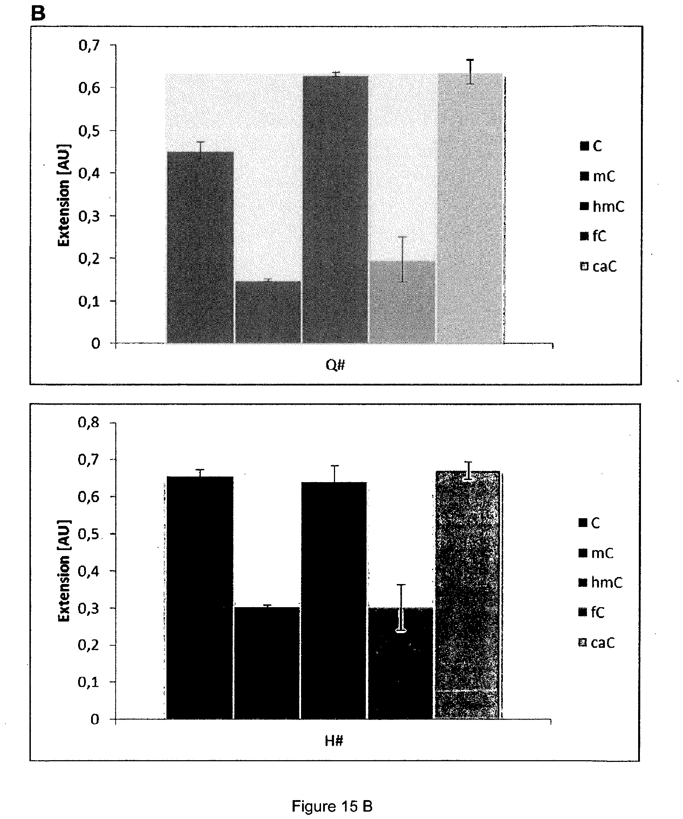

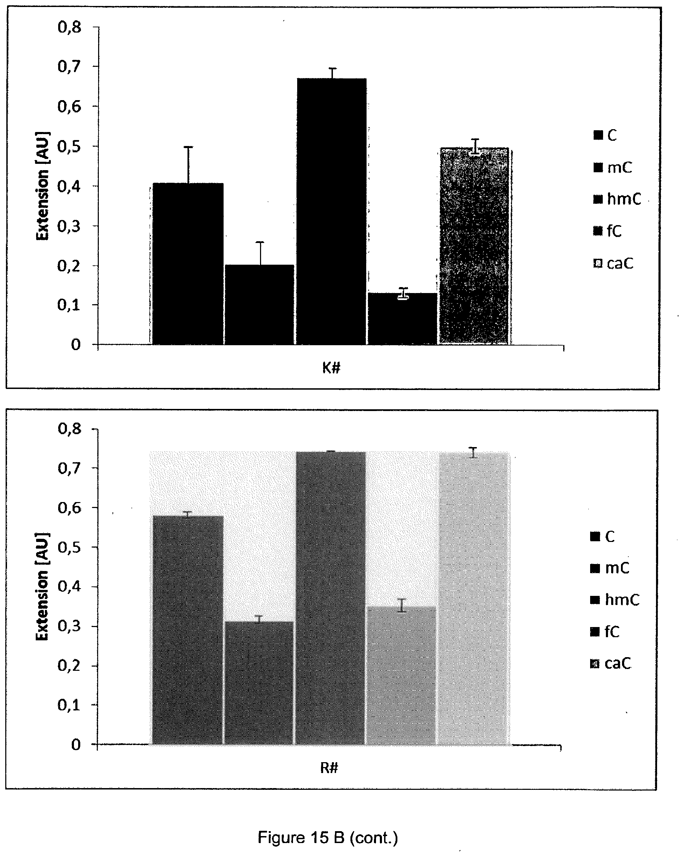

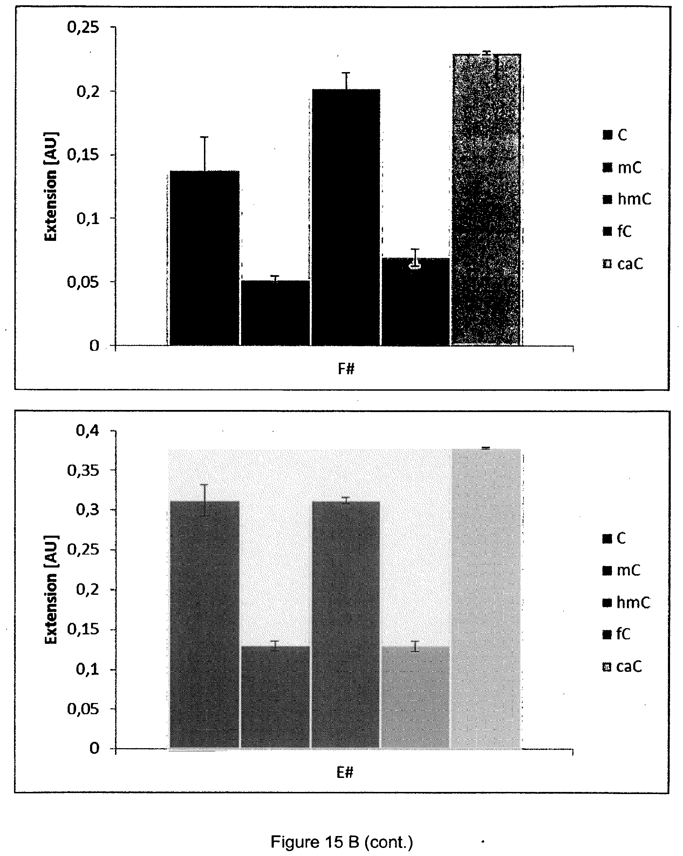

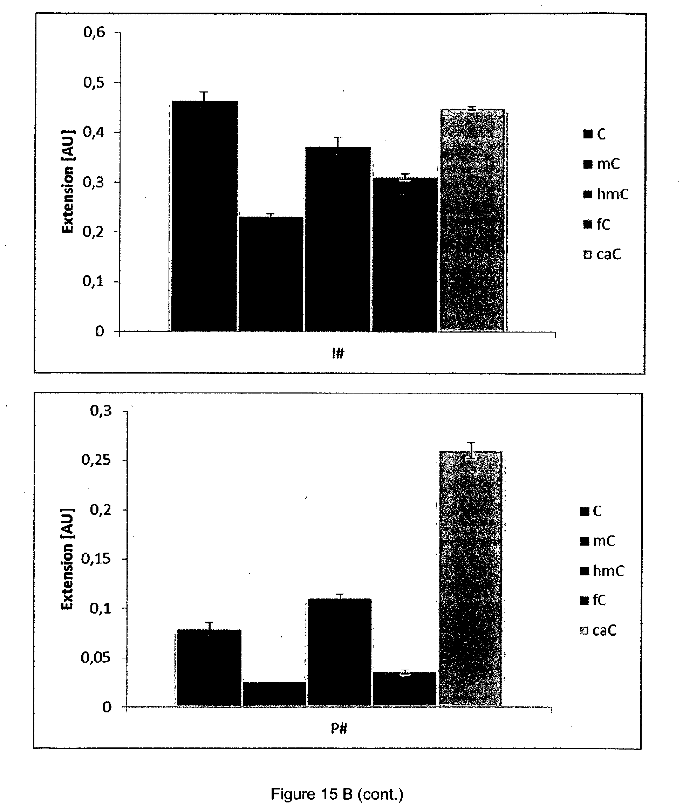

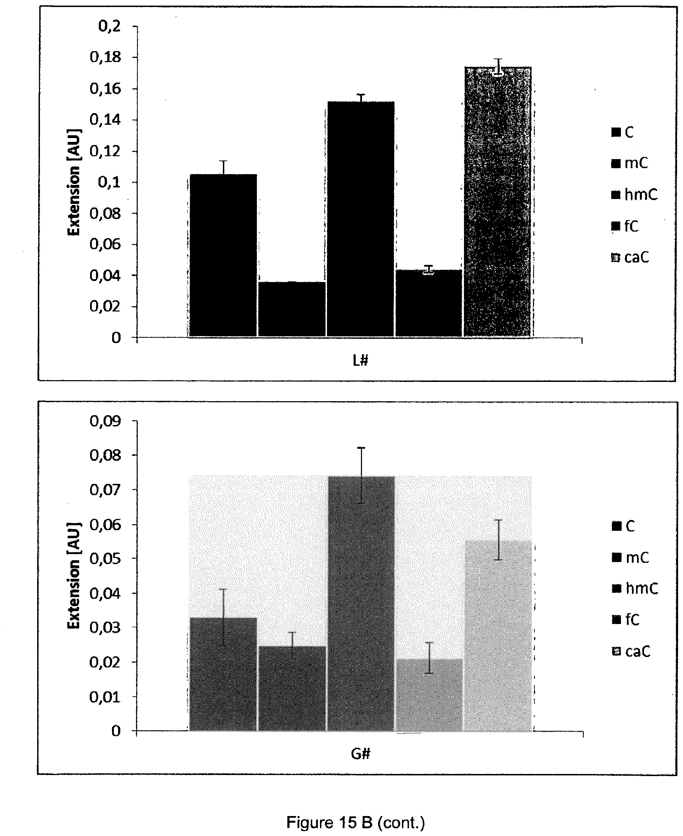

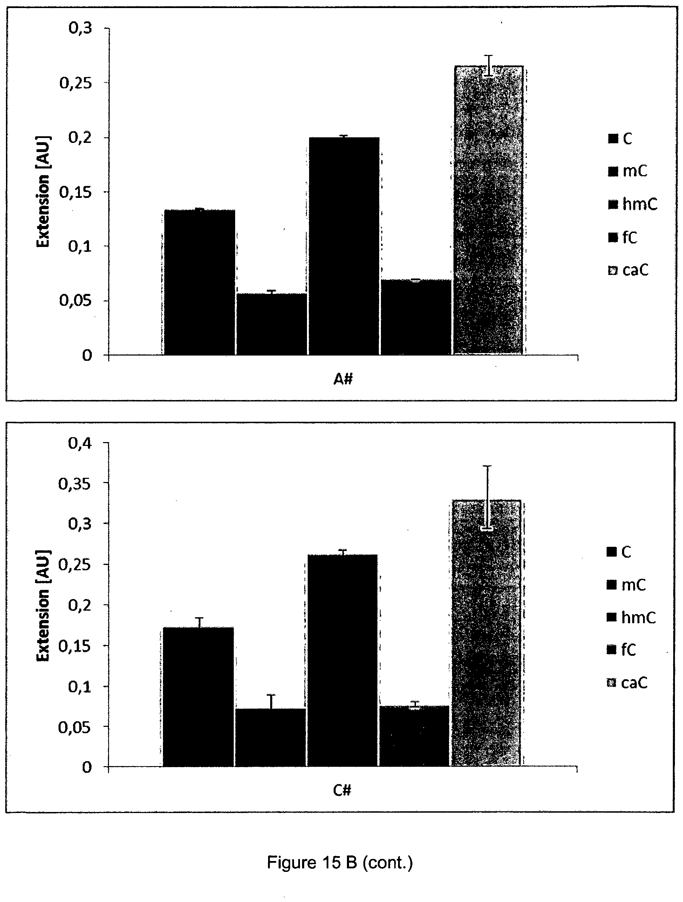

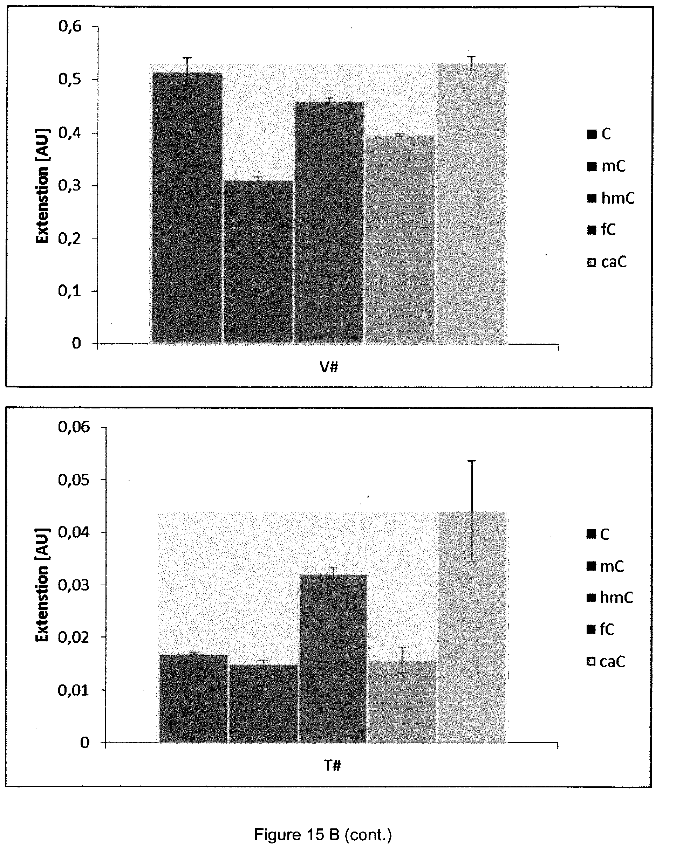

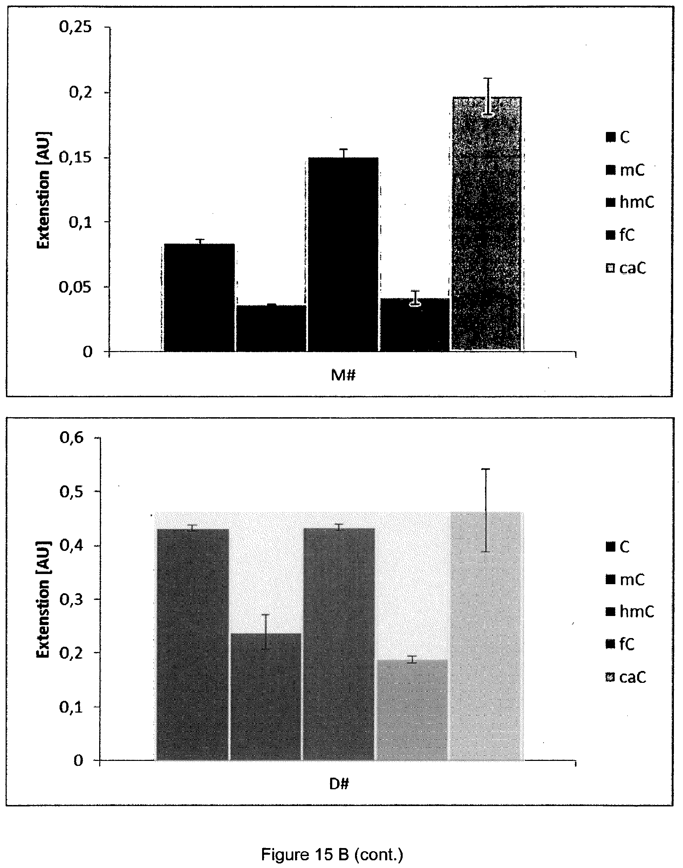

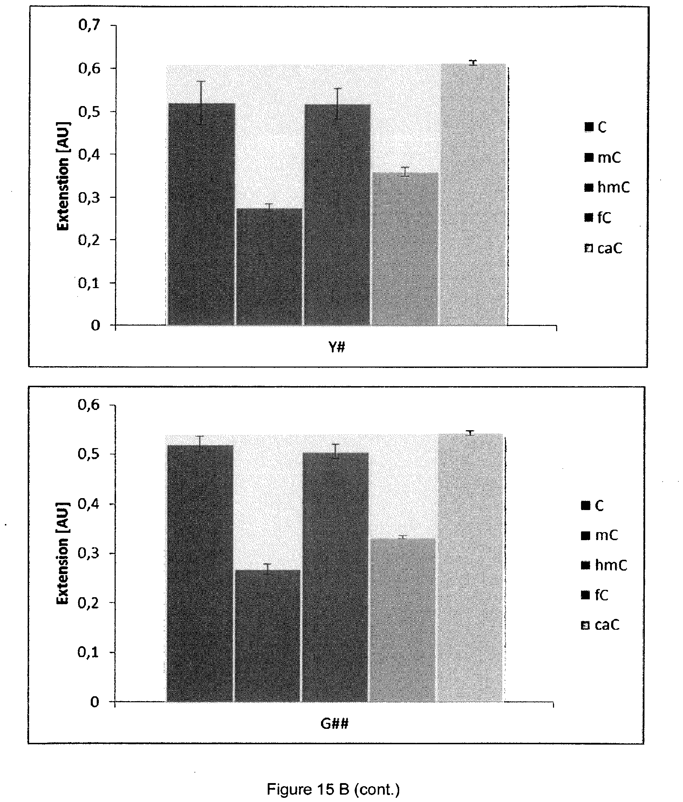

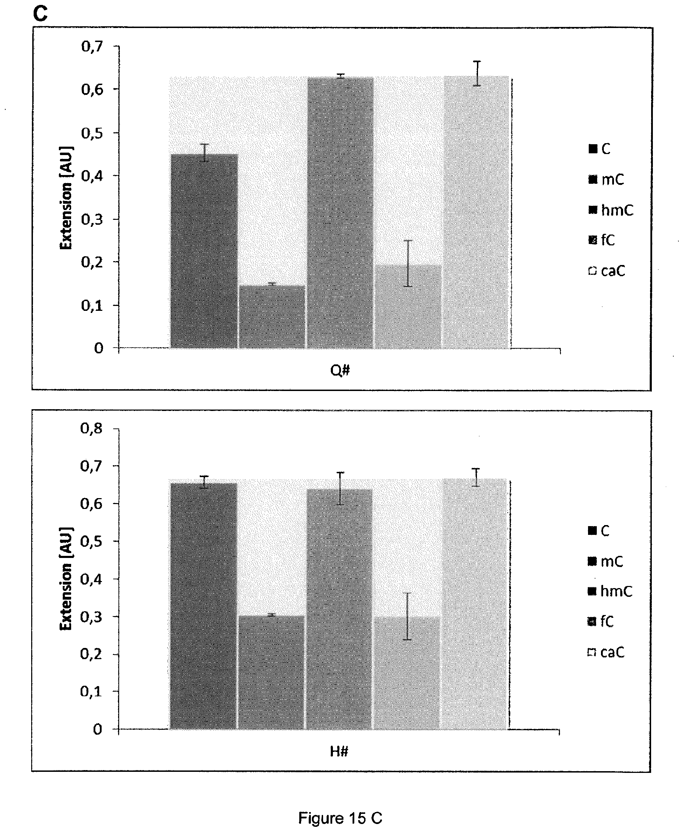

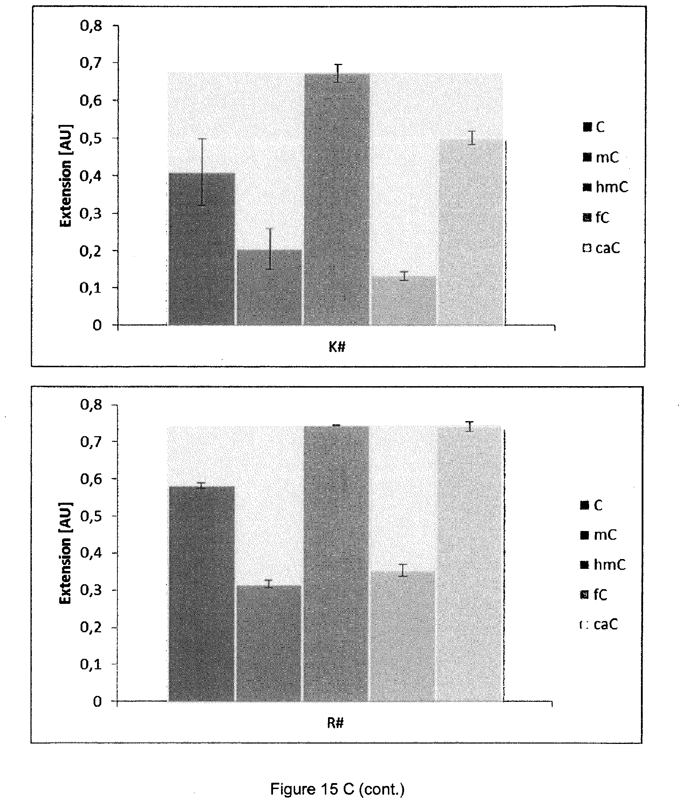

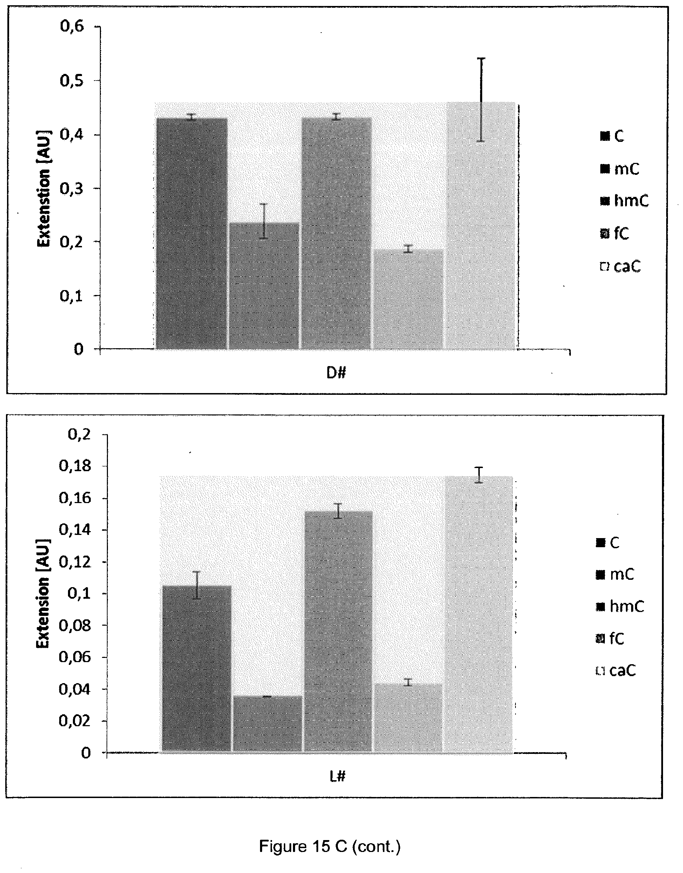

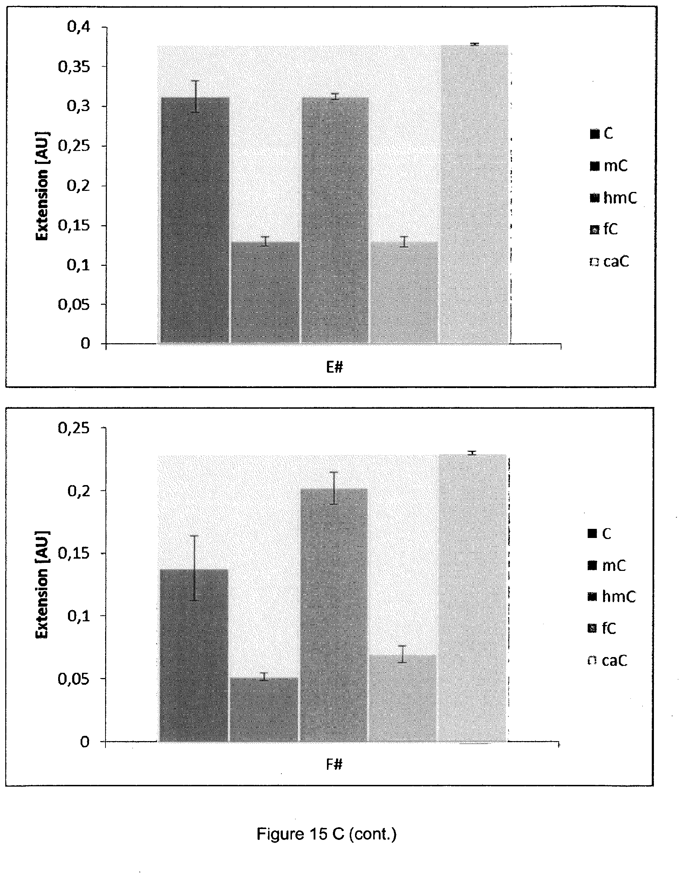

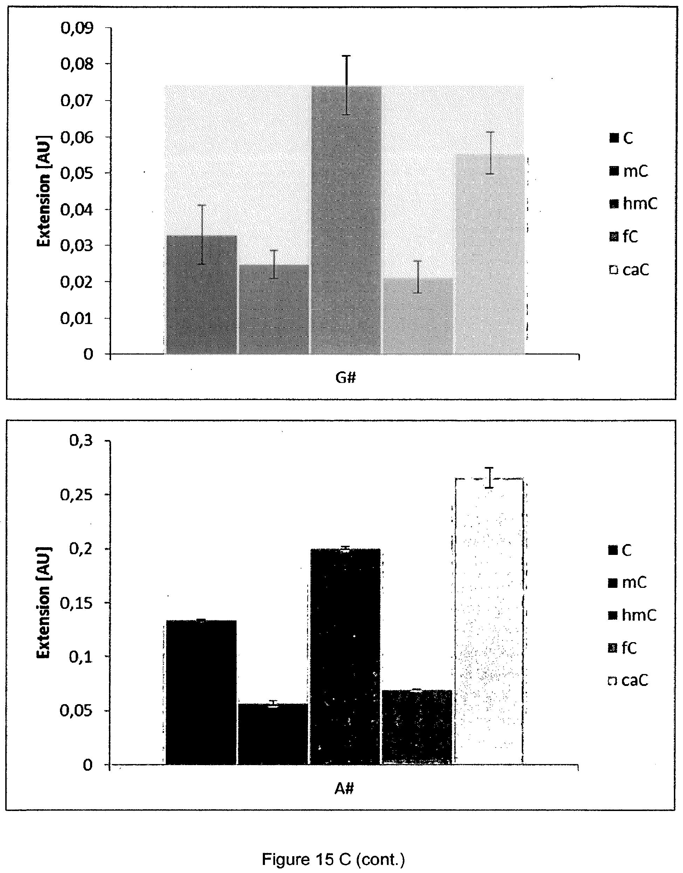

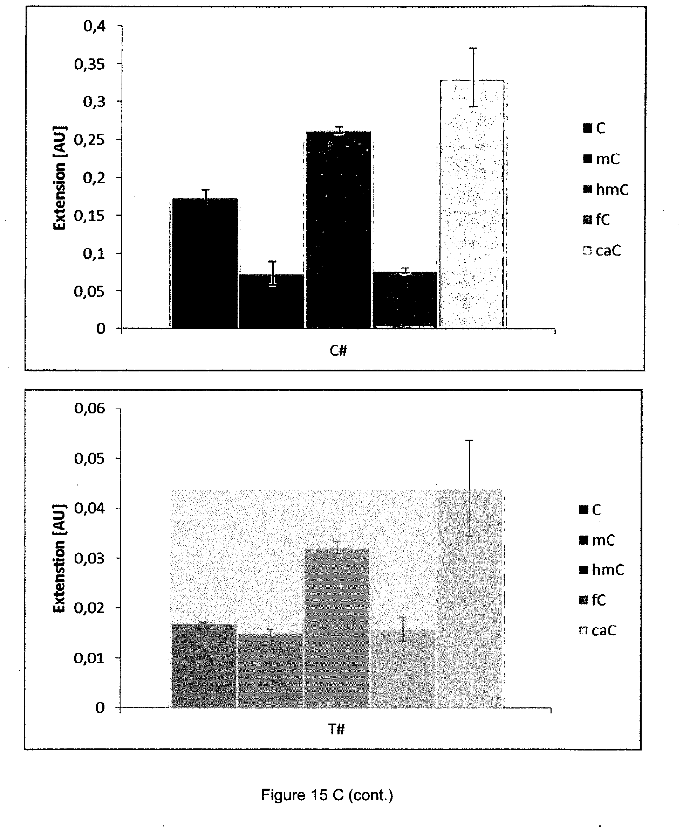

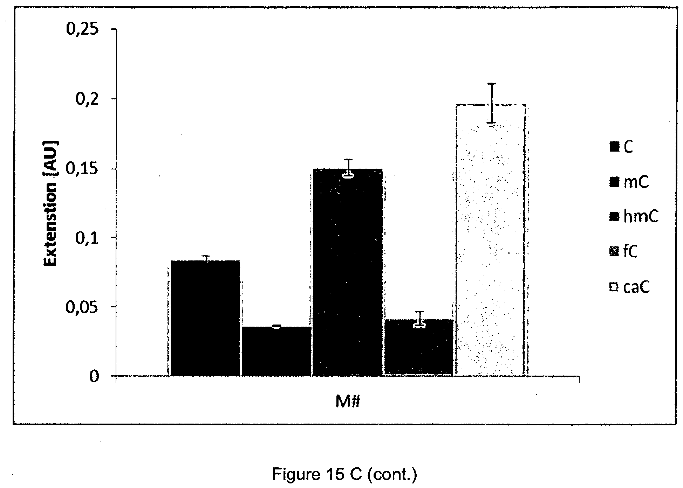

Primer extensions were performed as above with a TALE bearing a single TALE repeat with the RVD amino acids as specified in the diagrams (# = *) and a template bearing a single cytosine nucleobase opposite this RVD. Used TALE was targeting the sequence TGAGCTCTTCCGTTTCCACATCT with the variable C in bold (Fig. 15).

## Claims

1. A method for directly determining the presence or absence of a particular cytosine nucleobase of interest, selected from the group consisting of cytosine (C), 5-methylcytosine (mC), 5-hydroxymethylcytosine (hmC), 5-formylcytosine (fC), and 5-carboxylcytosine (caC), in a DNA molecule, comprising the steps of:
(a) providing a transcription-activator-like effector (TALE) protein, said TALE protein being capable of binding in a sequence-specific manner to a region of said DNA molecule that includes said cytosine nucleobase of interest;
(b) providing a first aliquot (aliquot 1) of said DNA molecule and subjecting said first aliquot to a modification reaction that selectively modifies the cytosine nucleobase of interest at the 5-position, wherein the presence of said chemical modification at the 5-position of the cytosine nucleobase of interest alters the binding affinity of said TALE protein to the DNA molecule; and
(c) subsequently determining the binding affinity of said TALE protein to said region of said DNA molecule in the first aliquot;
wherein
(i) the cytosine nucleobase of interest is present when the binding affinity of said TALE protein to said region of said DNA molecule in the first aliquot (first binding affinity) is different from the binding affinity of said TALE protein to said region of a DNA molecule that has not been subjected to said modification reaction (second binding affinity); and
(ii) the cytosine nucleobase of interest is not present when the first binding affinity is not different from the second binding affinity.

2. The method of claim 1, wherein the repeat variable diresidue (RVD) of said TALE protein whose position corresponds to the position of the nucleobase of interest is an RVD having one or more amino acid deletions.

3. The method of claim 1 or claim 2, wherein the alteration of the binding affinity of said TALE protein to the DNA molecule caused by the presence of the chemical modification in step (b) is an abolishment of binding or a significant weakening of the binding affinity.

4. The method of any one of claims 1 to 3, wherein:
(i) the cytosine nucleobase of interest is C;
the RVD of said TALE protein whose position corresponds to the position of the cytosine nucleobase of interest is selected from the group consisting of HD and ND; and
the modification reaction is the enzymatic conversion of C to a 5-modified C using DNA-methyltransferase (Dnmt) in the presence of S-adenosylmethionine (SAM) or a SAM analog;
(ii) the cytosine nucleobase of interest is mC;
the RVD of said TALE protein whose position corresponds to the position of the cytosine nucleobase of interest is selected from the group consisting of N*, S*, NG, HG, Q*, H*, K*, R*, L*, G*, E*, I*, F*, A*, C*, V*, Y*, D*, M*, P*, T*, and G**; and
the modification reaction is the enzymatic conversion of mC to caC via hmC and fC as intermediates, using ten-eleven-translocation (TET)-dioxygenase in the presence of α-ketoglutarate, Fe²⁺ and oxygen;
(iii) the cytosine nucleobase of interest is hmC;
the RVD of said TALE protein whose position corresponds to the position of the cytosine nucleobase of interest is selected from the group consisting of N*, G*, and 3*; and
the modification reaction is the enzymatic conversion of hmC to 5-glucosyl-hmC (ghmC) using T4 β-glucosyl-transferase (T4 BGT) in the presence of uridine diphosphate (UDP)-glucose or an analog thereof;
(iv) the cytosine nucleobase of interest is fC;
the RVD of said TALE protein whose position corresponds to the position of the cytosine nucleobase of interest is selected from the group consisting of NG, N*, G*, and 3*; and
the modification reaction is the conversion of fC to an oxime-modified cytosine using a hydroxylamine, to a hydrazone-modified cytosine using a hydrazine; to a substituted alkene using methylene components that react via aldol-addition/condensation-type reactions, or to an amine by reductive amination using an amine and a reductant;
(v) the cytosine nucleobase of interest is fC;
the RVD of said TALE protein whose position corresponds to the position of the cytosine nucleobase of interest is selected from the group consisting of NG, N*, S*, Q*, H*, K*, R*, L*, E*, F*, G*, A*, C*, D*, M*, T*; and
the modification reaction is the reduction of fC to hmC using a reductant; or
(vi) the cytosine nucleobase of interest is caC;
the RVD of said TALE protein whose position corresponds to the position of the cytosine nucleobase of interest is selected from the group consisting of N*, G*, and 3*; and
the modification reaction is the conversion of caC to an amide-modified cytosine using an amine, or to an ester-modified cytosine using an alcohol.

5. The method of any one of claims 1 to 4, wherein step (c) comprises the step of determining whether a specific DNA polymerase reaction using a primer that is capable of binding to said region of said DNA molecule that includes said nucleobase of interest is inhibited, thus indicating binding of said TALE protein to said region.

6. The method of claim 5, wherein step (c) comprises the steps of:
(c1) providing a primer that is capable of binding to the region of said DNA molecule that includes said nucleobase of interest;
(c2) providing a first aliquot of the first aliquot provided in step (b) (aliquot 1-1), and adding said TALE protein to said aliquot;
(c3) performing a specific DNA polymerase reaction with said primer using said aliquot of the DNA molecule (aliquot 1-1) as template;
(c4) determining at least one parameter, selected from the group consisting of the concentration, size, and sequence of the products of said specific DNA polymerase reaction; and
(c5) determining whether said TALE protein binds to said region,
wherein
(i) said TALE protein does not bind to said region or does bind to said region with a strongly reduced affinity when the parameters detected in step (c4) for the specific DNA polymerase reaction do not differ significantly from respective parameters for a specific DNA polymerase reaction performed with an aliquot of the DNA molecule as template to which no TALE protein has been added, and
(ii) said TALE protein binds to said region when the parameters detected in step (c4) for the specific DNA polymerase reaction differ significantly from respective parameters detected in step (c4) for a specific DNA polymerase reaction performed with an aliquot of the DNA molecule as template to which no TALE protein has been added.

7. The method of claim 6, wherein in step (c4)
(i) the concentration of the products of said specific DNA polymerase reaction is determined using a method, selected from the group consisting quantitative polymerase chain reaction (qPCR), fluorescence measurements using nucleic acid binding fluorescent probes, and photometry; and/or
(ii) the size of the products of said specific DNA polymerase reaction is determined using a method, selected from the group consisting of polymerase chain reaction (PCR), and gel electrophoresis; and/or
(iii) the sequence of the products of said specific DNA polymerase reaction is determined using a method, selected from the group consisting of Sanger sequencing, pyrosequencing, microarray hybridization, next-generation sequencing methods, and next-next-generation sequencing methods.

8. The method of claim 6 or 7, further comprising the steps of:
(d) comparing the parameter detected in step (c4) for the specific DNA polymerase reaction to one or more respective parameters obtained from DNA molecules having a known degree of presence of the cytosine nucleobase of interest; and
(e) thereby determining the degree of presence of the cytosine nucleobase of interest in the DNA molecule.

9. The method of any one of claims 6 to 8, wherein the DNA polymerase reaction in step (c3) is selected from the group consisting of primer extension reaction, arrayed primer extension (APEX), polymerase chain reaction (PCR), rolling circle amplification (RCA), and strand displacement amplification (SDA).

10. A method for the enrichment of DNA molecules in which a particular cytosine nucleobase of interest is present, said method comprising the steps of:
(a) providing a transcription-activator-like effector (TALE) protein, said TALE protein being capable of binding in a sequence-specific manner to a region of said DNA molecule that includes said nucleobase of interest, wherein said TALE protein is coupled directly or indirectly to an affinity ligand;
(b) subjecting the DNA molecule to a modification reaction that selectively modifies the cytosine nucleobase of interest at the 5-position, wherein the presence of said chemical modification at the 5-position of the cytosine nucleobase of interest abolishes or significantly weakens binding of said TALE protein to the DNA molecule;
(c) incubating the DNA molecules with said TALE protein; and
(d) depleting DNA molecules in which the cytosine nucleobase of interest is absent via affinity capture using said affinity ligand.

11. A method for the enrichment of DNA molecules in which a particular cytosine nucleobase of interest is absent, said method comprising the steps of:
(a) providing a transcription-activator-like effector (TALE) protein, said TALE protein being capable of binding in a sequence-specific manner to a region of said DNA molecule that includes said nucleobase of interest, wherein said TALE protein is coupled directly or indirectly to an affinity ligand;
(b) subjecting the DNA molecule to a modification reaction that selectively modifies the cytosine nucleobase of interest at the 5-position, wherein the presence of said chemical modification at the 5-position of the cytosine nucleobase of interest strengthens binding of said TALE protein to the DNA molecule;
(c) incubating the DNA molecules with said TALE protein; and
(d) depleting DNA molecules in which the cytosine nucleobase of interest is present via affinity capture using said affinity ligand.

12. A method for the differential digestion of DNA molecules in which a particular cytosine nucleobase of interest is absent, wherein DNA molecules in which the cytosine nucleobase of interest is present remain undigested, said method comprising the steps of:
(a) providing a transcription-activator-like effector (TALE) protein, said TALE protein being capable of binding in a sequence-specific manner to a region of said DNA molecule that includes said nucleobase of interest, wherein the TALE protein is coupled directly or indirectly to a protein having nuclease activity;
(b) subjecting the DNA molecule to a modification reaction that selectively modifies the cytosine nucleobase of interest at the 5-position, wherein the presence of said chemical modification at the 5-position of the cytosine nucleobase of interest abolishes or significantly weakens binding of said TALE protein to the DNA molecule;
(c) incubating the DNA molecules with said TALE protein; and
(d) digesting DNA molecules in which the cytosine nucleobase of interest is absent using said protein having nuclease activity.

13. A method for the differential digestion of DNA molecules in which a particular cytosine nucleobase of interest is present, wherein DNA molecules in which the cytosine nucleobase of interest is absent remain undigested, said method comprising the steps of:
(a) providing a transcription-activator-like effector (TALE) protein, said TALE protein being capable of binding in a sequence-specific manner to a region of said DNA molecule that includes said nucleobase of interest, wherein the TALE protein is coupled directly or indirectly to a protein having nuclease activity;
(b) subjecting the DNA molecule to a modification reaction that selectively modifies the cytosine nucleobase of interest at the 5-position, wherein the presence of said chemical modification at the 5-position of the cytosine nucleobase of interest strengthens binding of said TALE protein to the DNA molecule;
(c) incubating the DNA molecules with said TALE protein; and
(d) digesting DNA molecules in which the cytosine nucleobase of interest is present using said protein having nuclease activity.

14. A method for the differential digestion of DNA molecules in which a particular cytosine nucleobase of interest is absent, wherein DNA molecules in which the cytosine nucleobase of interest is present remain undigested, said method comprising the steps of:
(a) providing a transcription-activator-like effector (TALE) protein, said TALE protein being capable of binding in a sequence-specific manner to a region of said DNA molecule that includes said nucleobase of interest;
(b) subjecting the DNA molecule to a modification reaction that selectively modifies the cytosine nucleobase of interest at the 5-position, wherein the presence of said chemical modification at the 5-position of the cytosine nucleobase of interest strengthens binding of said TALE protein to the DNA molecule;
(c) incubating the DNA molecules with said TALE protein; and
(d) incubating the DNA molecules with a protein having nuclease activity;
wherein DNA molecules in which the cytosine nucleobase of interest is absent are digested using said protein having nuclease activity, and DNA molecules in which the cytosine nucleobase of interest is present are protected from digestion by the TALE protein bound to the DNA molecule.

15. A method for the differential digestion of DNA molecules in which a particular cytosine nucleobase of interest is present, wherein DNA molecules in which the cytosine nucleobase of interest is absent remain undigested, said method comprising the steps of:
(a) providing a transcription-activator-like effector (TALE) protein, said TALE protein being capable of binding in a sequence-specific manner to a region of said DNA molecule that includes said nucleobase of interest;
(b) subjecting the DNA molecule to a modification reaction that selectively modifies the cytosine nucleobase of interest at the 5-position, wherein the presence of said chemical modification at the 5-position of the cytosine nucleobase of interest abolishes or significantly weakens binding of said TALE protein to the DNA molecule;
(c) incubating the DNA molecules with said TALE protein; and
(d) incubating the DNA molecules with a protein having nuclease activity;
wherein DNA molecules in which the cytosine nucleobase of interest is present are digested using said protein having nuclease activity, and DNA molecules in which the cytosine nucleobase of interest is absent are protected from digestion by the TALE protein bound to the DNA molecule.

## Patentansprüche

1. Verfahren zur direkten Bestimmung der An- oder Abwesenheit einer bestimmten Zielcytosinnukleobase, ausgewählt aus der Gruppe, bestehend aus Cytosin (C), 5-Methylcytosin (mC), 5-Hydroxymethylcytosin (hmC), 5-Formylcytosin (fC) und 5-Carboxylcytosin (caC), in einem DNA-Molekül, umfassend die Schritte:
(a) Bereitstellen eines Transkriptionsaktivator-artiger Effektor (*transcription-activator-like effector,* TALE)-Proteins, wobei das TALE-Protein befähigt ist, in sequenzspezifischer Weise an einen Bereich des DNA-Moleküls zu binden, der die Zielcytosinnukleobase enthält,
(b) Bereitstellen eines ersten Aliquots (Aliquot 1) des DNA-Moleküls und Unterziehen des ersten Aliquots einer Modifikationsreaktion, die selektiv die Zielcytosinnukleobase an der 5-Position modifziert, wobei die Anwesenheit der chemischen Modifikation an der 5-Position der Zielcytosinnukleobase die Bindungsaffinität des TALE-Proteins an das DNA-Molekül verändert und
(c) nachfolgend Bestimmen der Bindungsaffinität des TALE-Proteins an den Bereich des DNA-Moleküls in dem ersten Aliquot,
wobei
(i) die Zielcytosinnukleobase vorhanden ist, wenn die Bindungsaffinität des TALE-Proteins an den Bereich des DNA-Moleküls in dem ersten Aliquot (erste Bindungsaffinität) von der Bindungsaffinität des TALE-Proteins an den Bereich eines DNA-Moleküls, das nicht der Modifikationsreaktion unterzogen wurde (zweite Bindungsaffinität), verschieden ist und
(ii) die Zielcytosinnukleobase nicht vorhanden ist, wenn die erste Bindungsaffinität nicht von der zweiten Bindungsaffinität verschieden ist.

2. Verfahren nach Anspruch 1, wobei der sich wiederholende, variable Doppelrest (*repeat variable diresidue;* RVD) des TALE-Proteins, dessen Position der Position der Zielnukleobase entspricht, ein RVD ist, der eine oder mehrere Aminosäuredeletion(en) aufweist.

3. Verfahren nach Anspruch 1 oder Anspruch 2, wobei die Änderung der Bindungsaffinität des TALE-Proteins an das DNA-Molekül, die durch die Anwesenheit der chemischen Modifikation in Schritt (b) verursacht wird, eine Aufhebung der Bindung oder eine signifikante Schwächung der Bindungsaffinität ist.

4. Verfahren nach einem der Ansprüche 1 bis 3, wobei:
(i) die Zielcytosinnukleobase C ist,
der RVD des TALE-Proteins, dessen Position der Position der Zielcytosinnukleobase entspricht, aus der Gruppe, bestehend aus HD und ND, ausgewählt ist und
die Modifikationsreaktion die enzymatische Umwandlung von C zu einem 5-modifizierten C, unter Verwendung von DNA-Methyltransferase (Dnmt) in der Anwesenheit von S-Adenosylmethionin (SAM) oder einem SAM-Analogon, ist,
(ii) die Zielcytosinnukleobase mC ist,
der RVD des TALE-Proteins, dessen Position der Position der Zielcytosinnukleobase entspricht, aus der Gruppe, bestehend aus N*, S*, NG, HG, Q*, H*, K*, R*, L*, G*, E*, I*, F*, A*, C*, V*, Y*, D*, M*, P*, T* und G**, ausgewählt ist und
die Modifikationsreaktion die enzymatische Umwandlung von mC zu caC über hmC und fC als Intermediate, unter Verwendung von *ten-eleven translocation* (TET)-Dioxygenase in der Anwesenheit von α-Ketoglutarat, Fe²⁺ und Sauerstoff, ist,
(iii) die Zielcytosinnukleobase hmC ist,
der RVD des TALE-Proteins, dessen Position der Position der Zielcytosinnukleobase entspricht, aus der Gruppe, bestehend aus N*, G* und 3*, ausgewählt ist und
die Modifikationsreaktion die enzymatische Umwandlung von hmC zu 5-Glycosyl-hmC (ghmC), unter Verwendung von T4 β-Glucosyltransferase (T4 BGT) in der Anwesenheit von Uridindiphosphat (UDP)-Glucose oder einem Analogon davon, ist,
(iv) die Zielcytosinnukleobase fC ist,
der RVD des TALE-Proteins, dessen Position der Position der Zielcytosinnukleobase entspricht, aus der Gruppe, bestehend aus NG, N*, G* und 3*, ausgewählt ist und
die Modifikationsreaktion die Umwandlung von fC zu einem Oximmodifzierten Cytosin unter Verwendung eines Hydroxylamins, zu einem Hydrazon-modifizierten Cytosin unter Verwendung eines Hydrazins, zu einem substituierten Alken unter Verwendung von Methylenbestandteilen, die über eine Reaktion vom Aldoladditions-/Kondensationstyp reagieren, oder zu einem Amin durch reduktive Aminierung unter Verwendung eines Amins und eines Reduktionsmittels, ist,
(v) die Zielcytosinnukleobase fC ist,
der RVD des TALE-Proteins, dessen Position der Position der Zielcytosinnukleobase entspricht, aus der Gruppe, bestehend aus NG, N*, S*, Q*, H*, K*, R*, L*, E*, F*, G*, A*, C*, D*, M*, T*, ausgewählt ist und
die Modifikationsreaktion die Reduktion von fC zu hmC, unter Verwendung eines Reduktionsmittels, ist oder
(vi) die Zielcytosinnukleobase caC ist,
der RVD des TALE-Proteins, dessen Position der Position der Zielcytosinnukleobase entspricht, aus der Gruppe, bestehend aus N*, G* und 3*, ausgewählt ist und
die Modifikationsreaktion die Umwandlung von caC zu einem Amidmodifizierten Cytosin unter Verwendung eines Amins oder zu einem Ester-modifzierten Cytosin unter Verwendung eines Alkohols ist.

5. Verfahren nach einem der Ansprüche 1 bis 4, wobei Schritt (c) den Schritt des Bestimmens umfasst, ob eine spezifische DNA-Polymerasereaktion, unter Verwendung eines Primers, der befähigt ist, an den Bereich des DNA-Moleküls zu binden, der die Zielnukleobase enthält, inhibiert ist, wodurch die Bindung des TALE-Proteins an den Bereich angezeigt wird.

6. Verfahren nach Anspruch 5, wobei Schritt (c) die Schritte umfaßt:
(c1) Bereitstellen eines Primers, der befähigt ist, an den Bereich des DNA-Moleküls zu binden, der die Zielnukleobase enthält,
(c2) Bereitstellen eines ersten Aliquots des in Schritt (b) bereitgestellten ersten Aliquots (Aliquot 1-1) und Zugeben des TALE-Proteins zu dem Aliquot,
(c3) Durchführen einer spezifischen DNA-Polymerasereaktion mit dem Primer unter Verwendung des Aliquots des DNA-Moleküls (Aliquot 1-1) als Matrize,
(c4) Bestimmen mindestens eines Parameters, ausgewählt aus der Gruppe, bestehend aus der Konzentration, Größe und Sequenz der Produkte der spezifischen DNA-Polymerasereaktion und
(c5) Bestimmen, ob das TALE-Protein an den Bereich bindet,
wobei
(i) das TALE-Protein nicht an den Bereich bindet oder mit einer stark reduzierten Affinität an den Bereich bindet, wenn die in Schritt (c4) nachgewiesenen Parameter für die spezifische DNA-Polymerasereaktion nicht signifikant von entsprechenden Parametern für eine spezifische DNA-Polymerasereaktion, die mit einem Aliquot des DNA-Moleküls als Matrize durchgeführt wurde, zu dem kein TALE-Protein zugefügt wurde, verschieden sind und
(ii) das TALE-Protein an den Bereich bindet, wenn die in Schritt (c4) nachgewiesenen Parameter für die spezifische DNA-Polymerasereaktion signifikant von entsprechenden Parametern, die in Schritt (c4) für eine spezifische DNA-Polymerasereaktion nachgewiesen wurden, die mit einem Aliquot des DNA-Moleküls als Matrize durchgeführt wurde, zu dem kein TALE-Protein zugegeben wurde, verschieden sind.

7. Verfahren nach Anspruch 6, wobei in Schritt (c4)
(i) die Konzentration der Produkte der spezifischen DNA-Polymerasereaktion unter Verwendung eines Verfahrens, ausgewählt aus der Gruppe, bestehend aus quantitativer Polymerasekettenreaktion (qPCR), Fluoreszenzmessungen unter Verwendung von Nukleinsäure-bindenden fluoreszierenden Sonden und Photometrie, bestimmt wird und/oder
(ii) die Größe der Produkte der spezifischen DNA-Polymerasereaktion unter Verwendung eines Verfahrens, ausgewählt aus der Gruppe, bestehend aus Polymerasekettenreaktion (PCR) und Gelelektrophorese, bestimmt wird und/oder
(iii) die Sequenz der Produkte der spezifischen DNA-Polymerasereaktion unter Verwendung eines Verfahrens, ausgewählt aus der Gruppe, bestehend aus Sanger-Sequenzierung, Pyrosequenzierung, Microarray-Hybridisierung, Next-Generation Sequenzierverfahren und Next-Next-Generation Sequenzierverfahren, bestimmt wird.

8. Verfahren nach Anspruch 6 oder 7, weiter umfassend die Schritte:
(d) Vergleichen des in Schritt (c4) nachgewiesen Parameters für die spezifische DNA-Polymerasereaktion mit einem oder mehreren entsprechenden Parameter(n), die von DNA-Molekülen erhalten wurden, die einen bekannten Grad der Anwesenheit der Zielcytosinnukleobase aufweisen und
(e) dadurch Bestimmen des Grads der Anwesenheit der Zielcytosinnukleobase in dem DNA-Molekül.

9. Verfahren nach einem der Ansprüche 6 bis 8, wobei die DNA-Polymerasereaktion in Schritt (c3) aus der Gruppe, bestehend aus Primerverlängerungsreaktion, *Arrayed Primer Extension* (APEX), Polymerasekettenreaktion (PCR), *Rolling Circle*-Amplifikation (RCA) und *Strand Displacement-*Amplifikation (SDA), ausgewählt ist.

10. Verfahren für das Anreichern von DNA-Molekülen, in denen eine bestimmte Zielcytosinnukleobase vorhanden ist, wobei das Verfahren die Schritte umfaßt:
(a) Bereitstellen eines Transkriptionsaktivator-artiger Effektor (*transcription-activator-like effector;* TALE)-Proteins, wobei das TALE-Protein befähigt ist, in sequenzspezifischer Weise an einen Bereich des DNA-Moleküls zu binden, der die Zielcytosinnukleobase enthält, wobei das TALE-Protein direkt oder indirekt an einen Affinitätsliganden gekoppelt ist,
(b) Unterziehen des DNA-Moleküls einer Modifikationsreaktion, die selektiv die Zielcytosinnukleobase an der 5-Position modifziert, wobei die Anwesenheit der chemischen Modifikation an der 5-Position der Zielcytosinnukleobase die Bindung des TALE-Proteins an das DNA-Molekül aufhebt oder signifikant schwächt,
(c) Inkubieren des DNA-Moleküls mit dem TALE-Protein und
(d) Depletieren von DNA-Molekülen, in denen die Zielcytosinnukleobase abwesend ist, mittels Affinitätseinfang unter Verwendung des Affinitätsliganden.

11. Verfahren für das Anreichern von DNA-Molekülen, in denen eine bestimmte Zielcytosinnukleobase abwesend ist, wobei das Verfahren die Schritte umfaßt:
(a) Bereitstellen eines Transkriptionsaktivator-artiger Effektor (*transcription-activator-like effector,* TALE)-Proteins, wobei das TALE-Protein befähigt ist, in sequenzspezifischer Weise an einen Bereich des DNA-Moleküls zu binden, der die Zielcytosinnukleobase enthält, wobei das TALE-Protein direkt oder indirekt an einen Affinitätsliganden gekoppelt ist,
(b) Unterziehen des DNA-Moleküls einer Modifikationsreaktion, die selektiv die Zielcytosinnukleobase an der 5-Position modifziert, wobei die Anwesenheit der chemischen Modifikation an der 5-Position der Zielcytosinnukleobase die Bindung des TALE-Proteins an das DNA-Molekül stärkt,
(c) Inkubieren des DNA-Moleküls mit dem TALE-Protein und
(d) Depletieren von DNA-Molekülen, in denen die Zielcytosinnukleobase vorhanden ist, mittels Affinitätseinfang unter Verwendung des Affinitätsliganden.

12. Verfahren für den differenziellen Verdau von DNA-Molekülen, in denen eine bestimmte Zielcytosinnukleobase abwesend ist, wobei DNA-Moleküle, in denen die Zielcytosinnukleobase vorhanden ist, unverdaut bleiben, wobei das Verfahren die Schritte umfaßt:
(a) Bereitstellen eines Transkriptionsaktivator-artiger Effektor (*transcription-activator-like effector,* TALE)-Proteins, wobei das TALE-Protein befähigt ist, in sequenzspezifischer Weise an einen Bereich des DNA-Moleküls zu binden, der die Zielcytosinnukleobase enthält, wobei das TALE-Protein direkt oder indirekt an ein Protein gekoppelt ist, das Nukleaseaktivität aufweist,
(b) Unterziehen des DNA-Moleküls einer Modifikationsreaktion, die selektiv die Zielcytosinnukleobase an der 5-Position modifziert, wobei die Anwesenheit der chemischen Modifikation an der 5-Position der Zielcytosinnukleobase die Bindung des TALE-Proteins an das DNA-Molekül aufhebt oder signifikant schwächt,
(c) Inkubieren des DNA-Moleküls mit dem TALE-Protein und
(d) Verdauen von DNA-Molekülen, in denen die Zielcytosinnukleobase abwesend ist, unter Verwendung des Proteins, das Nukleaseaktivität aufweist.

13. Verfahren für den differenziellen Verdau von DNA-Molekülen, in denen eine bestimmte Zielcytosinnukleobase vorhanden ist, wobei DNA-Moleküle, in denen die Zielcytosinnukleobase abwesend ist, unverdaut bleiben, wobei das Verfahren die Schritte umfaßt:
(a) Bereitstellen eines Transkriptionsaktivator-artiger Effektor (*transcription-activator-like effector,* TALE)-Proteins, wobei das TALE-Protein befähigt ist, in sequenzspezifischer Weise an einen Bereich des DNA-Moleküls zu binden, der die Zielcytosinnukleobase enthält, wobei das TALE-Protein direkt oder indirekt an ein Protein gekoppelt ist, das Nukleaseaktivität aufweist,
(b) Unterziehen des DNA-Moleküls einer Modifikationsreaktion, die selektiv die Zielcytosinnukleobase an der 5-Position modifziert, wobei die Anwesenheit der chemischen Modifikation an der 5-Position der Zielcytosinnukleobase die Bindung des TALE-Proteins an das DNA-Molekül stärkt,
(c) Inkubieren des DNA-Moleküls mit dem TALE-Protein und
(d) Verdauen von DNA-Molekülen, in denen die Zielcytosinnukleobase vorhanden ist, unter Verwendung des Proteins, das Nukleaseaktivität aufweist.

14. Verfahren für den differenziellen Verdau von DNA-Molekülen, in denen eine bestimmte Zielcytosinnukleobase abwesend ist, wobei DNA-Moleküle, in denen die Zielcytosinnukleobase vorhanden ist, unverdaut bleiben, wobei das Verfahren die Schritte umfaßt:
(a) Bereitstellen eines Transkriptionsaktivator-artiger Effektor (*transcription-activator-like effector,* TALE)-Proteins, wobei das TALE-Protein befähigt ist, in sequenzspezifischer Weise an einen Bereich des DNA-Moleküls zu binden, der die Zielcytosinnukleobase enthält,
(b) Unterziehen des DNA-Moleküls einer Modifikationsreaktion, die selektiv die Zielcytosinnukleobase an der 5-Position modifziert, wobei die Anwesenheit der chemischen Modifikation an der 5-Position der Zielcytosinnukleobase die Bindung des TALE-Proteins an das DNA-Molekül stärkt,
(c) Inkubieren des DNA-Moleküls mit dem TALE-Protein und
(d) Inkubieren der DNA-Moleküle mit einem Protein, das Nukleaseaktivität aufweist,
wobei DNA-Moleküle, in denen die Zielcytosinnukleobase abwesend ist, unter Verwendung des Proteins, das Nukleaseaktivität aufweist, verdaut werden und DNA-Moleküle, in denen die Zielcytosinnukleobase vorhanden ist, vor dem Verdau durch das an das DNA-Molekül gebundene TALE-Protein geschützt sind.

15. Verfahren für den differenziellen Verdau von DNA-Molekülen, in denen eine bestimmte Zielcytosinnukleobase vorhanden ist, wobei DNA-Moleküle, in denen die Zielcytosinnukleobase abwesend ist, unverdaut bleiben, wobei das Verfahren die Schritte umfaßt:
(a) Bereitstellen eines Transkriptionsaktivator-artiger Effektor (*transcription-activator-like effector;* TALE)-Proteins, wobei das TALE-Protein befähigt ist, in sequenzspezifischer Weise an einen Bereich des DNA-Moleküls zu binden, der die Zielcytosinnukleobase enthält,
(b) Unterziehen des DNA-Moleküls einer Modifikationsreaktion, die selektiv die Zielcytosinnukleobase an der 5-Position modifziert, wobei die Anwesenheit der chemischen Modifikation an der 5-Position der Zielcytosinnukleobase die Bindung des TALE-Proteins an das DNA-Molekül aufhebt oder signifikant schwächt,
(c) Inkubieren des DNA-Moleküls mit dem TALE-Protein und
(d) Inkubieren der DNA-Moleküle mit einem Protein, das Nukleaseaktivität aufweist,
wobei DNA-Moleküle, in denen die Zielcytosinnukleobase vorhanden ist, unter Verwendung des Proteins, das Nukleaseaktivität aufweist, verdaut werden und DNA-Moleküle, in denen die Zielcytosinnukleobase abwesend ist, vor dem Verdau durch das an das DNA-Molekül gebundene TALE-Protein geschützt sind.

## Revendications

1. Procédé pour déterminer directement la présence ou l'absence d'une nucléobase de cytosine d'intérêt, choisie dans le groupe constitué de la cytosine (C), la 5-méthylcytosine (mC), la 5-hydroxyméthylcytosine (hmC), la 5-formylcytosine (fC), et la 5-carboxylcytosine (caC), dans une molécule d'ADN, comprenant les étapes de :
(a) la fourniture d'une protéine effecteur de type activateur de la transcription (TALE), ladite protéine TALE étant capable de se lier d'une façon spécifique de la séquence à une région de ladite molécule d'ADN qui comprend ladite nucléobase de cytosine d'intérêt ;
(b) la fourniture d'une première aliquote (aliquote 1) de ladite molécule d'ADN et la soumission de ladite première aliquote à une réaction de modification qui modifie sélectivement la nucléobase de cytosine intérêt au niveau de la position 5, dans lequel la présence de ladite modification chimique au niveau de la position 5 de la nucléobase de cytosine d'intérêt modifie l'affinité de liaison de ladite protéine TALE à la molécule d'ADN ; et
(c) la détermination subséquente de l'affinité de liaison de ladite protéine TALE à ladite région de ladite molécule d'ADN dans la première aliquote ;
dans lequel
(i) la nucléobase de cytosine d'intérêt est présente lorsque l'affinité de liaison de ladite protéine TALE à ladite région de ladite molécule d'ADN dans la première aliquote (première affinité de liaison) est différente de l'affinité de liaison de ladite protéine TALE à ladite région d'une molécule d'ADN qui n'a pas été soumise à ladite réaction de modification (seconde affinité de liaison) ; et
(ii) la nucléobase de cytosine d'intérêt n'est pas présente lorsque la première affinité de liaison n'est pas différente de la seconde affinité de liaison.

2. Procédé selon la revendication 1, dans lequel le di-résidu variable répétitif (RVD) de ladite protéine TALE dont la position correspond à la position de la nucléobase d'intérêt est un RVD comportant une ou plusieurs délétions d'acides aminés.

3. Procédé selon la revendication 1 ou la revendication 2, dans lequel la modification de l'affinité de liaison de ladite protéine TALE à la molécule d'ADN provoquée par la présence de la modification chimique dans l'étape (b) est un abolissement de la liaison ou un affaiblissement significatif de l'affinité de liaison.

4. Procédé selon l'une quelconque des revendications 1 à 3, dans lequel :
(i) la nucléobase de cytosine d'intérêt est C ;
le RVD de ladite protéine TALE dont la position correspond à la position de la nucléobase de cytosine d'intérêt est choisi dans le groupe constitué de HD et ND ; et
la réaction de modification est la conversion enzymatique de C en une C modifiée en 5 en utilisant l'ADN-méthyltransférase (Dnmt) en présence de S-adénosylméthionine (SAM) ou un analogue de SAM ;
(ii) la nucléobase de cytosine d'intérêt est mC ;
le RVD de ladite protéine TALE dont la position correspond à la position de la nucléobase de cytosine d'intérêt est choisi dans le groupe constitué de N*, S*, NG, HG, Q*, H*, K*, R*, L*, G*, E*, I*, F*, A*, C*, V*, Y*, D*, M*, P*, T*, et G** ; et
la réaction de modification est la conversion enzymatique de mC en caC en passant par les hmC et fC en tant qu'intermédiaires, en utilisant la TET (ten-eleven-translocation)-dioxygénase en présence d'α-cétoglutarate, de Fe²⁺ et d'oxygène ;
(iii) la nucléobase de cytosine d'intérêt est hmC ;
le RVD de ladite protéine TALE dont la position correspond à la position de la nucléobase de cytosine d'intérêt est choisi dans le groupe constitué de N*, G*, et 3* ; et
la réaction de modification est la conversion enzymatique de hmC en 5-glucosyl-hmC (ghmC) en utilisant la T4 β-glucosyl-transférase (T4 BGT) en présence d'uridine diphosphate (UDP)-glucose ou de l'un de ses analogues ;
(iv) la nucléobase de cytosine d'intérêt est fC ;
le RVD de ladite protéine TALE dont la position correspond à la position de la nucléobase de cytosine d'intérêt est choisi dans le groupe constitué de NG, N*, G*, et 3* ; et
la réaction de modification est la conversion de fC en une cytosine modifiée par une oxime en utilisant une hydroxylamine, en une cytosine modifiée par une hydrazone en utilisant une hydrazine ; en un alcène substitué en utilisant des composants de méthylène qui réagissent en passant par des réactions de type addition aldolique/condensation, ou en une amine par amination réductrice en utilisant une amine et un réducteur ;
(v) la nucléobase de cytosine d'intérêt est fC ;
le RVD de ladite protéine TALE dont la position correspond à la position de la nucléobase de cytosine d'intérêt est choisi dans le groupe constitué de NG, N*, S*, Q*, H*, K*, R*, L*, E*, F*, G*, A*, C*, D*, M*, T* ; et
la réaction de modification est la réduction de fC en hmC en utilisant un réducteur ; ou
(vi) la nucléobase de cytosine d'intérêt est caC ;
le RVD de ladite protéine TALE dont la position correspond à la position de la nucléobase de cytosine d'intérêt est choisi dans le groupe constitué de N*, G*, et 3* ; et
la réaction de modification est la conversion de caC en une cytosine modifiée par un amide en utilisant une amine, ou en une cytosine modifiée par un ester en utilisant un alcool.

5. Procédé selon l'une quelconque des revendications 1 à 4, dans lequel l'étape (c) comprend l'étape de détermination si une réaction d'ADN polymérase spécifique en utilisant une amorce qui est capable de se lier à ladite région de ladite molécule d'ADN qui comprend ladite nucléobase d'intérêt est inhibée, indiquant ainsi la liaison de ladite protéine TALE à ladite région.

6. Procédé selon la revendication 5, dans lequel l'étape (c) comprend les étapes de :
(c1) la fourniture d'une amorce qui est capable de se lier à la région de ladite molécule d'ADN qui comprend ladite nucléobase d'intérêt ;
(c2) la fourniture d'une première aliquote de la première aliquote fournie dans l'étape (b) (aliquote 1-1), et l'ajout de ladite protéine TALE à ladite aliquote ;
(c3) la réalisation d'une réaction d'ADN polymérase spécifique avec ladite amorce en utilisant ladite aliquote de la molécule d'ADN (aliquote 1-1) comme matrice ;
(c4) la détermination d'au moins un paramètre, choisi dans le groupe constitué de la concentration, la taille, et la séquence des produits de ladite réaction d'ADN polymérase spécifique ; et
(c5) la détermination si ladite protéine TALE se lie à ladite région,
dans lequel
(i) ladite protéine TALE ne se lie pas à ladite région ou se lie à ladite région avec une affinité fortement réduite lorsque les paramètres détectés dans l'étape (c4) pour la réaction d'ADN polymérase spécifique ne diffèrent pas significativement des paramètres respectifs pour une réaction d'ADN polymérase spécifique effectuée avec une aliquote de la molécule d'ADN comme matrice à laquelle aucune protéine TALE n'a été ajoutée, et
(ii) ladite protéine TALE se lie à ladite région lorsque les paramètres détectés dans l'étape (c4) pour la réaction d'ADN polymérase spécifique diffèrent significativement des paramètres respectifs détectés dans l'étape (c4) pour une réaction d'ADN polymérase spécifique effectuée avec une aliquote de la molécule d'ADN comme matrice à laquelle aucune protéine TALE n'a été ajoutée.

7. Procédé selon la revendication 6, dans lequel dans l'étape (c4)
(i) la concentration des produits de ladite réaction d'ADN polymérase spécifique est déterminée en utilisant un procédé, choisi dans le groupe constitué de la réaction en chaîne de la polymérase quantitative (qPCR), des mesures de fluorescence en utilisant des sondes fluorescentes se liant à des acides nucléiques, et la photométrie ; et/ou
(ii) la taille des produits de ladite réaction d'ADN polymérase spécifique est déterminée en utilisant un procédé, choisi dans le groupe constitué de la réaction en chaîne de la polymérase (PCR), et l'électrophorèse sur gel ; et/ou
(iii) la séquence des produits de ladite réaction d'ADN polymérase spécifique est déterminée en utilisant un procédé, choisi dans le groupe constitué du séquençage de Sanger, le pyroséquençage, l'hybridation sur micropuces, les procédés de séquençage nouvelle génération, et les procédés de séquençage nouvelle-nouvelle génération.

8. Procédé selon la revendication 6 ou 7, comprenant en outre les étapes de :
(d) la comparaison du paramètre détecté dans l'étape (c4) pour la réaction d'ADN polymérase spécifique à un ou plusieurs paramètres respectifs obtenus à partir de molécule d'ADN ayant un degré connu de présence de la nucléobase de cytosine d'intérêt ; et
(e) la détermination de cette façon du degré de présence de la nucléobase de cytosine d'intérêt dans la molécule d'ADN.

9. Procédé selon l'une quelconque des revendications 6 à 8, dans lequel la réaction d'ADN polymérase dans l'étape (c3) est choisie dans le groupe constitué de la réaction d'extension d'amorce, l'extension d'amorce sur puce (APEX), la réaction en chaîne de la polymérase (PCR), l'amplification par cercle roulant (RCA), et l'amplification par déplacement de brin (SDA).

10. Procédé pour l'enrichissement des molécules d'ADN dans lesquelles une nucléobase de cytosine d'intérêt particulière est présente, ledit procédé comprenant les étapes de :
(a) la fourniture d'une protéine effecteur de type activateur de la transcription (TALE), ladite protéine TALE étant capable de se lier d'une façon spécifique de la séquence à une région de ladite molécule d'ADN qui comprend ladite nucléobase d'intérêt, dans lequel ladite protéine TALE est couplée directement ou indirectement à un ligand d'affinité ;
(b) la soumission de la molécule d'ADN à une réaction de modification qui modifie sélectivement la nucléobase de cytosine d'intérêt au niveau de la position 5, dans lequel la présence de ladite modification chimique au niveau de la position 5 de la nucléobase de cytosine d'intérêt abolit ou affaiblit significativement la liaison de ladite protéine TALE à la molécule d'ADN ;
(c) l'incubation des molécules d'ADN avec ladite protéine TALE ; et
(d) la déplétion des molécules d'ADN dans lesquelles la nucléobase de cytosine d'intérêt est absente en passant par une capture par affinité utilisant ledit ligand d'affinité.

11. Procédé pour l'enrichissement des molécules d'ADN dans lesquelles une nucléobase de cytosine d'intérêt particulière est absente, ledit procédé comprenant les étapes de :
(a) la fourniture d'une protéine effecteur de type activateur de la transcription (TALE), ladite protéine TALE étant capable de se lier d'une façon spécifique de la séquence à une région de ladite molécule d'ADN qui comprend ladite nucléobase d'intérêt, dans lequel ladite protéine TALE est couplée directement ou indirectement à un ligand d'affinité ;
(b) la soumission de la molécule d'ADN à une réaction de modification qui modifie sélectivement la nucléobase de cytosine d'intérêt au niveau de la position 5, dans lequel la présence de ladite modification chimique au niveau de la position 5 de la nucléobase de cytosine d'intérêt renforce la liaison de ladite protéine TALE à la molécule d'ADN ;
(c) l'incubation des molécules d'ADN avec ladite protéine TALE ; et
(d) la déplétion des molécules d'ADN dans lesquelles la nucléobase de cytosine d'intérêt est présente en passant par une capture par affinité utilisant ledit ligand d'affinité.

12. Procédé pour la digestion différentielle des molécules d'ADN dans lesquelles une nucléobase de cytosine d'intérêt particulière est absente, dans lequel les molécules d'ADN dans lesquelles la nucléobase de cytosine d'intérêt est présente demeurent non digérées, ledit procédé comprenant les étapes de :
(a) la fourniture d'une protéine effecteur de type activateur de la transcription (TALE), ladite protéine TALE étant capable de se lier d'une façon spécifique de la séquence à une région de ladite molécule d'ADN qui comprend ladite nucléobase d'intérêt, dans lequel la protéine TALE est couplée directement ou indirectement à une protéine dotée d'une activité nucléase ;
(b) la soumission de la molécule d'ADN à une réaction de modification qui modifie sélectivement la nucléobase de cytosine d'intérêt au niveau de la position 5, dans lequel la présence de ladite modification chimique au niveau de la position 5 de la nucléobase de cytosine d'intérêt abolit ou affaiblit significativement la liaison de ladite protéine TALE à la molécule d'ADN ;
(c) l'incubation des molécules d'ADN avec ladite protéine TALE ; et
(d) la digestion des molécules d'ADN dans lesquelles la nucléobase de cytosine d'intérêt est absente en utilisant ladite protéine dotée d'une activité nucléase.

13. Procédé pour la digestion différentielle des molécules d'ADN dans lesquelles une nucléobase de cytosine d'intérêt particulière est présente, dans lequel les molécules d'ADN dans lesquelles la nucléobase de cytosine d'intérêt est absente demeurent non digérées, ledit procédé comprenant les étapes de :
(a) la fourniture d'une protéine effecteur de type activateur de la transcription (TALE), ladite protéine TALE étant capable de se lier d'une façon spécifique de la séquence à une région de ladite molécule d'ADN qui comprend ladite nucléobase d'intérêt, dans lequel la protéine TALE est couplée directement ou indirectement à une protéine dotée d'une activité nucléase ;
(b) la soumission de la molécule d'ADN à une réaction de modification qui modifie sélectivement la nucléobase de cytosine d'intérêt au niveau de la position 5, dans lequel la présence de ladite modification chimique au niveau de la position 5 de la nucléobase de cytosine d'intérêt renforce la liaison de ladite protéine TALE à la molécule d'ADN ;
(c) l'incubation des molécules d'ADN avec ladite protéine TALE ; et
(d) la digestion des molécules d'ADN dans lesquelles la nucléobase de cytosine d'intérêt est présente en utilisant ladite protéine dotée d'une activité nucléase.

14. Procédé pour la digestion différentielle des molécules d'ADN dans lesquelles une nucléobase de cytosine d'intérêt particulière est absente, dans lequel les molécules d'ADN dans lesquelles la nucléobase de cytosine d'intérêt est présente demeurent non digérées, ledit procédé comprenant les étapes de :
(a) la fourniture d'une protéine effecteur de type activateur de la transcription (TALE), ladite protéine TALE étant capable de se lier d'une façon spécifique de la séquence à une région de ladite molécule d'ADN qui comprend ladite nucléobase d'intérêt ;
(b) la soumission de la molécule d'ADN à une réaction de modification qui modifie sélectivement la nucléobase de cytosine d'intérêt au niveau de la position 5, dans lequel la présence de ladite modification chimique au niveau de la position 5 de la nucléobase de cytosine d'intérêt renforce la liaison de ladite protéine TALE à la molécule d'ADN ;
(c) l'incubation des molécules d'ADN avec ladite protéine TALE ; et
(d) l'incubation des molécules d'ADN avec une protéine dotée d'une activité nucléase ;
dans lequel les molécules d'ADN dans lesquelles la nucléobase de cytosine d'intérêt est absente sont digérées en utilisant ladite protéine dotée d'une activité nucléase, et les molécules d'ADN dans lesquelles la nucléobase de cytosine d'intérêt est présente sont protégées contre la digestion par la protéine TALE liée à la molécule d'ADN.

15. Procédé pour la digestion différentielle des molécules d'ADN dans lesquelles une nucléobase de cytosine d'intérêt particulière est présente, dans lequel les molécules d'ADN dans lesquelles la nucléobase de cytosine d'intérêt est absente demeurent non digérées, ledit procédé comprenant les étapes de :
(a) la fourniture d'une protéine effecteur de type activateur de la transcription (TALE), ladite protéine TALE étant capable de se lier d'une façon spécifique de la séquence à une région de ladite molécule d'ADN qui comprend ladite nucléobase d'intérêt ;
(b) la soumission de la molécule d'ADN à une réaction de modification qui modifie sélectivement la nucléobase de cytosine d'intérêt au niveau de la position 5, dans lequel la présence de ladite modification chimique au niveau de la position 5 de la nucléobase de cytosine d'intérêt abolit ou affaiblit significativement la liaison de ladite protéine TALE à la molécule d'ADN ;
(c) l'incubation des molécules d'ADN avec ladite protéine TALE ; et
(d) l'incubation des molécules d'ADN avec une protéine dotée d'une activité nucléase ;
dans lequel les molécules d'ADN dans lesquelles la nucléobase de cytosine d'intérêt est présente sont digérées en utilisant ladite protéine dotée d'une activité nucléase, et les molécules d'ADN dans lesquelles la nucléobase de cytosine intérêt est absente sont protégées contre la digestion par la protéine TALE liée à la molécule d'ADN.
